# EUROPÄISCHE PATENTSCHRIFT

(11) **EP 3 095 791 B1**
(45) Veröffentlichungstag und Bekanntmachung des Hinweises auf die Patenterteilung: **10.06.2020**
(21) Anmeldenummer: 16176546.6
(22) Anmeldetag: 10.09.2004
(51) Int. Cl.: C07K 14/47, C07K 16/30, C12Q 1/68

(54) **DIFFERENTIELL IN TUMOREN EXPRIMIERTE GENPRODUKTE UND DEREN VERWENDUNG**
GENETIC PRODUCTS WHICH ARE DIFFERENTIALLY EXPRESSED IN TUMOURS AND USE THEREOF
PRODUITS GENIQUES EXPRIMES DE MANIERE DIFFERENTIELLE DANS DES TUMEURS ET LEUR UTILISATION

(30) Priorität: 10.09.2003 DE 10341812
(43) Veröffentlichungstag der Anmeldung: 23.11.2016
(62) Teilanmeldung aus: 04765088.2
(73) Patentinhaber: BioNTech SE, 55131 Mainz (DE)
(72) Erfinder: TÜRECI, Özlem, 55116 Mainz (DE); SAHIN, Ugur, 55116 Mainz (DE); KOSLOWSKI, Michael, 85764 Oberschleissheim (DE)
(74) Vertreter: Wilk, Thomas

(56) Entgegenhaltungen:
- WO-A-03/076631
- WO-A2-02/10363
- DONG X -Y ET AL: "Identification of two novel CT antigens and their capacity to elicit antibody response in hepatocellular carcinoma patients.", BRITISH JOURNAL OF CANCER, Bd. 89, Nr. 2, 21. Juli 2003 (2003-07-21), Seiten 291-297, XP002309818, ISSN: 0007-0920
- BRUN M-E ET AL: "Juxtacentromeric region of human chromosome 21: a boundary between centromeric heterochromatin and euchromatic chromosome arms", GENE: AN INTERNATIONAL JOURNAL ON GENES AND GENOMES, ELSEVIER SCIENCE PUBLISHERS, BARKING, GB, Bd. 312, 17. Juli 2003 (2003-07-17), Seiten 41-50, XP004443903, ISSN: 0378-1119
- TAPPAREL C ET AL: "The TPTE gene family: cellular expression, subcellular localization and alternative splicing", GENE: AN INTERNATIONAL JOURNAL ON GENES AND GENOMES, ELSEVIER SCIENCE PUBLISHERS, BARKING, GB, Bd. 323, 24. Dezember 2003 (2003-12-24), Seiten 189-199, XP004477044, ISSN: 0378-1119
- WALKER STEVEN M ET AL: "TPIP: A novel phosphoinositide 3-phosphatase", BIOCHEMICAL JOURNAL, Bd. 360, Nr. 2, 1. Dezember 2001 (2001-12-01), Seiten 277-283, XP002309819, ISSN: 0264-6021
- CHEN H ET AL: "A TESTIS-SPECIFIC GENE, TPTE, ENCODES A PUTATIVE TRANSMEMBRANE TYROSINE PHOSPHATASE AND MAPS TO THE PERICENTROMERIC REGION OF HUMAN CHROMOSOMES 21 AND 13, AND TO CHROMOSOMES 15, 22, AND Y", HUMAN GENETICS, BERLIN, DE, Bd. 105, Nr. 5, 1999, Seiten 399-409, XP001000438, ISSN: 0340-6717
- SCHEURLE DANIELA ET AL: "Cancer gene discovery using Digital Differential Display", CANCER RESEARCH, AMERICAN ASSOCIATION FOR CANCER RESEARCH, BALTIMORE, MD, US, Bd. 60, Nr. 15, 1. August 2000 (2000-08-01), Seiten 4037-4043, XP002256957, ISSN: 0008-5472
- KOSLOWSKI MICHAEL ET AL: "Frequent nonrandom activation of germ-line genes in human cancer", CANCER RESEARCH, Bd. 64, Nr. 17, 1. September 2004 (2004-09-01), Seiten 5988-5993, XP002309835, ISSN: 0008-5472
- EBSEN M ET AL: "New Methods, Abstract 253-261, Posters", PATHOLOGY RESEARCH AND PRACTICE, GUSTAV FISCHER, STUTTGART, DE, Bd. 199, Nr. 4, 1. Januar 2003 (2003-01-01), Seiten 244-247, XP004958947, ISSN: 0344-0338, DOI: DOI:10.1078/0344-0338-00404

## Beschreibung

Trotz interdisziplinärer Ansätze und Ausreizung klassischer Therapiemodalitäten gehören Krebserkrankungen weiterhin zu den führenden Todesursachen. Neuere therapeutische Konzepte zielen darauf ab, das patienteneigene Immunsystem durch Einsatz von rekombinanten Tumorvakzinen und anderen spezifischen Maßnahmen wie Antikörpertherapie in das therapeutische Gesamtkonzept mit einzubeziehen. Voraussetzung für den Erfolg einer solchen Strategie ist die Erkennung von Tumor-spezifischen oder Tumor-assoziierten Antigenen bzw. Epitopen durch das Immunsystem des Patienten, dessen Effektorfunktionen interventionell verstärkt werden sollen. Tumorzellen unterscheiden sich biologisch wesentlich von ihren nichtmalignen Ursprungszellen. Diese Differenzen sind durch während der Tumorentwicklung erworbene genetische Veränderungen bedingt und führen u.a. auch zur der Bildung qualitativ oder quantitativ veränderter molekularer Strukturen in den Krebszellen. Werden solche Tumor-assoziierten Strukturen vom spezifischen Immunsystem des tumortragenden Wirtes erkannt, spricht man von tumor-assoziierten Antigenen. An der spezifischen Erkennung von tumor-assoziierten Antigenen sind zelluläre und humorale Mechanismen beteiligt, die zwei miteinander funktionell vernetzte Einheiten darstellen: CD4⁺ und CD8⁺ T-Lymphozyten erkennen prozessierte Antigene, die auf den Molekülen der MHC-(Major Histocompatibility complex = HistokompatibilitätsAntigene) Klassen II bzw. I präsentiert werden, während B-Lymphozyten zirkulierende Antikörpermoleküle produzieren, die direkt an unprozessierte Antigene binden. Die potentielle klinisch-therapeutische Bedeutung von tumor-assoziierten Antigenen ergibt sich aus der Tatsache, dass die Erkennung von Antigenen auf neoplastischen Zellen durch das Immunsystem zur Initiierung von cytotoxischen Effektormechanismen führt und bei Vorhandensein von T-Helferzellen die Elimination der Krebszellen bewirken kann (Pardoll, Nat. Med. 4:525-31, 1998). Entsprechend ist es eine zentrale Zielsetzung der Tumorimmunologie, diese Strukturen molekular zu definieren. Die molekulare Natur dieser Antigene blieb lange enigmatisch. Erst als entsprechende Klonierungstechniken entwickelt wurden, gelang es, durch Analyse der Zielstrukturen von cytotoxischen T-Lymphozyten (CTL) (van der Bruggen et al., Science 254:1643-7, 1991) bzw. mit zirkulierenden Autoantikörpern (Sahin et al., Curr. Opin. Immunol. 9:709-16, 1997) als Sonden cDNA-Expressionbanken von Tumoren systematisch auf tumor-assoziierte Antigene zu screenen. Hierzu wurden cDNA-Expressionsbanken aus frischem Tumorgewebe hergestellt und in geeigneten Systemen als Proteine rekombinant exprimiert. Aus Patienten isolierte Immuneffektoren, nämlich CTL-Klone mit Tumorspezifischem Lysemuster, oder zirkulierende Autoantikörper wurden genutzt, um die respektiven Antigene zu klonieren.

Durch diese Ansätze sind in den letzten Jahren eine Vielzahl von Antigenen in verschiedenen Neoplasien definiert worden. Von großem Interesse ist dabei die Klasse der Cancer/Testis-Antigene (CTA). CTA und sie kodierende Gene (Cancer/Testis-Gene oder CTG) sind durch ihr charakteristisches Expressionsmuster definiert [Tureci et al, Mol Med Today. 3:342-9, 1997]. Sie finden sich nicht in Normalgeweben bis auf Testis bzw. Keimzellen, werden jedoch in einer Reihe von humanen Malignomen exprimiert und zwar nicht tumortypspezifisch, sondern mit unterschiedlicher Häufigkeit in Tumorentitäten ganz unterschiedlicher Herkunft (Chen & Old, Cancer J. Sci. Am. 5:16-7, 1999). Auch Serumreaktivitäten gegen CTA finden sich nicht in gesunden Kontrollen, sondern lediglich in Tumorpatienten. Insbesondere aufgrund ihrer Gewebeverteilung ist diese Antigenklasse von besonderem Wert für immuntherapeutische Vorhaben und wird in derzeit laufenden klinischen Patientenstudien getestet (Marchand et al., Int. J. Cancer 80:219-30, 1999; Knuth et al., Cancer Chemother. Pharmacol. 46: S46-51, 2000).

Allerdings nutzen die oben dargestellten klassischen Verfahren zur Antigenidentifizierung Immuneffektoren (zirkulierende Autoantikörper oder CTL-Klone) aus Patienten mit in der Regel bereits fortgeschrittenem Krebs als Sonden. Aus einer Reihe von Daten geht hervor, dass Tumoren z.B. zur Tolerisierung und Anergisierung von T-Zellen führen können und gerade im Verlauf der Erkrankung diejenigen Spezifitäten aus dem Immuneffektorenrepertoire verloren gehen, die eine effektive Immunerkennung bewirken könnten. Aus laufenden Patientenstudien hat sich noch kein gesicherter Beweis für eine tatsächliche Wirkung der bisher entdeckten und genutzten tumor-assoziierten Antigene ergeben. Entsprechend kann nicht ausgeschlossen werden, dass spontane Immunantworten evozierende Proteine die falschen Zielstrukturen sind.

Es war die Aufgabe der vorliegenden Erfindung Zielstrukturen für eine Diagnose und Therapie von Krebserkrankungen bereitzustellen.

Diese Aufgabe wird erfindungsgemäß durch den Gegenstand der Patentansprüche gelöst. Erfindungsgemäß wurde eine Strategie für eine Identifizierung und Bereitstellung Tumorassoziiert exprimierter Antigene und der dafür kodierenden Nukleinsäuren verfolgt. Diese Strategie beruht auf der Tatsache, dass eigentlich Testis- und damit Keimzell-spezifische Gene, die normalerweise in adulten Geweben silent sind, in Tumorzellen ektop und unerlaubt reaktiviert werden. Durch Datamining wird zunächst eine möglichst komplette Liste aller bekannten Testis-spezifischen Gene aufgestellt und diese sodann durch Expressionsanalysen mittels spezifischer RT-PCR auf ihre aberrante Aktivierung in Tumoren evaluiert. Datamining ist ein bekanntes Verfahren zur Identifizierung von Tumor-assoziierten Genen. Bei den herkömmlichen Strategien werden allerdings in der Regel Transkriptome von Normalgewebebanken elektronisch von Tumorgewebsbanken subtrahiert unter der Annahme, dass die verbleibenden Gene Tumor-spezifisch sind (Schmitt et al., Nucleic Acids Res. 27:4251-60, 1999; Vasmatzis et al., Proc. Natl. Acad. Sci. USA. 95:300-4, 1998. Scheurle et al., Cancer Res. 60:4037-43, 2000).

Das erfindungsgemäße Konzept, das sich als viel erfolgreicher erwiesen hat, beruht jedoch darauf, Datamining zur elektronischen Extraktion aller Testis-spezifischer Gene zu nutzen und diese sodann auf ektope Expression in Tumoren zu evaluieren.

Somit betrifft die vorliegende technische Lehre in einem Aspekt eine Strategie zur Identifizierung von differentiell in Tumoren exprimierten Genen. Diese kombiniert Datamining von öffentlichen Sequenzbanken ("*in silico*") mit darauffolgenden evaluierenden labor-experimentellen ("wet bench") Untersuchungen.

Eine kombinierte Strategie basierend auf zwei unterschiedlichen bioinformatischen Skripten ermöglichte erfindungsgemäß die Identifizierung neuer Mitglieder der Cancer/Testis (CT) Genklasse. Diese sind bisher als rein Testis-, Keimzell-, oder Spermien-spezifisch eingestuft worden. Die Erkenntnis, dass diese Gene aberrant in Tumorzellen aktiviert werden, erlaubt, ihnen eine substantiell neue Qualität mit funktionellen Implikationen zuzuordnen. Die Identifizierung und Bereitstellung dieser tumor-assoziierten Gene und der dadurch kodierten Genprodukte erfolgte offenbarungsgemäß unabhängig von einer immunogenen Wirkung.

Die identifizierten Tumor-assoziierten Antigene weisen eine Aminosäuresequenz auf, die von einer Nukleinsäure kodiert wird, die aus der Gruppe ausgewählt ist, bestehend aus (a) einer Nukleinsäure, die eine Nukleinsäuresequenz umfasst, die aus der Gruppe bestehend aus SEQ ID NOs: 19-21 und 54-57 einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist und (d) einer Nukleinsäure, die zu der Nukleinsäure unter (a), (b) oder (c) komplementär ist. In einer bevorzugten Ausführungsform weist ein erfindungsgemäß identifiziertes Tumor-assoziiertes Antigen eine Aminosäuresequenz auf, die von einer Nukleinsäure kodiert wird, die aus der Gruppe bestehend aus SEQ ID NOs: 19-21 und 54-57 ausgewählt ist. In einer weiteren bevorzugten Ausführungsform umfasst ein erfindungsgemäß identifiziertes tumor-assoziiertes Antigen eine Aminosäuresequenz, die aus der Gruppe bestehend aus SEQ ID NOs: 22-24, 58-61, 81, 82 und 103-105 einem Teil oder Derivat davon ausgewählt ist.

Die vorliegende technische Lehre betrifft allgemein die Verwendung von erfindungsgemäß identifizierten Tumor-assoziierten Antigenen oder von Teilen davon, von dafür kodierenden Nukleinsäuren oder von Nukleinsäuren, die gegen die kodierenden Nukleinsäuren gerichtet sind oder von Antikörpern, die gegen die erfindungsgemäß identifizierten Tumor-assoziierten Antigene oder Teile davon gerichtet sind, für die Therapie und Diagnose. Diese Nutzung kann einzelne, aber auch Kombinationen von mehreren dieser Antigene, funktionalen Fragmente, Nukleinsäuren, Antikörper etc betreffen, in einer Ausführungsform auch in Kombination mit anderen tumor-assoziierten Genen und Antigenen für eine Diagnose, Therapie und Verlaufskontrolle.

Bevorzugte Erkrankungen für eine Therapie und/oder Diagnose sind solche, bei denen eine selektive Expression oder abnormale Expression von einem oder mehreren der erfindungsgemäß identifizierten Tumor-assoziierten Antigenen vorliegt.

Die ektope Aktivierung von Genen in Tumoren kann auch zurückführbar sein auf ein verändertes Genmethylierungsmuster ihrer Nukleotidsequenz. So ist z.B. beschrieben worden, dass Alterationen der Methylierung am Cytosin hierzu beitragen (De Smet et al, 1996 und 1999).

Die vorliegende Offenbarung betrifft auch Nukleinsäuren und Genprodukte, die tumorzellassoziiert exprimiert werden und die durch verändertes Spleißen (Spleißvarianten) bekannter Gene bzw. durch veränderte Translation unter Nutzung alternativer offener Leserahmen entstehen. Diese Nukleinsäuren umfassen die Sequenzen gemäß SEQ ID NOs: 20, 21 und 54-57 des Sequenzprotokolls. Ferner umfassen die Genprodukte Sequenzen gemäß SEQ ID NOs: 23, 24 und 58-61 des Sequenzprotokolls. Die Spleißvarianten sind gemäß der technischen Lehre als Targets für die Diagnostik und Therapie von Tumorerkrankungen verwendbar.

Für die Entstehung von Spleißvarianten können verschiedenste Mechanismen ursächlich sein, beispielsweise
- die Nutzung variabler Transkriptionsinitiationsstellen
- die Nutzung zusätzlicher Exons
- vollständiges oder unvollständiges Aus Spleißen von einzelnen oder mehreren Exons,
- über Mutation veränderte Spleißregulatorsequenzen (Deletion bzw. Schaffung neuer Donor/Acceptorsequenzen),
- die unvollständige Elimination von Intronsequenzen.

Das veränderte Spleißen eines Gens führt zu einer veränderten Transkriptsequenz (Spleißvariante). Wird eine Spleißvariante im Bereich ihrer veränderten Sequenz translatiert, resultiert ein verändertes Protein, welches sich von dem ursprünglichen in Struktur und Funktion deutlich unterscheiden kann. Bei tumorassoziierten Spleißvarianten können tumorassoziierte Transkripte und tumorassoziierte Proteine/Antigene entstehen. Diese können als molekulare Marker sowohl zum Nachweis von Tumorzellen als auch zum therapeutischen Targeting von Tumoren genutzt werden. Die Detektion von Tumorzellen z.B. im Blut, Serum, Knochenmark, Sputum, Bronchial-Lavage, Körpersekreten und Gewebsbiopsien kann erfindungsgemäß z.B. nach Extraktion von Nukleinsäuren durch PCR-Amplifikation mit Spleißvariantenspezifischen Oligonukleotiden erfolgen. Zum Nachweis eignen sich erfindungsgemäß alle Sequenz-abhängigen Detektionssysteme. Neben der PCR sind diese z.B. Genchip-/Microarraysysteme, Northern-Blot, RNAse protection assays (RDA) und andere. Allen Detektionssystemen ist gemeinsam, dass die Detektion auf einer spezifischen Hybridisierung mit mindestens einer Spleißvarianten-spezifischen Nukleinsäuresequenz basiert. Die Detektion von Tumorzellen kann jedoch auch erfindungsgemäß durch Antikörper erfolgen, die ein durch die Spleißvariante kodiertes spezifisches Epitop erkennen. Für die Herstellung der Antikörper können Peptide zur Immunisierung verwendet werden, die für diese Spleißvariante spezifisch sind. Für die Immunisierung eignen sich besonders die Aminosäuren, die deutliche Epitopunterschiede zu der Variante(n) des Genprodukts aufweisen, welche(s) bevorzugt in gesunden Zellen gebildet wird. Der Nachweis der Tumorzellen mit Antikörper kann dabei an einer vom Patienten isolierten Probe oder als Imaging mit intravenös applizierten Antikörpern erfolgen. Neben der diagnostischen Nutzbarkeit stellen Spleißvarianten, die neue oder veränderte Epitope aufweisen, attraktive Targets für die Immuntherapie dar. Die Epitope können zum Targeting von therapeutisch wirksamen monoklonalen Antikörpern oder T-Lymphozyten genutzt werden. Bei der passiven Immuntherapie werden hierbei Antikörper oder T-Lymphozyten adoptiv transferriert, die Spleißvarianten-spezifische Epitope erkennen. Die Generierung von Antikörpern kann wie bei anderen Antigenen auch unter Nutzung von Standardtechnologien (Immunisierung von Tieren, Panningstrategien zur Isolation von rekombinanten Antikörpern) unter Nutzung von Polypeptiden, die diese Epitope beinhalten, erfolgen. Alternativ können zur Immunisierung Nukleinsäuren genutzt werden, die für Oligo- oder Polypeptide kodieren, die diese Epitope beinhalten. Verschiedene Techniken zur in vitro oder in vivo Generierung von epitopspezifischen T-Lymphozyten sind bekannt und ausführlich beschrieben z.B. (Kessler JH, et al. 2001, Sahin et al., 1997) und basieren ebenfalls auf der Nutzung von Oligo-oder Polypeptide, die die Spleißvarianten-spezifischen Epitope beinhalten oder Nukleinsäuren, die für diese kodieren. Oligo-oder Polypeptiden, die die Spleißvarianten-spezifischen Epitope beinhalten oder Nukleinsäuren, die für diese Polypeptide kodieren sind auch für die Nutzung als pharmazeutisch wirksame Substanzen bei der aktiven Immuntherapie (Vakzinierung, Vakzintherapie) verwendbar.

In einem Aspekt betrifft die technische Lehre eine pharmazeutische Zusammensetzung umfassend ein Mittel, das das erfindungsgemäß identifizierte Tumor-assoziierte Antigen erkennt und vorzugsweise selektiv für Zellen ist, die eine Expression oder abnormale Expression eines erfindungsgemäß identifizierten Tumor-assoziierten Antigens aufweisen. Das Mittel kann in bestimmten Ausführungsformen die Induktion des Zelltods, die Reduktion des Zellwachstums, die Schädigung der Zellmembran oder die Sekretion von Zytokinen bewirken und weist vorzugsweise eine tumorhemmende Aktivität auf. In einer Ausführungsform ist das Mittel eine Antisense-Nukleinsäure, die selektiv mit der Nukleinsäure hybridisiert, die für das Tumor-assoziierte Antigen kodiert. In einer weiteren Ausführungsform ist das Mittel ein Antikörper, der selektiv an das Tumor-assoziierte Antigen bindet, insbesondere ein komplementaktivierter Antikörper, der selektiv an das Tumorassoziierte Antigen bindet. In einer weiteren Ausführungsform umfasst das Mittel mehrere Mittel, die jeweils selektiv verschiedene Tumor-assoziierte Antigene erkennen, wobei mindestens eines der Tumor-assoziierten Antigene ein erfindungsgemäß identifiziertes Tumor-assoziiertes Antigen ist. Die Erkennung muss nicht direkt mit einer Hemmung von Aktivität oder Expression des Antigens einhergehen. In diesem Aspekt der Lehre dient das selektiv auf Tumoren beschränkte Antigen vorzugsweise als Markierung zur Rekrutierung von Effektormechanismen an diesen spezifischen Ort. In einer bevorzugten Ausführungsform ist das Mittel ein cytotoxischer T-Lymphozyt, der das Antigen auf einem HLA-Molekül erkennt und die derartig markierte Zelle lysiert. In einer weiteren Ausführungsform ist das Mittel ein Antikörper, der selektiv an das Tumor-assoziierte Antigen bindet und somit natürliche oder artifizielle Effektormechanismen zu dieser Zelle rekrutiert. In einer weiteren Ausführungsform ist das Mittel ein T-Helfer-Lymphozyt, der Effektorfunktionen von anderen Zellen, die spezifisch dieses Antigen erkennen, stärkt.

In einem Aspekt betrifft die vorliegende technische Lehre eine pharmazeutische Zusammensetzung umfassend ein Mittel, das die Expression oder Aktivität eines erfindungsgemäß identifizierten Tumor-assoziierten Antigens hemmt. In einer bevorzugten Ausführungsform ist das Mittel eine Antisense-Nukleinsäure, die selektiv mit der Nukleinsäure hybridisiert, die für das Tumor-assoziierte Antigen kodiert. In einer weiteren Ausführungsform ist das Mittel ein Antikörper, der selektiv an das Tumor-assoziierte Antigen bindet. In einer weiteren Ausführungsform umfasst das Mittel mehrere Mittel, die jeweils selektiv die Expression oder Aktivität verschiedener Tumor-assoziierter Antigene hemmen, wobei mindestens eines der Tumor-assoziierten Antigene ein erfindungsgemäß identifiziertes Tumor-assoziiertes Antigen ist.

Die Aktivität eines erfindungsgemäß identifizierten Tumor-assoziierten Antigens kann eine jegliche Aktivität eines Proteins oder Peptids sein. In einer weiteren Ausführungsform betrifft diese Aktivität eine Beteiligung bei einer zellulären Migration und/oder Metastatisierung wie im Fall der TPTE betreffenden Sequenzen (vgl. Beispiel 1). Somit können die erfindungsgemäßen Therapie- und Diagnosverfahren auch auf Hemmung oder Reduktion dieser Aktivität oder auf ein Testen dieser Aktivität abzielen.

Des weiteren betrifft die technische Lehre eine pharmazeutische Zusammensetzung, die ein Mittel umfasst, das bei einer Verabreichung selektiv die Menge an Komplexen zwischen einem HLA-Molekül und einem Peptidepitop aus dem erfindungsgemäß identifizierten Tumor-assoziierten Antigen erhöht. Das Mittel umfasst in einer Ausführungsform einen oder mehrere Bestandteile, die aus der Gruppe ausgewählt sind, bestehend aus (i) dem Tumorassoziierten Antigen oder einem Teil davon, (ii) einer Nukleinsäure, die für das Tumorassoziierte Antigen oder einen Teil davon kodiert, (iii) einer Wirtszelle, die das Tumorassoziierte Antigen oder einen Teil davon exprimiert, und (iv) isolierten Komplexen zwischen Peptidepitopen aus dem Tumor-assoziierten Antigen und einem MHC-Molekül. In einer Ausführungsform umfasst das Mittel mehrere Mittel, die jeweils selektiv die Menge an Komplexen zwischen MHC-Molekülen und Peptidepitopen verschiedener Tumor-assoziierter Antigene erhöhen, wobei mindestens eines der Tumor-assoziierten Antigene ein erfindungsgemäß identifiziertes Tumor-assoziiertes Antigen ist.

Des weiteren betrifft die technische Lehre eine pharmazeutische Zusammensetzung, die einen oder mehrer Bestandteile umfasst, die aus der Gruppe ausgewählt sind, bestehend aus (i) einem erfindungsgemäß identifizierten Tumor-assoziierten Antigen oder einem Teil davon, (ii) einer Nukleinsäure, die für ein erfindungsgemäß identifiziertes Tumor-assoziiertes Antigen oder einen Teil davon kodiert, (iii) einem Antikörper, der an ein erfindungsgemäß identifiziertes Tumor-assoziiertes Antigen oder einen Teil davon bindet, (iv) einer Antisense-Nukleinsäure, die spezifisch mit einer Nukleinsäure, die für ein erfindungsgemäß identifiziertes Tumor-assoziiertes Antigen kodiert, hybridisiert, (v) einer Wirtszelle, die ein erfindungsgemäß identifiziertes Tumor-assoziiertes Antigen oder einen Teil davon exprimiert, und (vi) isolierten Komplexen zwischen einem erfindungsgemäß identifizierten Tumorassoziierten Antigen oder einem Teil davon und einem HLA-Molekül.

Eine Nukleinsäure, die für ein erfindungsgemäß identifiziertes Tumor-assoziiertes Antigen oder einen Teil davon kodiert, kann in der pharmazeutische Zusammensetzung in einem Expressionsvektor vorliegen und funktionell mit einem Promotor verbunden sein.

Eine in einer pharmazeutischen Zusammensetzung enthaltene Wirtszelle kann das Tumorassoziierte Antigen oder den Teil davon sekretieren, auf der Oberfläche exprimieren oder kann zusätzlich ein HLA-Molekül exprimieren, das an das Tumor-assoziierte Antigen oder den Teil davon bindet. In einer Ausführungsform exprimiert die Wirtszelle das HLA-Molekül endogen. In einer weiteren Ausführungsform exprimiert die Wirtszelle das HLA-Molekül und/oder das Tumor-assoziierte Antigen oder den Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

Ein in einer pharmazeutischen Zusammensetzung enthaltener Antikörper kann ein monoklonaler Antikörper sein. In weiteren Ausführungsformen ist der Antikörper ein chimärer oder humanisierter Antikörper, ein Fragment eines natürlichen Antikörpers, oder ein synthetischer Antikörper, die durch kombinatorische Techniken hergestellt werden können. Der Antikörper kann mit einem therapeutisch oder diagnostisch nützlichen Mittel gekoppelt sein.

Eine in einer pharmazeutischen Zusammensetzung enthaltene Antisense-Nukleinsäure kann eine Sequenz von 6-50, insbesondere 10-30, 15-30 und 20-30 zusammenhängenden Nukleotiden aus der Nukleinsäure, die für das erfindungsgemäß identifizierte Tumorassoziierte Antigen kodiert, umfassen.

In weiteren Ausführungsformen bindet ein durch eine pharmazeutische Zusammensetzung entweder direkt oder durch die Expression einer Nukleinsäure bereitgestelltes Tumor-assoziiertes Antigen oder ein Teil davon an MHC-Moleküle auf der Oberfläche von Zellen, wobei die Bindung vorzugsweise eine cytolytische Reaktion hervorruft und/oder eine Cytokinausschüttung induziert.

Eine pharmazeutische Zusammensetzung kann einen pharmazeutisch verträglichen Träger und/oder ein Adjuvans umfassen. Das Adjuvans kann aus Saponin, GM-CSF, CpG-Nukleotiden, RNA, einem Zytokin oder einem Chemokin ausgewählt sein. Eine erfindungsgemäße pharmazeutische Zusammensetzung wird vorzugsweise zur Behandlung einer Erkrankung eingesetzt, die sich durch die selektive Expression oder abnormale Expression eines Tumor-assoziierten Antigens auszeichnet. In einer bevorzugten Ausführungsform ist die Erkrankung Krebs.

Desweiteren betrifft die vorliegende Lehre Verfahren zur Behandlung oder Diagnose einer Erkrankung, die sich durch die Expression oder abnormale Expression eines oder mehrerer Tumor-assoziierter Antigene auszeichnet. In einer Ausführungsform umfasst die Behandlung die Verabreichung einer offenbarungsgemäßen pharmazeutischen Zusammensetzung.

In einem Aspekt betrifft die Lehre ein Verfahren zur Diagnose einer Erkrankung, die sich durch die Expression oder abnormale Expression eines erfindungsgemäß identifizierten Tumor-assoziierten Antigens auszeichnet. Das Verfahren umfasst den Nachweis (i) einer Nukleinsäure, die für das Tumor-assoziierte Antigen kodiert, oder eines Teils davon und/oder (ii) den Nachweis des Tumor-assoziierten Antigens oder eines Teils davon, und/oder (iii) den Nachweis eines Antikörpers gegen das Tumor-assoziierte Antigen oder einen Teil davon und/oder (iv) den Nachweis von cytotoxischen oder Helfer-T-Lymphozyten, die für das Tumor-assoziierte Antigen oder einen Teil davon spezifisch sind in einer aus einem Patienten isolierten biologischen Probe. In bestimmten Ausführungsformen umfasst der Nachweis (i) die Kontaktierung der biologischen Probe mit einem Mittel, das spezifisch an die Nukleinsäure, die für das Tumor-assoziierte Antigen kodiert, oder den Teil davon, an das Tumor-assoziierte Antigen oder den Teil davon, an den Antikörper oder an cytotoxische oder Helfer-T-Lymphozyten, die für das Tumor-assoziierte Antigen oder Teile davon spezifisch sind, bindet und (ii) den Nachweis der Komplexbildung zwischen dem Mittel und der Nukleinsäure oder dem Teil davon, dem Tumor-assoziierten Antigen oder dem Teil davon, dem Antikörper oder den cytotoxischen oder Helfer-T-Lymphozyten. In einer Ausführungsform zeichnet sich die Erkrankung durch die Expression oder abnormale Expression mehrerer verschiedener Tumor-assoziierter Antigene aus und der Nachweis umfasst einen Nachweis mehrerer Nukleinsäuren, die für die mehreren verschiedenen Tumorassoziierten Antigene kodieren, oder von Teilen davon, den Nachweis der mehreren verschiedenen Tumor-assoziierten Antigene oder von Teilen davon, den Nachweis mehrerer Antikörper, die an die mehreren verschiedenen Tumor-assoziierten Antigene oder an Teile davon binden oder den Nachweis mehrerer cytotoxischer oder Helfer-T-Lymphozyten, die für die mehreren verschiedenen Tumor-assoziierten Antigene spezifisch sind. In einer weiteren Ausführungsform wird die isolierte biologische Probe aus dem Patienten mit einer vergleichbaren normalen biologischen Probe verglichen.

Die offenbarten Diagnoseverfahren können auch eine Nutzung der erfindungsgemäß identifizierten Tumor-assoziierten Antigene als prognostische Marker betreffen, um eine Metastatisierung z.B. durch Testen des Migrationsverhalten von Zellen und daher einen verschlechterten Krankheitsverlauf zu prädizieren, wodurch unter anderem die Planung einer aggressiveren Therapie ermöglicht wird. Insbesondere sind die TPTE betreffenden Sequenzen (vgl. Beispiel 1) für diesen Zweck geeignet. Sie sind auch dafür geeignet, noch gutartige Veränderungen z.B. Hyperplasien von bereits ungünstig zu wertenden Tumor-Vorstufen abzugrenzen und somit eine Krebsneigung vorherzusehen noch bevor ein invasiver Tumor sich gebildet hat.

In dieser Ausführungsform betrifft die offenbarte Lehre ein Verfahren zur Diagnose einer Erkrankung, die sich durch die Expression oder abnormale Expression eines erfindungsgemäß identifizierten Tumor-assoziierten Antigens auszeichnet, wobei das Verfahren eine Bewertung des Migrationsverhaltens von Zellen aus einem Patienten umfasst. In besonders bevorzugten Ausführungsformen dient das Verfahren einer Bestimmung des Ausmaßes oder einer Prädiktion einer Metastatisierung und/oder von Lymphknoten- und/oder Fernmetastasen, wobei ein erhöhtes Migrationsverhalten von Zellen in bestimmten Ausführungsformen darauf hinweist, dass der getestete Patient an der Erkrankung, die sich durch die Expression oder abnormale Expression eines erfindungsgemäß identifizierten Tumor-assoziierten Antigens auszeichnet, erkrankt ist, daran erkranken wird und/oder ein Potential für eine derartige Erkrankung aufweist. In besonders bevorzugten Ausführungsformen weist ein erhöhtes Migrationsverhalten auf eine Metastatisierung und/oder Bildung von Lymphknoten- und/oder Fernmetastasen oder ein Potential dafür hin. In dieser Ausführungsform weist das erfindungsgemäß identifizierte Tumor-assoziierte Antigen vorzugsweise eine Sequenz auf, die von einer Nukleinsäure kodiert wird, die aus der Gruppe ausgewählt ist, bestehend aus: (a) einer Nukleinsäure, die eine Nukleinsäuresequenz umfasst, die aus der Gruppe bestehend aus SEQ ID NOs: 19-21 und 54-57, einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist und (d) einer Nukleinsäure, die zu der Nukleinsäure unter (a), (b) oder (c) komplementär ist. Das Tumor-assoziierte Antigen umfaßt vorzugsweise eine Aminosäuresequenz, die aus der Gruppe bestehend aus SEQ ID NOs: 22-24, 58-61, 81, 82 und 103-105, einem Teil oder Derivat davon ausgewählt ist.

Die offenbarten Verfahren zur Behandlung zielen in diesem Aspekt vorzugsweise auf die Verabreichung von pharmazeutischen Zusammensetzungen ab, die das Migrationsverhalten normalisieren, vorzugsweise hemmen.

In einer weiteren Ausführungsform betrifft die vorliegende Offenbarung ein Verfahren zur Diagnose einer Erkrankung, die sich durch die Expression oder abnormale Expression eines erfindungsgemäß identifizierten Tumor-assoziierten Antigens auszeichnet, wobei das Verfahren eine Bestimmung des Methylierungsmusters und/oder des Grades an Methylierung innerhalb einer Nukleinsäure, die eine Nukleinsäuresequenz umfasst, die für das Tumorassoziierte Antigen kodiert, insbesondere innerhalb der nicht-kodierenden Bereiche davon, bevorzugt innerhalb des Promotorbereiches davon umfaßt. Vorzugsweise weist das erfindungsgemäß identifizierte Tumor-assoziierte Antigen eine Sequenz auf, die von einer Nukleinsäure kodiert wird, die aus der Gruppe ausgewählt ist, bestehend aus: (a) einer Nukleinsäure, die eine Nukleinsäuresequenz umfasst, die aus der Gruppe bestehend aus SEQ ID NOs: 19-21, und 54-57, einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist und (d) einer Nukleinsäure, die zu der Nukleinsäure unter (a), (b) oder (c) komplementär ist, und die Bestimmung erfolgt bezüglich dieser Nukleinsäure, vorzugsweise innerhalb des Promotorbereiches davon, und insbesondere innerhalb der in SEQ ID NO: 822 gezeigten Sequenz, insbesondere innerhalb der Nukleotide von Position 121 bis 540 der in SEQ ID NO:822 gezeigten Sequenz (TPTE Promotorsequenz auf Chromosom 21; Position -368/+952 bezogen auf den Transkriptionsstart). Das Tumor-assoziierte Antigen umfaßt vorzugsweise eine Aminosäuresequenz, die aus der Gruppe bestehend aus SEQ ID NOs: 22-24, 58-61, 81, 82 und 103-105, einem Teil oder Derivat davon ausgewählt ist. Eine im Vergleich zu einer Kontrolle geringere Methylierung oder eine fehlende Methylierung weist vorzugsweise darauf hin, dass der getestete Patient an der Erkrankung, die sich durch die Expression oder abnormale Expression eines erfindungsgemäß identifizierten Tumor-assoziierten Antigens auszeichnet, erkrankt ist, daran erkranken wird und/oder ein erhöhtes Potential für eine derartige Erkrankung aufweist.

Verfahren zur Behandlung zielen in diesem Aspekt vorzugsweise auf die Verabreichung von pharmazeutischen Zusammensetzungen ab, die das Methylierungsmuster und/oder den Grad der Methylierung normalisieren, vorzugsweise erhöhen.

In einem weiteren Aspekt betrifft die vorliegende Lehre ein Verfahren zur Bestimmung der Regression, des Verlaufs oder des Ausbruchs einer Erkrankung, die sich durch die Expression oder abnormale Expression eines erfindungsgemäß identifizierten Tumor-assoziierten Antigens auszeichnet, umfassend die Überwachung einer Probe aus einem Patienten, der die Erkrankung aufweist oder in Verdacht steht, an der Erkrankung zu erkranken in Bezug auf einen oder mehrere Parameter, die aus der Gruppe ausgewählt sind, bestehend aus (i) der Menge der Nukleinsäure, die für das Tumor-assoziierte Antigen kodiert, oder eines Teil davon, (ii) der Menge des Tumor-assoziierten Antigens oder eines Teils davon, (iii) der Menge an Antikörpern, die an das Tumor-assoziierte Antigen oder einen Teil davon binden, und (iv) der Menge an cytolytischen T-Zellen oder Helfer-T-Zellen, die für einen Komplex zwischen dem Tumor-assoziierten Antigen oder einem Teil davon und einem MHC-Molekül spezifisch sind. Vorzugsweise umfasst das Verfahren die Bestimmung des oder der Parameter zu einem ersten Zeitpunkt in einer ersten Probe und zu einem zweiten Zeitpunkt in einer weiteren Probe, wobei durch einen Vergleich der beiden Proben der Verlauf der Erkrankung ermittelt wird. In bestimmten Ausführungsformen zeichnet sich die Erkrankung durch die Expression oder abnormale Expression mehrerer verschiedener Tumor-assoziierter Antigene aus und die Überwachung umfasst eine Überwachung (i) der Menge mehrerer Nukleinsäuren, die für die mehreren verschiedenen Tumor-assoziierten Antigene kodieren, oder von Teilen davon und/oder (ii) der Menge der mehreren verschiedenen Tumor-assoziierten Antigene oder von Teilen davon und/oder (iii) der Menge mehrerer Antikörper, die an die mehreren verschiedenen Tumor-assoziierten Antigene oder an Teile davon binden, und/oder (iv) der Menge mehrerer cytolytischer T-Zellen oder Helfer-T-Zellen, die für Komplexe zwischen den mehreren verschiedenen Tumor-assoziierten Antigenen oder von Teilen davon und MHC-Molekülen spezifisch sind.

Ein Nachweis einer Nukleinsäure oder eines Teils davon oder eine Überwachung der Menge einer Nukleinsäure oder eines Teils davon kann mit einer Polynukleotid-Sonde erfolgen, die spezifisch mit der Nukleinsäure oder dem Teil davon hybridisiert oder kann durch selektive Amplifikation der Nukleinsäure oder des Teils davon erfolgen. In einer Ausführungsform umfasst die Polynukleotid-Sonde eine Sequenz von 6-50, insbesondere 10-30, 15-30 und 20-30 zusammenhängenden Nukleotiden aus der Nukleinsäure.

In bestimmten Ausführungsformen liegt das nachzuweisende Tumor-assoziierte Antigen oder der Teil davon intrazellulär oder auf der Zelloberfläche vor. Ein Nachweis eines Tumorassoziierten Antigens oder eines Teils davon oder eine Überwachung der Menge eines Tumor-assoziierten Antigens oder eines Teils davon kann mit einem Antikörper erfolgen, der spezifisch an das Tumor-assoziierte Antigen oder den Teil davon bindet.

In weiteren Ausführungsformen liegt das nachzuweisende Tumor-assoziierte Antigen oder der Teil davon in einem Komplex mit einem MHC-Molekül, insbesondere einem HLA-Molekül vor.

Ein Nachweis eines Antikörpers oder die Überwachung der Menge an Antikörpern kann mit einem Protein oder Peptid erfolgen, das spezifisch an den Antikörper bindet.

Ein Nachweis von cytolytischen T-Zellen oder Helfer-T-Zellen oder die Überwachung der Menge an cytolytischen T-Zellen oder Helfer-T-Zellen, die für Komplexe zwischen einem Antigen oder einem Teil davon und MHC-Molekülen spezifisch sind, kann mit einer Zelle erfolgen, die den Komplex zwischen dem Antigen oder dem Teil davon und einem MHC-Molekül präsentiert.

Die für einen Nachweis oder für eine Überwachung verwendete Polynukleotid-Sonde, der Antikörper, das Protein oder Peptid oder die Zelle sind vorzugsweise nachweisbar markiert. In bestimmten Ausführungsformen ist der nachweisbare Marker ein radioaktiver Marker oder ein Enzymmarker. Der Nachweis von T-Lymphozyten kann zusätzlich erfolgen durch Nachweis ihrer Proliferation, ihrer Zytokinproduktion, sowie ihret cytotoxischen Aktivität, die ausgelöst wird durch die spezifische Stimulation mit dem Komplex aus MHC und tumorassoziiertem Antigen oder Teilen davon. Der Nachweis von T-Lymphozyten kann ferner erfolgen durch ein rekombinantes MHC-Molekül oder auch ein Komplex aus mehreren MHC-Molekülen, die beladen sind mit dem jeweiligen immunogenen Fragment aus einem oder mehreren der tumorassoziierten Antigene und durch Kontaktierung des spezifischen T-Zell-Rezeptors die spezifischen T-Lymphozyten identifizieren können.

In einem weiteren Aspekt betrifft die vorliegende technische Lehre ein Verfahren zur Behandlung, Diagnose oder Überwachung einer Erkrankung, die sich durch die Expression oder abnormale Expression eines erfindungsgemäß identifizierten Tumor-assoziierten Antigens auszeichnet, umfassend die Verabreichung eines Antikörpers, der an das Tumorassoziierte Antigen oder einen Teil davon bindet und mit einem therapeutischen oder diagnostischen Mittel gekoppelt ist. Der Antikörper kann ein monoklonaler Antikörper sein. In weiteren Ausführungsformen ist der Antikörper ein chimärer oder humanisierter Antikörper oder ein Fragment eines natürlichen Antikörpers.

Die Offenbarung betrifft auch ein Verfahren zur Behandlung eines Patienten mit einer Erkrankung, die sich durch die Expression oder abnormale Expression eines erfindungsgemäß identifizierten Tumor-assoziierten Antigens auszeichnet, umfassend (i) die Entfernung einer Probe mit immunreaktiver Zellen aus dem Patienten, (ii) die Kontaktierung der Probe mit einer Wirtszelle, die das Tumor-assoziierte Antigen oder einen Teil davon exprimiert, unter Bedingungen, die eine Produktion cytolytischer T-Zellen gegen das Tumor-assoziierte Antigen oder einen Teil davon begünstigen, und (iii) das Einbringen der cytolytischen T-Zellen in den Patienten in einer Menge, die geeignet ist, Zellen zu lysieren, die das Tumorassoziierte Antigen oder einen Teil davon exprimieren. Die Offenbarung betrifft ebenfalls die Klonierung des T-Zell-Rezeptors von cytolytischen T-Zellen gegen das Tumor-assoziierte Antigen. Dieser kann in andere T-Zellen transferiert werden, die damit die erwünschte Spezifität erhalten und wie unter (iii) in den Patienten eingebracht werden können.

In einer Ausführungsform exprimiert die Wirtszelle ein HLA-Molekül endogen. In einer weiteren Ausführungsform exprimiert die Wirtszelle ein HLA-Molekül und/oder das Tumorassoziierte Antigen oder den Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

In einem weiteren Aspekt betrifft die offenbarte technische Lehre ein Verfahren zur Behandlung eines Patienten mit einer Erkrankung, die sich durch die Expression oder abnormale Expression eines Tumor-assoziierten Antigens auszeichnet, umfassend (i) die Identifizierung einer für ein erfindungsgemäß identifiziertes Tumor-assoziiertes Antigen kodierenden Nukleinsäure, die von Zellen exprimiert wird, die mit der Erkrankung assoziiert sind, (ii) die Transfektion einer Wirtszelle mit der Nukleinsäure oder einem Teil davon, (iii) die Kultivierung der transfizierten Wirtszelle für eine Expression der Nukleinsäure (dies ist bei Erreichen einer hohen Transfektionsrate nicht obligat), und (iv) das Einbringen der Wirtszellen oder eines Extrakts davon in den Patienten in einer Menge, die geeignet ist, die Immunreaktion gegen die Zellen des Patienten, die mit der Erkrankung assoziiert sind, zu erhöhen. Das Verfahren kann ferner die Identifizierung eines MHC-Moleküls, das das Tumorassoziierte Antigen oder einen Teil davon präsentiert, umfassen, wobei die Wirtszelle das identifizierte MHC-Molekül exprimiert und das Tumor-assoziierte Antigen oder einen Teil davon präsentiert. Die Immunreaktion kann eine B-Zellen-Reaktion oder eine T-Zellen-Reaktion umfassen. Des weiteren kann eine T-Zellen-Reaktion die Produktion von cytolytischen T-Zellen und/oder Helfer-T-Zellen umfassen, die spezifisch für die Wirtszellen sind, die das Tumor-assoziierte Antigen oder einen Teil davon präsentieren oder spezifisch für Zellen des Patienten sind, die das Tumor-assoziierte Antigen oder einen Teil davon exprimieren.

Die offenbarte Lehre betrifft auch ein Verfahren zur Behandlung einer Erkrankung, die sich durch die Expression oder abnormale Expression eines erfindungsgemäß identifizierten Tumor-assoziierten Antigens auszeichnet, umfassend (i) die Identifikation von Zellen aus dem Patienten, die abnormale Mengen des Tumor-assoziierten Antigens exprimieren, (ii) die Isolierung einer Probe der Zellen, (iii) die Kultivierung der Zellen und (iv) das Einbringen der Zellen in den Patienten in einer Menge, die geeignet ist, eine Immunreaktion gegen die Zellen auszulösen.

Vorzugsweise sind die verwendeten Wirtszellen nicht-proliferativ oder werden nicht-proliferativ gemacht. Eine Erkrankung, die sich durch die Expression oder abnormale Expression eines Tumor-assoziierten Antigens auszeichnet, ist insbesondere Krebs.

Des weiteren betrifft die vorliegend offenbarte Lehre eine Nukleinsäure, die aus der Gruppe ausgewählt ist, bestehend aus (a) einer Nukleinsäure, die eine Nukleinsäuresequenz umfasst, die aus der Gruppe bestehend aus SEQ ID NOs: 20-21 und 54-57 einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist und (d) einer Nukleinsäure, die zu der Nukleinsäure unter (a), (b) oder (c) komplementär ist. Des weiteren betrifft die Lehre eine Nukleinsäure die für ein Protein oder Polypeptid kodiert, das eine Aminosäuresequenz umfasst, ausgewählt aus der Gruppe bestehend aus SEQ ID NOs: 22-24, 58-61, 81, 82 und 103-105, einem Teil oder Derivat davon.

In einem weiteren Aspekt betrifft die vorliegende Lehre Promotorsequenzen von erfindungsgemäßen Nukleinsäuren. Diese können funktionell mit einem anderen Gen vorzugsweise in einem Expressionsvektor verbunden werden, und somit die selektive Expression dieses Gens in entsprechenden Zellen gewährleisten.

In einem weiteren Aspekt betrifft die vorliegende Lehre ein rekombinantes Nukleinsäuremolekül, insbesondere DNA- oder RNA-Molekül, das eine erfindungsgemäße Nukleinsäure umfasst.

Die Offenbarung betrifft auch Wirtszellen, die eine erfindungsgemäße Nukleinsäure oder ein rekombinantes Nukleinsäuremolekül, das eine erfindungsgemäße Nukleinsäure umfasst, enthalten.

Die Wirtszelle kann ferner eine Nukleinsäure umfassen, die für ein HLA-Molekül kodiert. In einer Ausführungsform exprimiert die Wirtszelle das HLA-Molekül endogen. In einer weiteren Ausführungsform exprimiert die Wirtszelle das HLA-Molekül und/oder die erfindungsgemäße Nukleinsäure oder einen Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

In einer weiteren Ausführungsform betrifft die vorliegende Lehre auch Oligonukleotide, die mit einer erfindungsgemäß identifizierten Nukleinsäure hybridisieren und als genetische Sonden oder als "Antisense"-Moleküle verwendet werden können. Nukleinsäuremoleküle in der Form von Oligonukleotid-Primern oder kompetenten Proben, die mit einer erfindungsgemäß identifizierten Nukleinsäure oder Teilen davon hybridisieren, können zum Auffinden von Nukleinsäuren verwendet werden, die zu der erfindungsgemäß identifizierten Nukleinsäure homolog sind. PCR-Amplifikation, Southern- und Northern-Hybridisierung können zum Auffinden homologer Nukleinsäuren eingesetzt werden. Die Hybridisierung kann unter niedrig-, besser unter mittel- und am besten unter hoch-stringenten Bedingungen erfolgen. Der Begriff "stringente Bedingungen" betrifft erfindungsgemäß Bedingungen, die eine spezifische Hybridisierung zwischen Polynukleotiden erlauben.

In einem weiteren Aspekt betrifft die vorliegende Offenbarung ein Protein oder Polypeptid, das von einer Nukleinsäure kodiert wird, die aus der Gruppe ausgewählt ist, bestehend aus (a) einer Nukleinsäure, die eine Nukleinsäuresequenz umfasst, die aus der Gruppe bestehend aus SEQ ID NOs: 20-21 und 54-57 einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist und (d) einer Nukleinsäure, die zu der Nukleinsäure unter (a), (b) oder (c) komplementär ist. In einer bevorzugten Ausführungsform betrifft die Offenbarung ein Protein oder Polypeptid, das eine Aminosäuresequenz umfasst, ausgewählt aus der Gruppe bestehend aus SEQ ID NOs: 23-24 und 58-61, 103-105, einem Teil oder Derivat davon.

In einem weiteren Aspekt betrifft die vorliegende Lehre ein immunogenes Fragment eines erfindungsgemäß identifizierten Tumor-assoziierten Antigens. Das Fragment bindet vorzugsweise an einen menschlichen HLA-Rezeptor oder menschlichen Antikörper. Vorzugsweise umfasst ein offenbarungsgemäßesFragment eine Sequenz von mindestens 6, insbesondere mindestens 8, mindestens 10, mindestens 12, mindestens 15, mindestens 20, mindestens 30 oder mindestens 50 Aminosäuren.

In einem weiteren Aspekt betrifft die offenbarte Lehre ein Mittel, das an ein erfindungsgemäß identifiziertes Tumor-assoziiertes Antigen oder an einen Teil davon bindet. In einer bevorzugten Ausführungsform ist das Mittel ein Antikörper. In weiteren Ausführungsformen ist der Antikörper ein chimärer, ein humanisierter oder mit kombinatorischen Techniken hergestellte Antikörper oder ein Fragment eines Antikörpers. Des weiteren betrifft die Offenbarung einen Antikörper, der selektiv an einen Komplex aus (i) einem erfindungsgemäß identifizierten Tumor-assoziierten Antigen oder einem Teil davon und (ii) einem MHC-Molekül bindet, an das das erfindungsgemäß identifizierte Tumor-assoziierte Antigen oder der Teil davon bindet, wobei der Antiköper nicht alleine an (i) oder (ii) bindet. Ein erfindungsgemäßer Antikörper kann ein monoklonaler Antikörper sein. In weiteren Ausführungsformen ist der Antikörper ein chimärer oder humanisierter Antikörper oder ein Fragment eines natürlichen Antikörpers.

Des weiteren betrifft die offfenbarte technische Lehre ein Konjugat zwischen einem offenbarungsgemäßen Mittel, das an ein erfindungsgemäß identifiziertes Tumor-assoziiertes Antigen oder an einen Teil davon bindet, oder einem offenbarungsgemäßen Antikörper und einem therapeutischen oder diagnostischen Mittel. In einer Ausführungsform ist das therapeutische oder diagnostische Mittel ein Toxin.

In einem weiteren Aspekt betrifft die Offenbarung einen Kit zum Nachweis der Expression oder abnormalen Expression eines erfindungsgemäß identifizierten Tumor-assoziierten Antigens, umfassend Mittel zum Nachweis (i) der Nukleinsäure, die für das Tumorassoziierte Antigen kodiert, oder eines Teils davon, (ii) des Tumor-assoziierten Antigens oder eines Teils davon, (iii) von Antikörpern, die an das Tumor-assoziierte Antigen oder einen Teil davon binden, und/oder (iv) von T-Zellen, die für einen Komplex zwischen dem Tumorassoziierten Antigen oder einem Teil davon und einem MHC-Molekül spezifisch sind. In einer Ausführungsform sind die Mittel zum Nachweis der Nukleinsäure oder des Teils davon Nukleinsäuremoleküle für die selektive Amplifikation der Nukleinsäure, die insbesondere eine Sequenz von 6-50, insbesondere 10-30, 15-30 und 20-30 zusammenhängenden Nukleotiden aus der Nukleinsäure umfassen.

### Detaillierte Beschreibung der Erfindung

Erfindungsgemäß werden Gene beschrieben, die in Tumorzellen selektiv exprimiert oder aberrant exprimiert werden und Tumor-assoziierte Antigene darstellen.

Erfindungsgemäß sind diese Gene oder ihre Derivate bevorzugte Zielstrukturen für therapeutische Ansätze. Konzeptionell können die therapeutischen Ansätze auf eine Hemmung der Aktivität des selektiv exprimierten tumor-assoziierten Genproduktes zielen. Dies ist dann sinnvoll, wenn die aberrante respektive selektive Expression funktionell von tumorpathogenetischer Bedeutung ist und ihre Unterbindung mit einer selektiven Schädigung der entsprechenden Zellen einhergeht. Andere therapeutische Konzepte betrachten tumorassoziierte Antigene als Markierungen, die Effektormechanismen mit zellschädigendem Potential selektiv zu Tumorzellen rekrutieren. Hierbei ist die Funktion des Zielmoleküls selbst und seine Rolle bei der Tumorentstehung vollkommen unerheblich.

Mit "Derivat" einer Nukleinsäure ist erfindungsgemäß gemeint, dass einzelne oder multiple Nukleotidsubstitution, -deletion und/oder -addition in der Nukleinsäure vorliegen. Weiterhin umfasst der Begriff "Derivat" auch eine chemische Derivatisierung einer Nukleinsäure an einer Nukleotidbase, am Zucker oder am Phosphat. Der Begriff "Derivat" umfasst auch Nukleinsäuren, die nicht in der Natur vorkommende Nukleotide und Nukleotidanaloga enthalten.

Eine Nukleinsäure ist vorzugsweise Desoxyribonukleinsäure (DNA) oder Ribonukleinsäure (RNA). Nukleinsäuren umfassen erfindungsgemäß genomische DNA, cDNA, mRNA, rekombinant hergestellte und chemisch synthetisierte Moleküle. Eine Nukleinsäure kann als einzelsträngiges oder doppelsträngiges und lineares oder kovalent kreisförmig geschlossenes Molekül vorliegen.

Die beschriebenen Nukleinsäuren sind vorzugsweise isoliert. Der Begriff "isolierte Nukleinsäure" bedeutet erfindungsgemäß, dass die Nukleinsäure (i) *in vitro* amplifiziert wurde, zum Beispiel durch Polymerase-Kettenreaktion (PCR), (ii) rekombinant durch Klonierung produziert wurde, (iii) gereinigt wurde, zum Beispiel durch Spaltung und gelelektrophoretische Auftrennung oder (iv) synthetisiert wurde, zum Beispiel durch chemische Synthese. Eine isolierte Nukleinsäure ist eine Nukleinsäure, die für eine Manipulierung durch rekombinante DNA-Techniken zur Verfügung steht.

Eine Nukleinsäure ist dann zu einer anderen Nukleinsäure "komplementär", wenn die beiden Sequenzen miteinander hybridisieren und ein stabiles Duplex eingehen können, wobei die Hybridisierung vorzugsweise unter Bedingungen erfolgt, die eine spezifische Hybridisierung zwischen Polynukleotiden erlauben (stringente Bedingungen). Stringente Bedingungen sind beispielsweise in Molecular Cloning: A Laboratory Manual, J. Sambrook et al., Hrsg., 2. Auflage, Cold Spring Harbor Laboratory Press, Cold Spring Harbor, New York, 1989 oder Current Protocols in Molecular Biology, F.M. Ausubel et al., Hrsg., John Wiley & Sons, Inc., New York beschrieben und betreffen beispielsweise die Hybridisierung bei 65°C in Hybridisierungspuffer (3,5 x SSC, 0,02% Ficoll, 0,02% Polyvinylpyrrolidon, 0,02% Rinderserumalbumin, 2,5mM NaH₂PO₄ (pH7), 0,5% SDS, 2mM EDTA). SSC ist 0,15 M Natriumchlorid/ 0,15 M Natriumcitrat, pH 7. Nach der Hybridisierung wird die Membran, auf die die DNA übertragen wurde beispielsweise in 2 x SSC bei Raumtemperatur und sodann in 0,1 - 0,5 x SSC/ 0,1 x SDS bei Temperaturen bis 68°C gewaschen.

Komplementäre Nukleinsäuren weisen erfindungsgemäß mindestens 40%, insbesondere mindestens 50%, mindestens 60%, mindestens 70%, mindestens 80%, mindestens 90% und vorzugsweise mindestens 95%, mindestens 98 oder mindestens 99% Identität der Nukleotide auf.

Nukleinsäuren, die für Tumor-assoziierte Antigene kodieren, können alleine oder in Kombination mit anderen Nukleinsäuren, insbesondere heterologen Nukleinsäuren, vorliegen. In bevorzugten Ausführungsformen liegt eine Nukleinsäure funktionell in Verbindung mit Expressionskontrollsequenzen oder regulatorischen Sequenzen vor, die in Bezug zu der Nukleinsäure homolog oder heterolog sein können. Eine kodierende Sequenz und eine regulatorische Sequenz sind dann "funktionell" miteinander verbunden, falls sie derart kovalent miteinander verknüpft sind, dass die Expression oder Transkription der kodierenden Sequenz unter der Kontrolle oder unter dem Einfluss der regulatorischen Sequenz steht. Falls die kodierende Sequenz in ein funktionelles Protein translatiert werden soll, führt bei einer funktionellen Verbindung einer regulatorischen Sequenz mit der kodierenden Sequenz eine Induktion der regulatorischen Sequenz zu einer Transkription der kodierenden Sequenz, ohne dass es zu einer Leserasterverschiebung in der kodierenden Sequenz oder zu einem Unvermögen der kodierenden Sequenz kommt, in das gewünschte Protein oder Peptid translatiert zu werden.

Der Begriff "Expressionskontrollsequenz" oder "regulatorische Sequenz" umfasst Promotoren, Enhancer und andere Kontrollelemente, die die Expression eines Gens steuern. In bestimmten Ausführungsformen sind die Expressionskontrollsequenzen regulierbar. Die genaue Struktur von regulatorischen Sequenzen kann speziesabhängig oder zelltypusabhängig variieren, umfasst jedoch im allgemeinen 5'-nicht-transkribierte und 5'-nicht-translatierte Sequenzen, die an der Initiation der Transkription bzw. Translation beteiligt sind wie TATA-Box, Capping-Sequenz, CAAT-Sequenz und ähnliches. Insbesondere umfassen 5'-nicht-transkribierte Regulationssequenzen eine Promotorregion, die eine Promotorsequenz für eine transkriptionelle Kontrolle des funktionell verbundenen Gens einschließt. Regulatorische Sequenzen können auch Enhancer-Sequenzen oder stromaufwärts gelegene Aktivatorsequenzen umfassen.

Zum einen können also die hier dargestellten tumorassoziierten Antigene mit beliebigen Expressionskontrollsequenzen und Promotoren kombiniert werden. Zum anderen aber können die Promotoren der hier dargestellten tumor-assoziierten Genprodukte mit beliebigen anderen Genen kombiniert werden. Dies erlaubt, die selektive Aktivität dieser Promotoren zu nutzen.

Desweiteren kann eine Nukleinsäure in Verbindung mit einer anderen Nukleinsäure vorliegen, die für ein Polypeptid kodiert, das eine Sekretion des durch die Nukleinsäure kodierten Proteins oder Polypeptids aus einer Wirtszelle steuert. Auch kann eine Nukleinsäure in Verbindung mit einer anderen Nukleinsäure vorliegen, die für ein Polypeptid kodiert, das eine Verankerung des kodierten Proteins oder Polypeptids auf der Zellmembran der Wirtszelle oder seine Kompartimentalisierung in bestimmte Organellen dieser Zelle herbeiführt.

In einer bevorzugten Ausführungsform ist ein rekombinantes DNA-Molekül ein Vektor, gegebenenfalls mit einem Promotor, der die Expression einer Nukleinsäure, z.B. einer Nukleinsäure, die für ein erfindungsgemäßes Tumor-assoziiertes Antigen kodiert, steuert. Der Begriff "Vektor" wird dabei in seiner allgemeinsten Bedeutung verwendet und umfasst jegliche intermediären Vehikel für eine Nukleinsäure, die es z.B. ermöglichen die Nukleinsäure in prokaryotische und/oder in eukaryotische Zellen einzubringen und gegebenenfalls in ein Genom zu integrieren. Solche Vektoren werden vorzugsweise in der Zelle repliziert und/oder exprimiert. Ein intermediäres Vehikel kann z.B. für den Gebrauch bei der Elektroporation, beim Mikroprojektilbeschuss, bei der liposomalen Verabreichung, beim Transfer mit Hilfe von Agrobakterien oder bei der Insertion über DNA- oder RNA-Viren angepasst sein. Vektoren umfassen Plasmide, Phagemide oder Virusgenome.

Die Nukleinsäuren, die für ein erfindungsgemäß identifiziertes Tumor-assoziiertes Antigen kodieren, können für eine Transfektion von Wirtszellen eingesetzt werden. Mit Nukleinsäuren ist dabei sowohl rekombinante DNA wie auch RNA gemeint. Rekombinante RNA kann durch in-vitro-Transkription von einer DNA-Matritze hergestellt werden. Sie kann desweiteren vor Applikation durch stabilisierende Sequenzen, Capping und Poly-Adenylierung modifiziert werden. Der Begriff "Wirtszelle" betrifft erfindungsgemäß jede Zelle, die mit einer exogenen Nukleinsäure transformierbar oder transfizierbar ist. Der Begriff "Wirtszellen" umfasst erfindungsgemäß prokaryontische (z.B. *E. coli*) oder eukaryontische (z.B. dendritische Zellen, B-Zellen, CHO-Zellen, COS-Zellen, K562-Zellen, Hefezellen und Insektenzellen). Besonders bevorzugt sind Säugerzellen wie Zellen aus Mensch, Maus, Hamster, Schwein, Ziege, Primaten. Die Zellen können aus einer Vielzahl von Gewebetypen abgeleitet sein und umfassen primäre Zellen und Zelllinien. Spezifische Beispiele umfassen Keratinozyten, periphere Blutleukozyten, Stammzellen des Knochenmarks und embryonale Stammzellen. In weiteren Ausführungsformen ist die Wirtszelle eine Antigen-präsentierende Zelle, insbesondere eine dendritische Zelle, Monozyt oder ein Makrophage. Eine Nukleinsäure kann in der Wirtszelle in einer einzigen oder in mehreren Kopien vorliegen und wird in einer Ausführungsform in der Wirtszelle exprimiert.

Der Begriff "Expression" wird erfindungsgemäß in seiner allgemeinsten Bedeutung verwendet und umfasst die Produktion von RNA oder von RNA und Protein. Er umfasst auch eine teilweise Expression von Nukleinsäuren. Desweiteren kann die Expression transient oder stabil erfolgen. Bevorzugte Expressionssysteme in Säugerzellen umfassen pcDNA3.1 und pRc/CMV (Invitrogen, Carlsbad, CA), die einen selektierbaren Marker enthalten wie ein Gen, das eine Resistenz gegenüber G418 verleiht (und somit eine Selektion stabil transfizierter Zelllinien ermöglicht) und die Enhancer-Promotor-Sequenzen von Cytomegalovirus (CMV). In den Fällen der Erfindung, in denen ein HLA-Molekül ein Tumor-assoziiertes Antigen oder einen Teil davon präsentiert, kann ein Expressionsvektor auch eine Nukleinsäuresequenz umfassen, das für das HLA-Molekül kodiert. Die Nukleinsäuresequenz, die für das HLA-Molekül kodiert, kann auf demselben Expressionsvektor wie die Nukleinsäure, die für das Tumor-assoziierte Antigen oder den Teil davon kodiert, vorliegen oder beide Nukleinsäuren können auf verschiedenen Expressionsvektoren vorliegen. Im letzteren Fall können die beiden Expressionsvektoren in eine Zelle cotransfiziert werden. Falls eine Wirtszelle weder das Tumor-assoziierte Antigen oder den Teil davon noch das HLA-Molekül exprimiert, werden beide dafür kodierenden Nukleinsäuren entweder auf demselben Expressionsvektor oder auf verschiedenen Expressionsvektoren in die Zelle transfiziert. Falls die Zelle bereits das HLA-Molekül exprimiert, kann nur die Nukleinsäuresequenz, die für das Tumor-assoziierte Antigen oder den Teil davon kodiert, in die Zelle transfiziert werden.

Die Offenbarung umfasst auch Kits zur Amplifikation einer Nukleinsäure, die für ein Tumor-assoziiertes Antigen kodiert. Solche Kits umfassen beispielsweise ein Paar von Amplifikationsprimern, die an die Nukleinsäure hybridisieren, die für das Tumor-assoziierte Antigen kodiert. Die Primer umfassen vorzugsweise eine Sequenz von 6-50, insbesondere 10-30, 15-30 und 20-30 zusammenhängenden Nukleotiden aus der Nukleinsäure und sind nichtüberlappend, um die Bildung von Primer-Dimeren zu vermeiden. Einer der Primer wird an einen Strang der Nukleinsäure hybridisieren, die für das Tumor-assoziierte Antigen kodiert, und der andere Primer wird an den komplementären Strang in einer Anordnung hybridisieren, die eine Amplifikation der Nukleinsäure, die für das Tumor-assoziierte Antigen kodiert, erlaubt.

"Antisense"-Moleküle oder "Antisense"-Nukleinsäuren können zur Regulierung, insbesondere der Reduktion der Expression einer Nukleinsäure verwendet werden. Der Begriff "Antisense-Molekül" oder "Antisense-Nukleinsäure" betrifft gemäß der offenbarten technischen Lehre ein Oligonukleotid, das ein Oligoribonukleotid, Oligodesoxyribonukleotid, modifiziertes Oligoribonukleotid oder modifiziertes Oligodesoxyribonukleotid ist und das unter physiologischen Bedingungen an DNA, die ein bestimmtes Gen umfasst, oder mRNA dieses Gens hybridisiert, wodurch die Transkription dieses Gens und/oder die Translation dieser mRNA gehemmt wird. Ein "Antisense-Molekül" umfasst auch ein Konstrukt, das eine Nukleinsäure oder einen Teil davon in reverser Orientierung in Bezug auf ihren natürlichen Promotor enthält. Ein Antisense-Transkript einer Nukleinsäure oder eines Teils davon kann eine Duplex mit der natürlich vorkommenden mRNA, die das Enzym spezifiziert, eingehen und so eine Akkumulation von oder die Translation der mRNA in das aktive Enzym verhindern. Eine weitere Möglichkeit ist die Verwendung von Ribozymen zur Inaktivierung einer Nukleinsäure. Gemäß der offenbarten Lehre weisen bevorzugte Antisense-Oligonukleotide eine Sequenz von 6-50, insbesondere 10-30, 15-30 und 20-30 zusammenhängenden Nukleotiden aus der Ziel-Nukleinsäure auf und sind vorzugsweise vollständig zu der Ziel-Nukleinsäure oder einem Teil davon komplementär.

In bevorzugten Ausführungsformen hybridisiert das Antisense-Oligonukleotid mit einer N-terminalen oder 5'-stromaufwärts gelegenen Stelle wie einer Translationsinitiations-, Transkriptionsinitiations- oder Promotorstelle. In weiteren Ausführungsformen hybridisiert das Antisense-Oligonukleotid mit einer 3'-nicht-translatierten Region oder mRNA-Splicing-Stelle.

In einer Ausführungsform besteht gemäß der offenbarten Lehre ein Oligonukleotid aus Ribonukleotiden, Desoxyribonukleotiden oder einer Kombination davon. Dabei sind das 5'-Ende eines Nukleotids und das 3'-Ende eines anderen Nukleotids durch eine Phosphodiesterbindung miteinander verknüpft. Diese Oligonukleotide können in herkömmlicher Weise synthetisiert oder rekombinant produziert werden.

In bevorzugten Ausführungsformen ist ein Oligonukleotid ein "modifiziertes" Oligonukleotid. Dabei kann das Oligonukleotid, um beispielsweise seine Stabilität oder therapeutische Wirksamkeit zu erhöhen, auf verschiedenste Art und Weise modifiziert sein ohne dass seine Fähigkeit, an sein Ziel zu binden, beeinträchtigt wird. Der Begriff "modifiziertes Oligonukleotid" bedeutet erfindungsgemäß ein Oligonukleotid bei dem (i) mindestens zwei seiner Nukleotide durch eine synthetische Internukleosidbindung (d.h. eine Internukleosidbindung, die keine Phosphodiesterbindung ist) miteinander verknüpft sind und/oder (ii) eine chemische Gruppe kovalent mit dem Oligonukleotid verbunden ist, die normalerweise nicht bei Nukleinsäuren auftritt. Bevorzugte synthetische Internukleosidbindungen sind Phosphorothioate, Alkylphosphonate, Phosphorodithioate, Phosphatester, Alkylphosphonothioate, Phosphoramidate, Carbamate, Carbonate, Phosphattriester, Acetamidate, Carboxymethylester und Peptide.

Der Begriff "modifiziertes Oligonukleotid" umfasst auch Oligonukleotide mit einer kovalent modifizierten Base und/oder Zucker. "Modifizierte Oligonukleotide" umfassen beispielsweise Oligonukleotide mit Zuckerresten, die kovalent an organische Gruppen mit einem geringen Molekulargewicht gebunden sind, die keine Hydroxylgruppe an der 3'-Position und keine Phosphatgruppe an der 5'-Position sind. Modifizierte Oligonukleotide können beispielsweise einen 2'-O-alkylierten Riboserest oder einen anderen Zucker anstelle von Ribose wie Arabinose umfassen.

Die erfindungsgemäß beschriebenen Proteine und Polypeptide sind vorzugsweise isoliert. Die Begriffe "isoliertes Protein" oder "isoliertes Polypeptid" bedeuten, dass das Protein oder Polypeptid von seiner natürlichen Umgebung getrennt ist. Ein isoliertes Protein oder Polypeptid kann in einem im Wesentlichen aufgereinigten Zustand vorliegen. Der Begriff "im wesentlichen aufgereinigt" bedeutet, dass das Protein oder Polypeptid im Wesentlichen frei von anderen Substanzen vorliegt, mit denen es in der Natur oder *in vivo* vorliegt.

Solche Proteine und Polypeptide dienen beispielsweise der Herstellung von Antikörpern und sind in einem immunologischen oder diagnostischen Assay oder als Therapeutika einsetzbar. Erfindungsgemäß beschriebene Proteine und Polypeptide können aus biologischen Proben wie Gewebe- oder Zellhomogenaten isoliert werden und können auch rekombinant in einer Vielzahl pro- oder eukaryontischer Expressionssysteme exprimiert werden.

"Derivate" eines Proteins oder Polypeptids oder einer Aminosäuresequenz im Sinne dieser Erfindung umfassen Aminosäure-Insertionsvarianten, Aminosäure-Deletionsvarianten und/oder Aminosäure-Substitutionsvarianten.

Aminosäure-Insertionsvarianten umfassen amino- und/oder carboxyterminale Fusionen, sowie Insertionen von einzelnen oder mehreren Aminosäuren in einer bestimmten Aminosäuresequenz. Bei Aminosäure-Sequenzvarianten mit einer Insertion werden ein oder mehrere Aminosäurereste in eine vorbestimmte Stelle in einer Aminosäuresequenz eingebracht, obwohl eine zufällige Insertion mit geeignetem Screening des resultierenden Produkts auch möglich ist. Aminosäure-Deletionsvarianten sind durch das Entfernen von einer oder mehreren Aminosäuren aus der Sequenz charakterisiert. Aminosäure-Substitutionsvarianten zeichnen sich dadurch aus, dass wenigstens ein Rest in der Sequenz entfernt und ein anderer Rest an dessen Stelle eingefügt wird. Vorzugsweise befinden sich die Modifikationen an Positionen in der Aminosäuresequenz, die zwischen homologen Proteinen oder Polypeptiden nicht konserviert sind. Vorzugsweise werden Aminosäuren durch andere mit ähnlichen Eigenschaften ersetzt, wie Hydrophobizität, Hydrophilizität, Elektronegativität, Volumen der Seitenkette und ähnliches (konservative Substitution). Konservative Substitutionen betreffen beispielsweise den Austausch einer Aminosäure durch eine andere, nachstehend in derselben Gruppe wie die substituierte Aminosäure aufgeführte Aminosäure:
1. kleine aliphatische, nicht-polare oder leicht-polare Reste: Ala, Ser, Thr (Pro, Gly)
2. negativ geladene Reste und ihre Amide: Asn, Asp, Glu, Gln
3. positiv geladene Reste: His, Arg, Lys
4. große aliphatische, nicht-polare Reste: Met, Leu, Ile, Val (Cys)
5. große aromatische Reste: Phe, Tyr, Trp.

Drei Reste sind aufgrund ihrer besonderen Rolle für die Proteinarchitektur in Klammern gesetzt. Gly ist der einzige Rest ohne eine Seitenkette und verleiht der Kette somit Flexibilität. Pro besitzt eine ungewöhnliche Geometrie, die die Kette stark einschränkt. Cys kann eine Disulfidbrücke bilden.

Die oben beschriebenen Aminosäure-Varianten können leicht mit Hilfe von bekannten Peptidsynthesetechniken wie z.B. durch "Solid Phase Synthesis" (Merrifield, 1964) und ähnliche Verfahren oder durch rekombinante DNA-Manipulation hergestellt werden. Techniken, um Substitutionsmutationen an vorbestimmten Stellen in DNA einzubringen, die eine bekannte oder teilweise bekannte Sequenz besitzt, sind gut bekannt und umfassen z.B. M13-Mutagenese. Die Manipulation von DNA-Sequenzen zur Herstellung von Proteinen mit Substitutionen, Insertionen oder Deletionen ist z.B. in Sambrook et. al. (1989) ausführlich beschrieben.

"Derivate" von Proteinen oder Polypeptiden umfassen auch einzelne oder multiple Substitutionen, Deletionen und/oder Additionen jeglicher Moleküle, die mit dem Enzym assoziiert sind, wie Kohlenhydrate, Lipide und/oder Proteine oder Polypeptide. Ferner erstreckt sich der Begriff "Derivat" auch auf alle funktionellen chemischen Äquivalente der Proteine oder Polypeptide.

Ein Teil oder Fragment eines Tumor-assoziierten Antigens weist offenbarungsgemäß eine funktionelle Eigenschaft des Polypeptids auf, aus dem es abgeleitet ist. Solche funktionellen Eigenschaften umfassen die Interaktion mit Antikörpern, die Interaktion mit anderen Polypeptiden oder Proteinen, die selektive Bindung von Nukleinsäuren und eine enzymatische Aktivität. Eine bedeutende Eigenschaft ist die Fähigkeit, einen Komplex mit HLA einzugehen und gegebenenfalls eine Immunreaktion zu erzeugen. Diese Immunreaktion kann auf Stimulation von cytotoxischen oder Helfer T-Zellen beruhen. Vorzugsweise umfasst ein offenbarungsgemäßer Teil oder Fragment eines Tumor-assoziierten Antigens eine Sequenz von mindestens 6, insbesondere mindestens 8, mindestens 10, mindestens 12, mindestens 15, mindestens 20, mindestens 30 oder mindestens 50 aufeinanderfolgenden Aminosäuren aus dem Tumor-assoziierten Antigen.

Bevorzugte Teile oder Fragmente eines Tumor-assoziierten Antigens weisen erfindungsgemäß vorzugsweise eine der nachstehend aufgeführten Sequenzen auf, die von den erfindungsgemäß identifizierten Tumor-assoziierten Antigenen abgeleitet sind, und sind vorzugsweise Peptidepitope, die erfindungsgemäß insbesondere zur Stimulierung von cytotoxischen T-Lymphozyten in vivo aber auch zur Herstellung expandierter und stimulierter T-Lymphozyten zum therapeutischen adoptiven Transfer ex vivo geeignet sind. Diese Sequenzen sind insbesondere Peptidepitope für die nachstehend angegebenen MHC Klasse I Allele:
HLA-A*0201
HLA-A*2402
HLA-A*01
HLA-A*03
HLA-B*0702

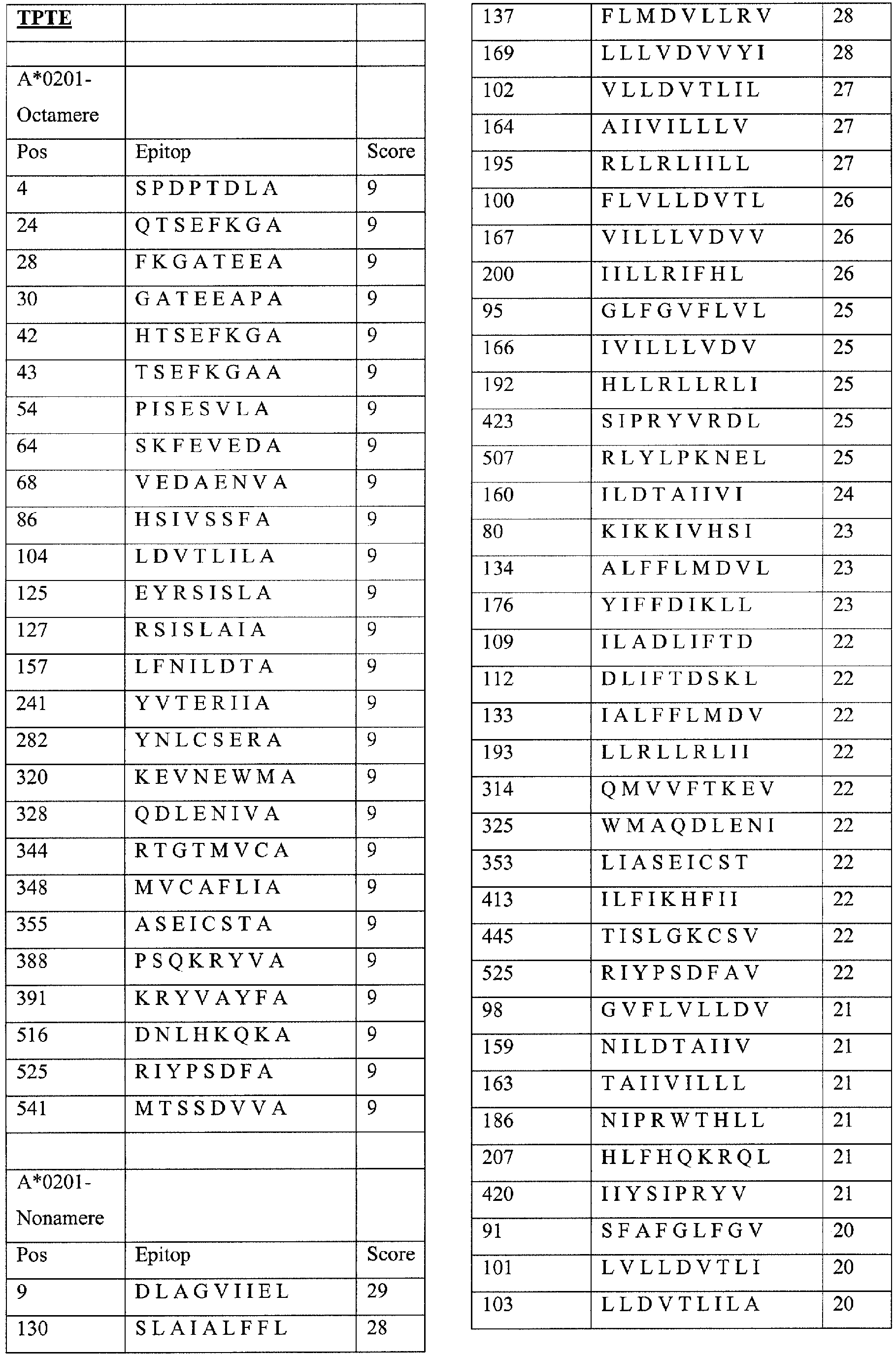

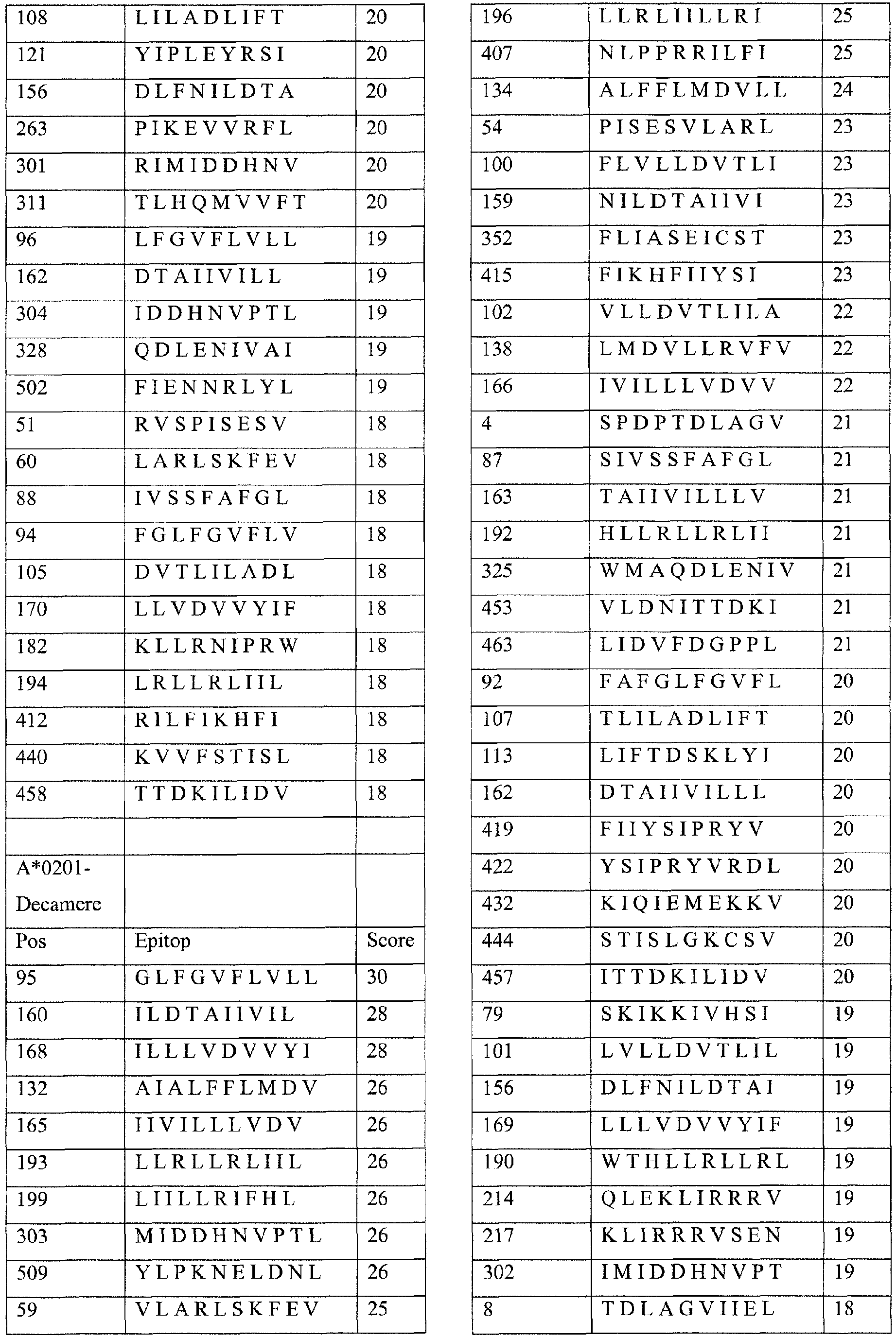

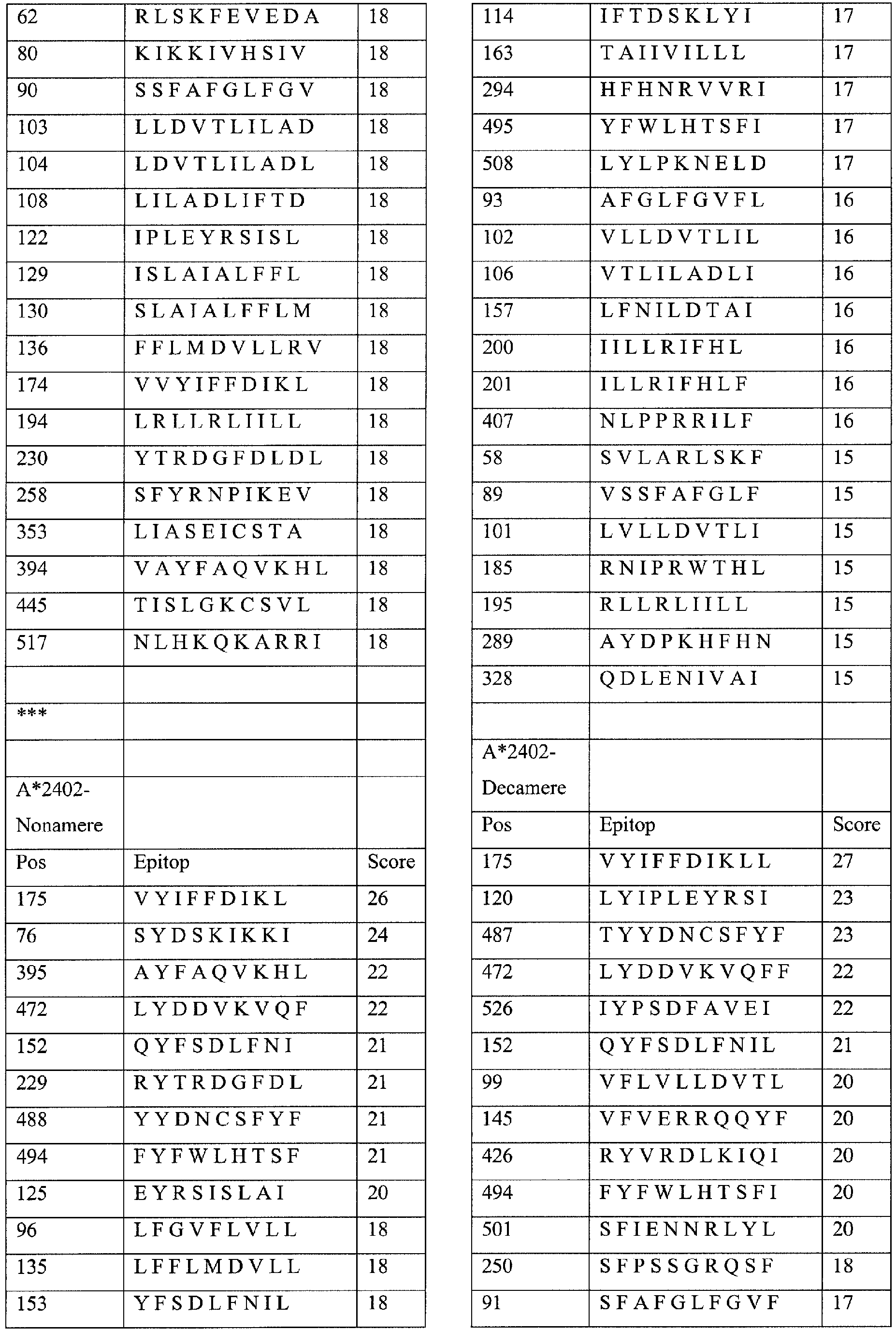

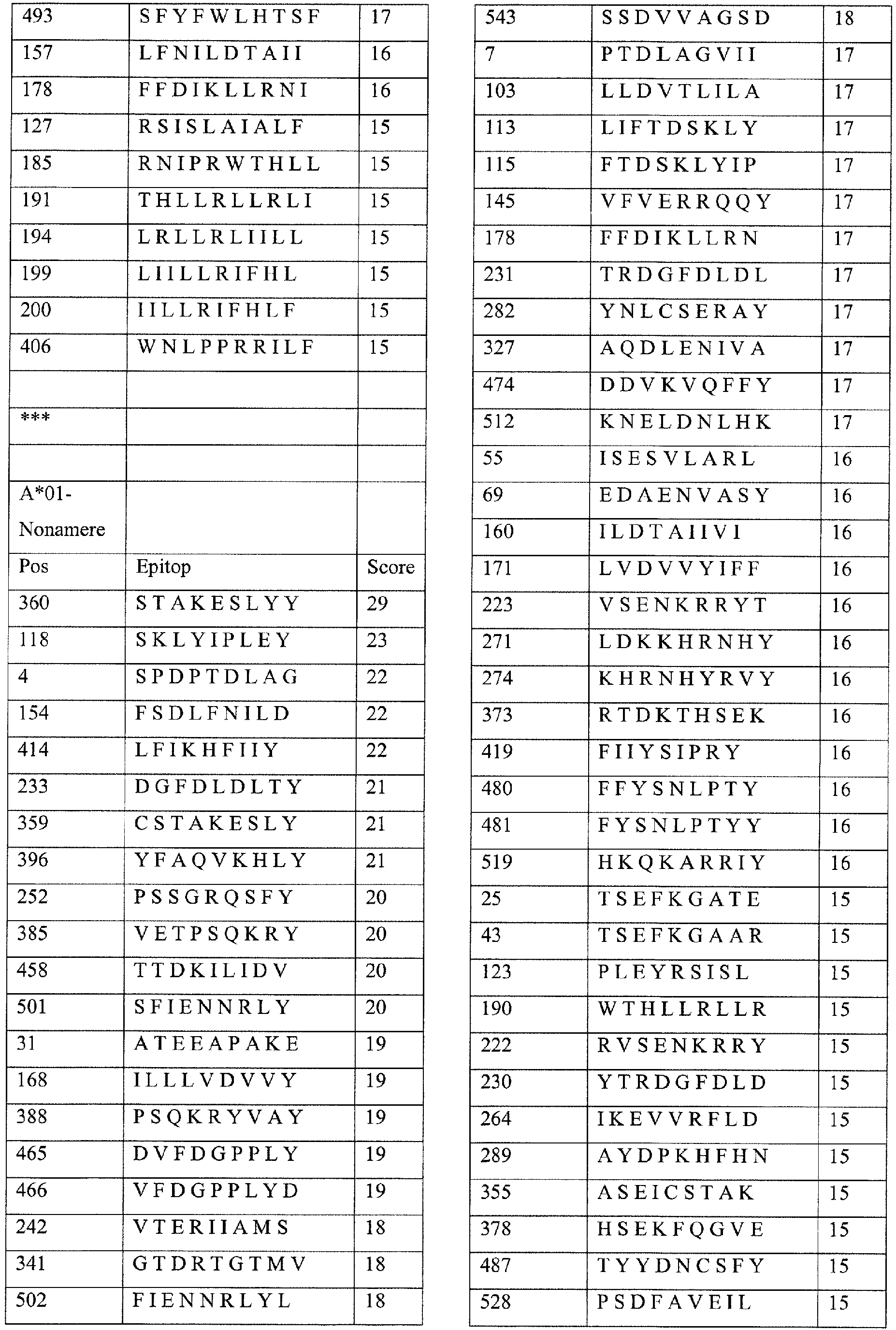

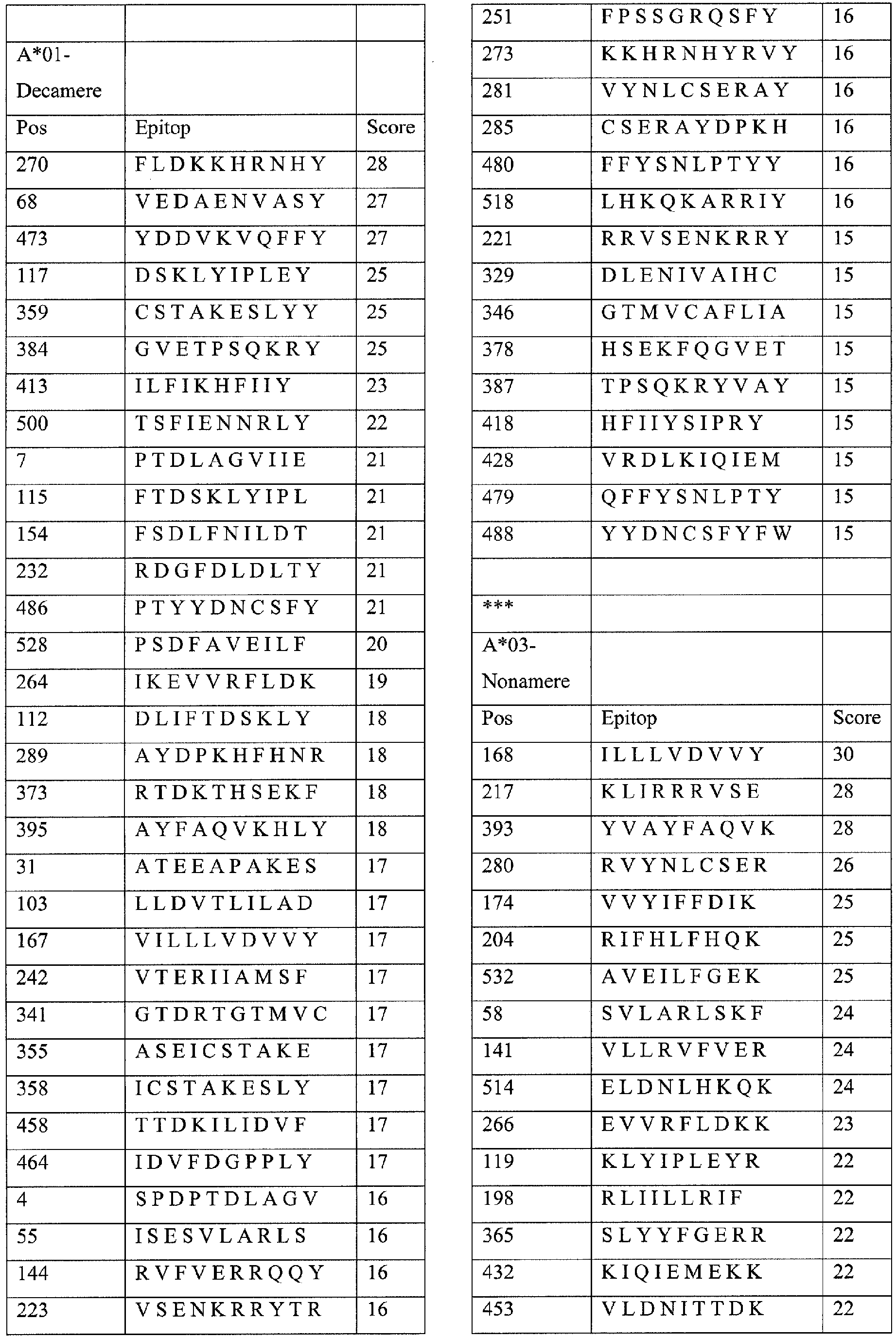

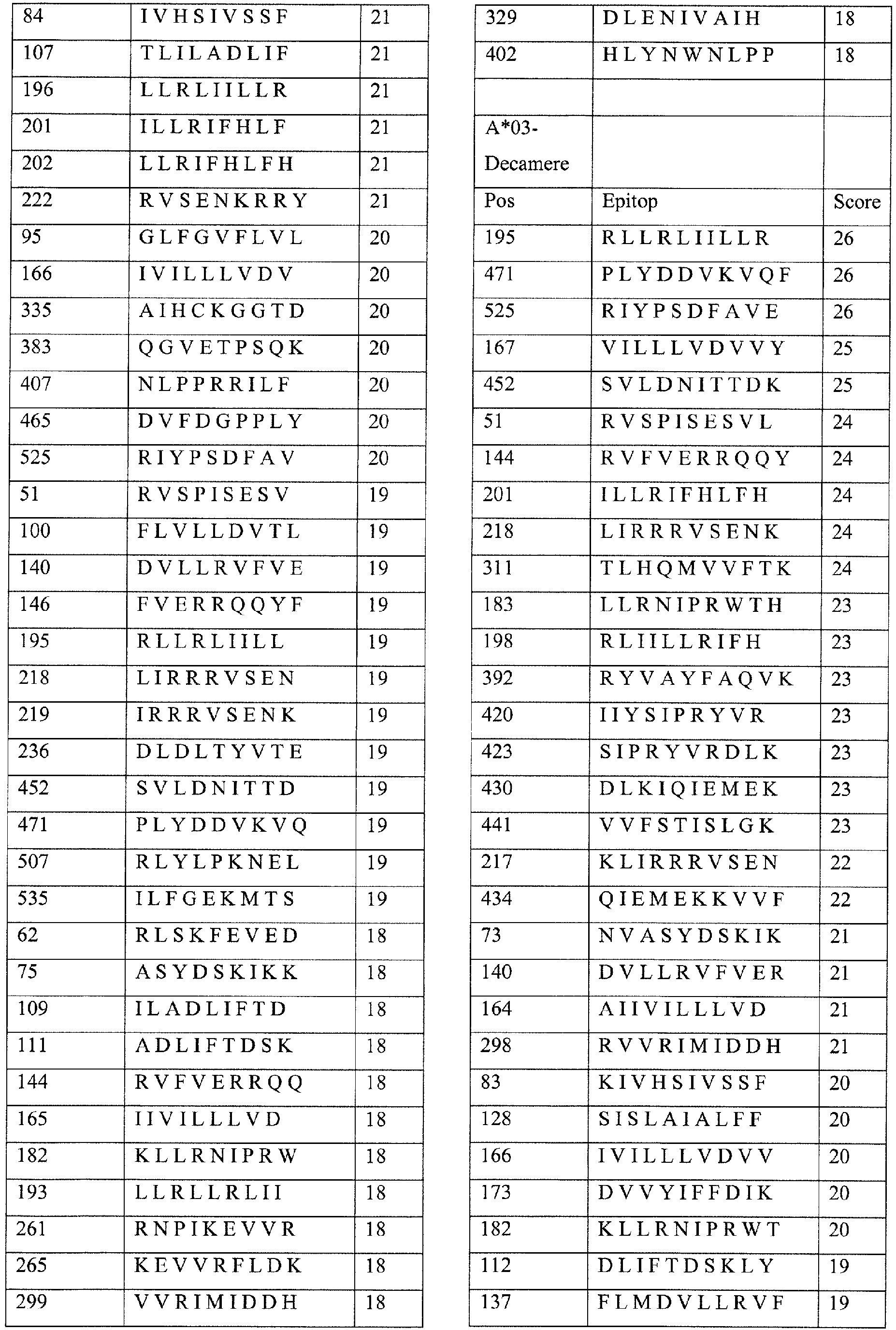

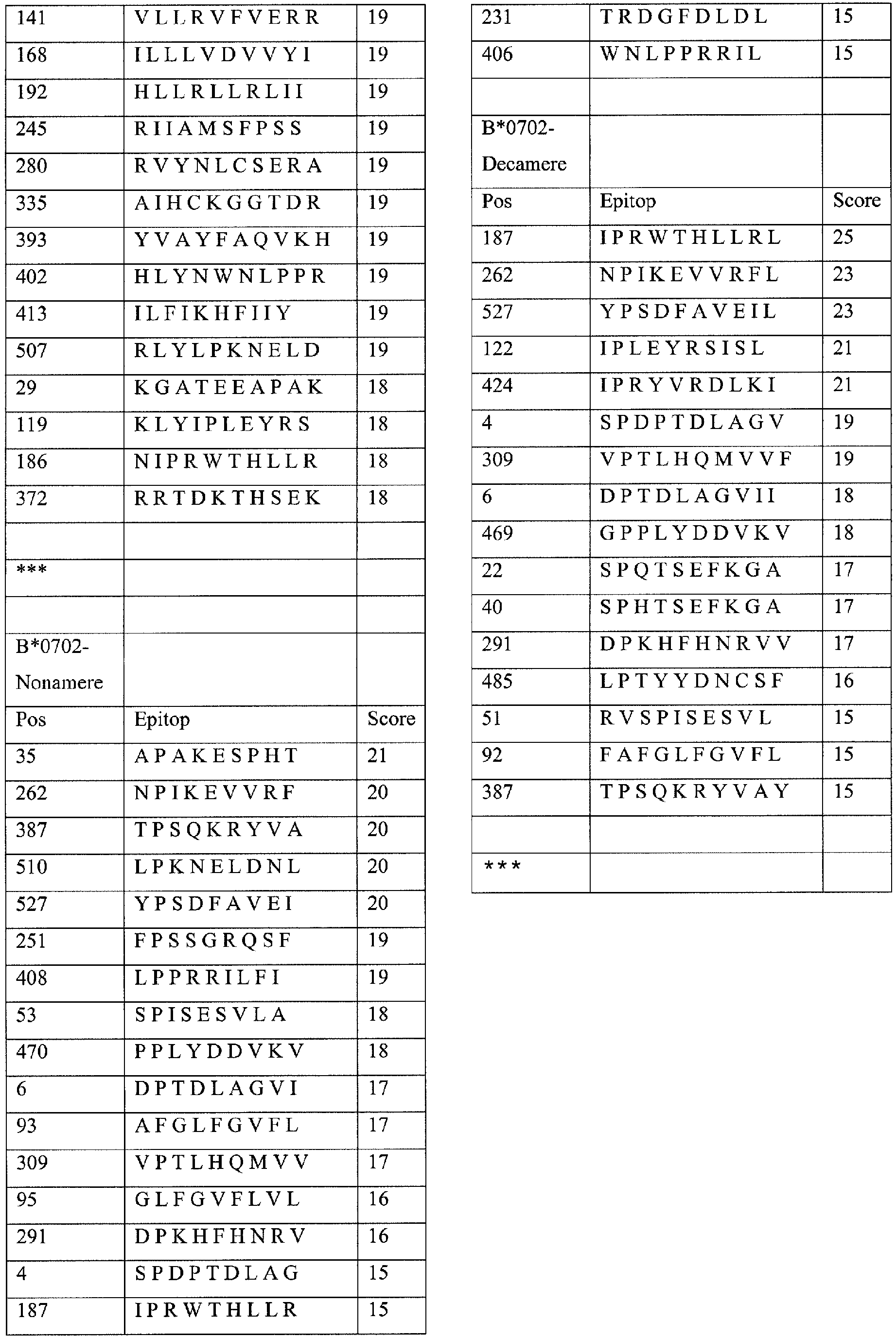

Ein Teil oder ein Fragment einer Nukleinsäure, die für ein Tumor-assoziiertes Antigen kodiert, betrifft offenbarungsgemäß den Teil der Nukleinsäure, der zumindest für das Tumorassoziierte Antigen kodiert und/oder für einen Teil oder ein Fragment des Tumor-assoziierten Antigens wie vorstehend definiert kodiert.

Die Isolierung und Identifizierung von Genen, die für Tumor-assoziierte Antigene kodieren, ermöglicht auch die Diagnose einer Erkrankung, die sich durch die Expression von einem oder mehreren Tumor-assoziierten Antigenen auszeichnet. Diese Verfahren umfassen die Bestimmung einer oder mehrerer Nukleinsäuren, die für ein Tumor-assoziiertes Antigen kodieren, und/oder die Bestimmung der kodierten Tumor-assoziierten Antigene und/oder von davon abgeleiteten Peptiden. Eine Bestimmung der Nukleinsäure kann in herkömmlicher Weise erfolgen, einschließlich durch Polymerase-Kettenreaktion oder Hybridisierung mit einer markierten Sonde. Eine Bestimmung von Tumor-assoziierten Antigenen oder davon abgeleiteten Peptiden kann durch ein Screening von Patienten-Antiseren in Bezug auf eine Erkennung des Antigens und/oder der Peptide erfolgen. Sie kann auch erfolgen durch ein Screening von T-Zellen des Patienten auf Spezifität für das entsprechende tumor-assoziierte Antigen.

Die vorliegende Lehre ermöglicht auch die Isolierung von Proteinen, die an hier beschriebene Tumor-assoziierte Antigene binden, einschließlich Antikörper und zelluläre Bindepartner der Tumor-assoziierten Antigene.

Offenbarungsgemäß werden auch in bestimmten Ausführungsformen "dominant negative" Polypeptide bereitgestellt, die von Tumor-assoziierten Antigenen abgeleitet sind. Ein dominant negatives Polypeptid ist eine inaktive Variante eines Proteins, die durch Interaktion mit der zellulären Maschinerie ein aktives Protein von seiner Interaktion mit der zellulären Maschinerie verdrängt oder mit dem aktiven Protein kompetitiert, wodurch die Wirkung des aktiven Proteins verringert wird. Zum Beispiel kann ein dominant negativer Rezeptor, der einen Liganden bindet, jedoch kein Signal in Reaktion auf die Bindung des Liganden erzeugt, die biologische Wirkung des Liganden verringern. In ähnlicher Weise kann eine dominant negative katalytisch-inaktive Kinase, die normalerweise mit Zielproteinen interagiert, jedoch die Zielproteine nicht phosphoryliert, die Phosphorylierung der Zielproteine in Reaktion auf ein zelluläres Signal verringern. In ähnlicher Weise kann ein dominant negativer Transkriptionsfaktor, der an eine Promotorstelle in der Kontrollregion eines Gens bindet, jedoch die Transkription des Gens nicht erhöht, die Wirkung eines normalen Transkriptionsfaktors durch die Besetzung von Promotorbindestellen ohne eine Erhöhung der Transkription verringern.

Das Ergebnis der Expression eines dominant negativen Polypeptids in einer Zelle ist eine Verringerung der Funktion aktiver Proteine. Der Fachmann kann dominant negative Varianten eines Proteins beispielsweise durch herkömmliche Mutageneseverfahren und Bewerten der dominant negativen Wirkung des Varianten-Polypeptids herstellen.

Offenbarungsgemäß umfasst sind auch Stoffe wie Polypeptide, die an Tumor-assoziierte Antigene binden. Solche Bindestoffe können z.B. in Screening-Assays für einen Nachweis von Tumor-assoziierten Antigenen und Komplexen von Tumor-assoziierten Antigenen mit ihren Bindepartnern sowie bei einer Aufreinigung der Tumor-assoziierten Antigene und von Komplexen davon mit ihren Bindepartnern Verwendung finden. Solche Stoffe können auch für eine Hemmung der Aktivität Tumor-assoziierter Antigene beispielsweise durch Bindung an solche Antigene Verwendung finden.

Offenbarungsgemäß umfasst sind daher Bindestoffe wie z.B. Antikörper oder Antikörperfragmente, die die Fähigkeit aufweisen, selektiv an Tumor-assoziierte Antigene zu binden. Antikörper umfassen polyklonale und monoklonale Antikörper, die in herkömmlicher Weise hergestellt werden.

Es ist bekannt, dass nur ein kleiner Teil eines Antikörpermoleküls, das Paratop, an der Bindung des Antikörpers an sein Epitop beteiligt ist (vgl. Clark, W.R. (1986), The Experimental Foundations of Modern Immunology, Wiley & Sons, Inc., New York; Roitt, I. (1991), Essential Immunology, 7. Auflage, Blackwell Scientific Publications, Oxford). Die pFc'- und Fc-Regionen sind z.B. Effektoren der Komplementkaskade, sind jedoch nicht an der Antigenbindung beteiligt. Ein Antikörper, von dem die pFc'-Region enzymatisch abgespalten wurde oder der ohne die pFc'-Region hergestellt wurde, bezeichnet als F(ab')₂-Fragment, trägt beide Antigenbindestellen eines vollständigen Antikörpers. In ähnlicher Weise trägt ein Antikörper, von dem die Fc-Region enzymatisch abgespalten wurde oder der ohne die Fc-Region hergestellt wurde, bezeichnet als Fab-Fragment, eine Antigenbindestelle eines intakten Antikörpermoleküls. Desweiteren bestehen Fab-Fragmente aus einer kovalent gebundenen leichten Kette eines Antikörpers und einem Teil der schweren Kette des Antikörpers, bezeichnet als Fd. Die Fd-Fragmente sind die Haupt-Determinanten der Antikörper-Spezifität (ein einzelnes Fd-Fragment kann mit bis zu zehn verschiedenen leichten Ketten assoziiert werden, ohne die Spezifität des Antikörpers zu verändern) und Fd-Fragmente behalten bei einer Isolierung die Fähigkeit, an ein Epitop zu binden.

Innerhalb des Antigen-bindenden Teils eines Antikörpers befinden sich komplementaritätsbestimmende Regionen (CDRs), die direkt mit dem Epitop des Antigens wechselwirken, und Gerüstregionen (FRs), die die Tertiärstruktur des Paratops aufrechterhalten. Sowohl in dem Fd-Fragment der schweren Kette als auch in der leichten Kette von IgG-Immunglobulinen befinden sich vier Gerüstregionen (FR1 bis FR4), die jeweils durch drei komplementaritätsbestimmende Regionen (CDR1 bis CDR3) getrennt sind. Die CDRs und insbesondere die CDR3-Regionen und noch mehr die CDR3-Region der schweren Kette sind größtenteils für die Antikörper-Spezifität verantwortlich.

Man weiß, dass die Nicht-CDR-Regionen eines Säuger-Antikörpers durch ähnliche Regionen von Antikörpern mit der gleichen oder einer anderen Spezifität ersetzt werden können, wobei die Spezifität für das Epitop des ursprünglichen Antikörpers erhalten bleibt. Dies ermöglichte die Entwicklung sogenannter "humanisierter" Antikörper, bei denen nicht-menschliche CDRs kovalent mit menschlichen FR- und/oder Fc/pFc'-Regionen für die Herstellung eines funktionellen Antikörpers verbunden sind.

Zum Beispiel beschreibt die WO 92/04381 die Herstellung und Verwendung von humanisierten RSV-Antikörpern aus Maus, bei denen mindestens ein Teil der FR-Regionen aus Maus durch FR-Regionen eines menschlichen Ursprungs ersetzt wurden. Solche Antikörper, einschließlich Fragmente intakter Antikörper mit einer Antigen-Bindefähigkeit werden oft als "chimäre" Antikörper bezeichnet.

Offenbarungsgemäß werden auch F(ab')₂-, Fab-, Fv- und Fd-Fragmente von Antikörpern, chimäre Antikörper, bei denen die Fc- und/oder FR- und/oder CDR1- und/oder CDR2- und/oder leichte Kette-CDR3-Regionen durch homologe menschliche oder nicht-menschliche Sequenzen ersetzt wurden, chimäre F(ab')₂-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, chimäre Fab-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, und chimäre Fd-Fragment-Antikörper, bei denen die FR- und/oder CDR1- und/oder CDR2-Regionen durch homologe menschliche oder nicht-menschliche Sequenzen ersetzt wurden, bereitgestellt. Offenbarungsgemäß umfasst sind auch sogenannte einzelkettige Antikörper.

Vorzugsweise ist ein offenbarungsgemäß verwendeter Antikörper gegen eine der in SEQ ID NOs: 81, 82 und 103-105 des Sequenzprotokolls dargestellten Sequenzen gerichtet und/oder kann durch Immunisierung mit diesen Peptiden erhalten werden.

Offenbarungsgemäß umfasst sind auch Polypeptide, die spezifisch an Tumor-assoziierte Antigene binden. Beispielsweise können solche Polypeptid-Bindestoffe durch degenerierte Peptid-Bibliotheken bereitgestellt werden, die einfach in Lösung in einer immobilisierten Form oder als Phagen-Display-Bibliotheken hergestellt werden können. Kombinatorische Bibliotheken aus Peptiden mit einer oder mehreren Aminosäuren können ebenfalls hergestellt werden. Ferner können Bibliotheken aus Peptoiden und nicht-peptidischen synthetischen Resten hergestellt werden.

Phagen-Display kann besonders wirksam bei der Identifizierung offenbarungsgemäßer Bindepeptide sein. Dabei wird beispielsweise eine Phagen-Bibliothek (durch Verwendung beispielsweise des m13-, fd- oder lambda-Phagen) hergestellt, die Inserts einer Länge von 4 bis etwa 80 Aminosäureresten präsentiert. Es werden sodann Phagen ausgewählt, die Inserts tragen, die an das Tumor-assoziierte Antigen binden. Dieser Prozess kann über mehrere Zyklen einer Rückselektion von Phagen wiederholt werden, die an das Tumor-assoziierte Antigen binden. Wiederholte Runden führen zu einer Anreicherung von Phagen, die bestimmte Sequenzen tragen. Es kann eine Analyse von DNA-Sequenzen erfolgen, um die Sequenzen der exprimierten Polypeptide zu identifizieren. Der kleinste lineare Anteil der Sequenz, der an das Tumor-assoziierte Antigen bindet, kann bestimmt werden. Das "twohybrid-System" aus Hefe kann auch für die Identifizierung von Polypeptiden eingesetzt werden, die an ein Tumor-assoziiertes Antigen binden. Erfindungsgemäß beschriebene Tumor-assoziierte Antigene oder Fragmente davon können für ein Screening von Peptid-Bibliotheken, einschließlich Phagen-Display-Bibliotheken, eingesetzt werden, um Peptid-Bindepartner der Tumor-assoziierten Antigene zu identifizieren und selektieren. Solche Moleküle können beispielsweise für Screening-Assays, Aufreinigungsprotokolle, für eine Interferenz mit der Funktion des Tumor-assoziierten Antigens und für andere Zwecke, die dem Fachmann bekannt sind, verwendet werden.

Die vorstehend beschriebenen Antikörper und andere Bindemoleküle können beispielsweise für die Identifizierung von einem Gewebe verwendet werden, das ein Tumor-assoziiertes Antigen exprimiert. Antikörper können auch an spezifische diagnostische Stoffe für eine Darstellung von Zellen und Geweben gekoppelt werden, die Tumor-assoziierte Antigene exprimieren. Sie können ferner an therapeutisch nützliche Stoffe gekoppelt werden. Diagnostische Stoffe umfassen in nicht begrenzender Weise Bariumsulfat, Iocetaminsäure, Iopansäure, Calcium-Ipodat, Natrium-Diatrizoat, Meglumin-Diatrizoat, Metrizamid, Natrium-Tyropanoat und Radiodiagnostika, einschließlich Positronen-Emitter wie Fluorin-18 und Carbon-11, gamma-Emitter wie Iodin-123, Technitium-99m, Iod-131 und Indium-111, Nuklide für magnetische Kernresonanz wie Fluorin und Gadolinium. Der Begriff "therapeutisch nützlicher Stoff" meint erfindungsgemäß jedes therapeutische Molekül, das wunschgemäß selektiv zu einer Zelle geführt wird, die ein oder mehrere Tumor-assoziierte Antigene exprimiert, einschließlich Antikrebsmittel, mit radioaktivem Iod versehene Verbindungen, Toxine, cytostatische oder cytolytische Arzneistoffe, usw. Antikrebsmittel umfassen beispielsweise Aminoglutethimid, Azathioprin, Bleomycinsulfat, Busulfan, Carmustin, Chlorambucil, Cisplatin, Cyclophosphamid, Cyclosporin, Cytarabidin, Dacarbazin, Dactinomycin, Daunorubin, Doxorubicin, Taxol, Etoposid, Fluoruracil, Interferon-α, Lomustin, Mercaptopurin, Methotrexat, Mitotan, Procarbazin-HCl, Thioguanin, Vinblastinsulfat und Vincristinsulfat. Weitere Antikrebsmittel sind beispielsweise in Goodman und Gilman, "The Pharmacological Basis of Therapeutics", 8. Auflage, 1990, McGraw-Hill, Inc., insbesondere Kapitel 52 (Antineoplastic Agents (Paul Calabresi und Bruce A. Chabner) beschrieben. Toxine können Proteine wie Pokeweed-antivirales Protein, Choleratoxin, Pertussistoxin, Ricin, Gelonin, Abrin, Diphtherie-Exotoxin oder *Pseudomonas-*Exotoxin sein. Toxinreste können auch Hochenergie-emittierende Radionuklide wie Kobalt-60 sein.

Im Fall von TPTE (vgl. Beispiel 1) konnte erfindungsgemäß gezeigt werden, dass dieses nicht nur ein membranständiges Protein ist, sondern auch zu internalisieren vermag. Daher können die TPTE betreffenden erfingungsgemäß identifizierten Tumor-assoziierten Antigene selbst einem Transport von daran bindenden Substanzen, insbesondere den vorstehend beschriebenen therapeutischen Antikörpern, von der Membran ins Cytoplasma und somit einer Internalisierung dienen, wo diese Substanzen vorzugsweise ihre Wirkung wie eine zellzerstörende Wirkung entfalten können.

Der Begriff "Patient" bedeutet erfindungsgemäß Mensch, nicht menschlicher Primat oder ein anderes Tier, insbesondere Säugetier wie Kuh, Pferd, Schwein, Schaf, Ziege, Hund, Katze oder Nagetier wie Maus und Ratte. In einer besonders bevorzugten Ausführungsform ist der Patient ein Mensch.

Der Begriff "Erkrankung" betrifft erfindungsgemäß jeden pathologischen Zustand, bei dem Tumor-assoziierte Antigene exprimiert oder abnormal exprimiert werden. "Abnormale Expression" bedeutet erfindungsgemäß, dass die Expression gegenüber dem Zustand bei einem gesunden Individuum verändert, vorzugsweise erhöht ist. Eine Erhöhung der Expression betrifft eine Erhöhung um mindestens 10%, insbesondere mindestens 20%, mindestens 50% oder mindestens 100%. In einer Ausführungsform wird das Tumorassoziierte Antigen nur in Gewebe eines erkrankten Individuums exprimiert, während die Expression bei einem gesunden Individuum reprimiert ist. Ein Beispiel einer solchen Erkrankung ist Krebs, insbesondere Seminome, Melanome, Teratome, Gliome, Kolorektal-, Brust-, Prostata-, Gebärmutter-, Ovarial-, und Lungenkrebs.

Eine biologische Probe kann erfindungsgemäß eine Gewebe- und/oder zelluläre Probe sein und kann für eine Verwendung in den verschiedenen, hier beschriebenen Verfahren in herkömmlicher Weise gewonnen werden, wie durch Gewebebiopsie, einschließlich Stanzbiopsie, und Entnahme von Blut, Bronchialaspirat, Urin, Fäces oder anderen Körperflüssigkeiten.

Der Begriff "immunreaktive Zelle" bedeutet erfindungsgemäß eine Zelle, die in eine Immunzelle (wie B-Zelle, T-Helferzelle oder cytolytische T-Zelle) bei geeigneter Stimulierung reifen kann. Immunreaktive Zellen umfassen CD34⁺ hämatopoietische Stammzellen, unreife und reife T-Zellen sowie unreife und reife B-Zellen. Falls die Herstellung cytolytischer oder Helfer T-Zellen, die ein Tumor-assoziiertes Antigen erkennen, gewünscht ist, wird die immunreaktive Zelle mit einer Zelle, die ein Tumor-assoziiertes Antigen exprimiert, unter Bedingungen in Kontakt gebracht, die eine Produktion, Differenzierung und/oder Selektion von cytolytischen sowie Helfer T-Zellen begünstigen. Die Differenzierung von T-Zell-Vorläufem in eine cytolytische T-Zelle bei einer Exposition gegenüber einem Antigen ist ähnlich zur klonalen Selektion des Immunsystems.

Manche therapeutische Verfahren beruhen auf einer Reaktion des Immunsystems eines Patienten, die zu einer Lyse Antigen-präsentierender Zellen führt, wie Krebszellen, die ein oder mehrere Tumor-assoziierte Antigene präsentieren. Dabei werden beispielsweise autologe cytotoxische T-Lymphozyten, die für einen Komplex aus einem Tumor-assoziierten Antigen und einem MHC-Molekül spezifisch sind, an einen Patienten mit einer Zellabnormalie verabreicht. Die Produktion solcher cytotoxischer T-Lymphozyten *in vitro* ist bekannt. Ein Beispiel für ein Verfahren zur Differenzierung von T-Zellen findet sich in der WO-A-9633265. Im Allgemeinen wird eine Probe mit Zellen wie Blutzellen aus dem Patienten entnommen und die Zellen werden mit einer Zelle in Kontakt gebracht, die den Komplex präsentiert und eine Vermehrung von cytotoxischen T-Lymphozyten auslösen kann (z.B. dendritische Zellen). Die Zielzelle kann eine transfizierte Zelle wie eine COS-Zelle sein. Diese transfizierten Zellen präsentieren den gewünschten Komplex auf ihrer Oberfläche und stimulieren bei einer Kontaktierung mit cytotoxischen T-Lymphozyten deren Vermehrung. Die klonal expandierten autologen cytotoxischen T-Lymphozyten werden sodann an den Patienten verabreicht.

Bei einem anderen Verfahren zur Selektion Antigen-spezifischer cytotoxischer T-Lymphozyten werden fluorogene Tetramere von MHC-Klasse I-Molekül/Peptid-Komplexen für einen Nachweis spezifischer Klone von cytotoxischen T-Lymphozyten verwendet (Altman et al., Science 274:94-96, 1996; Dunbar et al., Curr. Biol. 8:413-416, 1998). Lösliche MHC-Klasse I-Moleküle werden *in vitro* in Gegenwart von β₂-Mikroglobulin und eines Peptid-Antigens, das an das Klasse I-Molekül bindet, gefaltet. Nach Aufreinigung der MHC/Peptid-Komplexe werden diese mit Biotin markiert. Tetramere werden durch Mischen der biotinylierten Peptid-MHC-Komplexe mit markiertem Avidin (z.B. Phycoerythrin) bei einem molaren Verhältnis von 4:1 gebildet. Tetramere werden sodann mit cytotoxischen T-Lymphozyten wie peripherem Blut oder Lymphknoten in Kontakt gebracht. Die Tetramere binden an cytotoxische T-Lymphozyten, die den Peptid-Antigen/MHC-Klasse I-Komplex erkennen. Zellen, die an die Tetramere gebunden werden, können durch Fluoreszenzgesteuerte Zellsortierung für eine Isolierung reaktiver cytotoxischer T-Lymphozyten sortiert werden. Die isolierten cytotoxischen T-Lymphozyten können sodann *in vitro* vermehrt werden.

Bei einem therapeutischen Verfahren, das als adoptiver Transfer bezeichnet wird (Greenberg, J. Immunol. 136(5):1917, 1986; Riddel et al., Science 257:238, 1992; Lynch et al., Eur. J. Immunol. 21:1403-1410, 1991; Kast et al., Cell 59:603-614, 1989), werden Zellen, die den gewünschten Komplex präsentieren (z.B. dendritische Zellen) mit cytotoxischen T-Lymphozyten des zu behandelnden Patienten kombiniert, was zu einer Vermehrung spezifischer cytotoxischer T-Lymphozyten führt. Die vermehrten cytotoxischen T-Lymphozyten werden sodann an einen Patienten mit einer zellulären Abnormalie verabreicht, die sich durch bestimmte abnormale Zellen auszeichnet, die den spezifischen Komplex präsentieren. Die cytotoxischen T-Lymphozyten lysieren sodann die abnormalen Zellen, wodurch eine gewünschte therapeutische Wirkung erreicht wird.

Oft lassen sich aus dem T-Zell-Repertoire eines Patienten lediglich niedrig-affine T-Zellen gegen einen solchen spezifischen Komplex vermehren, da die hochaffinen durch Toleranzentwicklung ausgelöscht worden sind. Eine Alternative kann hier ein Transfer des T-Zell-Rezeptors selbst sein. Hierfür werden ebenfalls Zellen, die den gewünschten Komplex präsentieren (z.B. dendritische Zellen) mit cytotoxischen T-Lymphozyten von Gesunden kombiniert. Dies führt zu einer Vermehrung hochaffiner spezifischer cytotoxischer T-Lymphozyten, wenn der Spender mit dem spezifischen Komplex bisher keinen Kontakt hatte. Der hochaffine T-Zell-Rezeptor aus diesen vermehrten spezifischen T-Lymphozyten wird kloniert und kann durch Gentransfer z.B. mit retroviralen Vektoren beliebig in T-Zellen von anderen Patienten transduziert werden. Adoptiver Transfer erfolgt dann mit diesen genetisch veränderten T-Lymphozyten (Stanislawski et al., Nat Immunol. 2:962-70, 2001; Kessels et al., Nat Immunol. 2:957-61, 2001).

Die vorstehenden therapeutischen Aspekte gehen davon aus, dass zumindest manche der abnormalen Zellen des Patienten einen Komplex aus einem Tumor-assoziierten Antigen und einem HLA-Molekül präsentieren. Eine Identifizierung solcher Zellen kann in an sich bekannter Weise erfolgen. Sobald Zellen, die den Komplex präsentieren, identifiziert wurden, können sie mit einer Probe aus dem Patienten, die cytotoxische T-Lymphozyten enthält, kombiniert werden. Falls die Zellen, die den Komplex präsentieren, durch die cytotoxischen T-Lymphozyten lysiert werden, kann angenommen werden, dass ein Tumor-assoziiertes Antigen präsentiert wird.

Der adoptive Transfer ist nicht die einzige Therapieform, die erfindungsgemäß anwendbar ist. Cytotoxische T-Lymphozyten können auch *in vivo* in an sich bekannter Weise erzeugt werden. Bei einem Verfahren werden nicht-proliferative Zellen verwendet, die den Komplex exprimieren. Die Zellen, die dabei verwendet werden, werden diejenigen sein, die normalerweise den Komplex exprimieren, wie bestrahlte Tumorzellen oder Zellen, die mit einem oder beiden Genen transfiziert wurden, die für eine Präsentation des Komplexes notwendig sind (d.h. das Antigene Peptid und das präsentierende HLA-Molekül). Verschiedene Zelltypen können eingesetzt werden. Desweiteren können Vektoren verwendet werden, die eines oder beide der interessierenden Gene tragen. Virale oder bakterielle Vektoren sind besonders bevorzugt. Zum Beispiel können Nukleinsäuren, die für ein Tumor-assoziiertes Antigen oder einen Teil davon kodieren, funktionell mit Promotor- und Enhancersequenzen verknüpft werden, die eine Expression des Tumor-assoziierten Antigens oder eines Fragments davon in bestimmten Geweben oder Zelltypen steuern. Die Nukleinsäure kann in einen Expressionsvektor eingebaut werden. Expressionsvektoren können nicht-modifizierte extrachromosomale Nukleinsäuren, Plasmide oder virale Genome sein, in die eine Insertion exogener Nukleinsäuren möglich ist. Nukleinsäuren, die für ein Tumor-assoziiertes Antigen kodieren, können auch in ein retrovirales Genom inseriert werden, wodurch die Integration der Nukleinsäure in das Genom des Zielgewebes oder der Zielzelle ermöglicht wird. Bei diesen Systemen trägt ein Mikroorganismus wie Vacciniavirus, Poxvirus, Herpes simplex-Virus, Retrovirus oder Adenovirus das interessierende Gen und "infiziert" de facto Wirtszellen. Eine weitere bevorzugte Form ist die Einbringung des tumorassoziierten Antigenes in Form von rekombinanter RNA. Diese kann z.B. durch liposomalen Transfer oder durch Elektroporation in Zellen eingebracht werden. Die resultierenden Zellen präsentieren den interessierenden Komplex und werden von autologen cytotoxischen T-Lymphozyten erkannt, die sich sodann vermehren.

Eine ähnliche Wirkung kann durch Kombination des Tumor-assoziierten Antigens oder eines Fragments davon mit einem Adjuvans erreicht werden, um einen Einbau in Antigen-präsentierende Zellen *in vivo* zu ermöglichen. Das tumor-assoziierte Antigen oder ein Fragment davon können als Protein, als DNA (z.B. innerhalb eines Vektors) oder als RNA repräsentiert sein. Das Tumor-assoziierte Antigen wird prozessiert, um einen Peptidpartner für das HLA-Molekül zu ergeben, während ein Fragment davon präsentiert werden kann, ohne dass eine weitere Prozessierung erforderlich ist. Letzteres ist insbesondere der Fall, wenn diese and HLA-Moleküle binden können. Verabreichungsformen, bei denen das Gesamt-Antigen *in vivo* von einer Dendritischen Zelle prozessiert wird, sind bevorzugt, da hier auch Helfer T-Zell-Antworten entstehen können. Eine effektive Immunantwort benötigt diese (Ossendorp et al, Immunol Lett. 74:75-9, 2000; Ossendorp et al, J. Exp. Med. 187:693-702, 1998). Im Allgemeinen kann eine wirksame Menge des Tumor-assoziierten Antigens an einen Patienten z.B. durch eine intradermale Injektion verabreicht werden. Die Injektion kann aber auch intranodal in einen Lymphknoten erfolgen (Maloy et al, Proc Natl Acad Sci USA 98:3299-303, 2001). Sie kann auch in Kombination mit Reagenzien erfolgen, die eine Aufnahme in Dendritische Zellen erleichtern. In vivo bevorzugte Tumor-assoziierte Antigene umfassen diejenigen, die mit allogenen Krebs-Antiseren oder mit T-Zellen vieler Krebs-Patienten reagieren. Von besonderem Interesse sind aber auch solche, gegen die keine spontanen Immunantworten vorbestehen. Gegen diese können nachweislich Immunantworten induziert werden, die Tumoren lysieren können (Keogh et al, J Immunol. 167:787-96, 2001; Appella et al, Biomed Pept Proteins Nucleic Acids 1:177-84, 1995; Wentworth et al, Mol Immunol. 32:603-12, 1995).

Die offenbarungsgemäß beschriebenen pharmazeutischen Zusammensetzungen können auch als Vacccinen für die Immunisierung eingesetzt werden. Die Begriffe "Immunisierung" oder "Vaccinierung" bedeuten gemäß der offenbarten Lehre eine Erhöhung oder Aktivierung einer Immunreaktion gegenüber einem Antigen. Tiermodelle können für ein Testen einer immunisierenden Wirkung gegenüber Krebs durch Verwendung eines Tumor-assoziierten Antigens oder einer dafür kodierenden Nukleinsäure eingesetzt werden. Zum Beispiel können menschliche Krebszellen in eine Maus für die Schaffung eines Tumors eingebracht werden und eine oder mehrere Nukleinsäuren, die für Tumor-assoziierte Antigene kodieren, können verabreicht werden. Die Wirkung auf die Krebszellen (beispielsweise Verringerung der Tumorgröße) kann als Maß für die Wirksamkeit einer Immunisierung durch die Nukleinsäure gemessen werden.

Als Teil der Zusammensetzung für eine Immunisierung werden eines oder mehrere Tumorassoziierte Antigene oder stimulierende Fragmente davon mit einem oder mehreren Adjuvanzien für eine Induktion einer Immunreaktion oder eine Erhöhung einer Immunreaktion verabreicht. Ein Adjuvans ist eine Substanz, die in das Antigen eingebaut oder gemeinsam mit diesem verabreicht wird und die Immunreaktion verstärkt. Adjuvanzien können die Immunreaktion durch Bereitstellen eines Antigen-Reservoirs (extrazellulär oder in Makrophagen), Aktivierung von Makrophagen und Stimulierung bestimmter Lymphozyten verstärken. Adjuvanzien sind bekannt und umfassen in nicht begrenzender Weise Monophosphoryl-Lipid-A (MPL, SmithKline Beecham), Saponine wie QS21 (SmithKline Beecham), DQS21 (SmithKline Beecham; WO 96/33739), QS7, QS17, QS18 und QS-L1 (So et al., Mol. Cells 7:178-186, 1997), unvollständiges Freundsches Adjuvans, vollständiges Feundsches Adjuvans, Vitamin E, Montanid, Alaun, CpG-Oligonukleotide (vgl. Kreig et al., Nature 374:546-9, 1995) und verschiedene Wasser-in-Öl-Emulsionen, die aus biologisch abbaubaren Ölen wie Squalen und/oder Tocopherol hergestellt werden. Vorzugsweise werden die Peptide in einer Mischung mit DQS21/MPL verabreicht. Das Verhältnis von DQS21 zu MPL beträgt typischerweise etwa 1:10 bis 10:1, vorzugsweise etwa 1:5 bis 5:1 und insbesondere etwa 1:1. Für eine Verabreichung an den Menschen sind DQS21 und MPL typischerweise in einer Vaccine-Formulierung in einem Bereich von etwa 1 µg bis etwa 100 µg vorhanden.

Andere Stoffe, die eine Immunreaktion des Patienten stimulieren, können auch verabreicht werden. Zum Beispiel sind Cytokine bei einer Vaccinierung aufgrund ihrer regulatorischen Eigenschaften auf Lymphozyten verwendbar. Solche Cytokine umfassen z.B. Interleukin-12 (IL-12), von dem gezeigt wurde, dass es die schützenden Wirkungen von Vaccinen verstärkt (vgl. Science 268:1432-1434, 1995), GM-CSF und IL-18.

Es gibt eine Reihe von Verbindungen, die eine Immunreaktion verstärken und die daher bei einer Vaccinierung eingesetzt werden können. Diese umfassen co-stimulierende Moleküle, die in Form von Proteinen oder Nukleinsäuren bereitgestellt werden. Solche co-stimulierenden Moleküle sind beispielsweise B7-1 und B7-2 (CD80 bzw. CD86), die auf dendritischen Zellen (DC) exprimiert werden und mit dem auf den T-Zellen exprimierten CD28-Molekül interagieren. Diese Interaktion stellt eine Co-Stimulierung (Signal 2) für eine Antigen/MHC/TCR-stimulierte (Signal 1) T-Zelle bereit, wodurch die Vermehrung der T-Zelle und die Effektorfunktion verstärkt wird. B7 interagiert auch mit CTLA4 (CD152) auf T-Zellen und Untersuchungen, die CTLA4- und B7-Liganden einbeziehen, zeigen, dass die B7-CTLA4-Interaktion eine Antitumor-Immunität und CTL-Vermehrung verstärken kann (Zheng, P. et al., Proc. Natl. Acad. Sci. USA 95(11):6284-6289 (1998)).

B7 wird typischerweise nicht auf Tumorzellen exprimiert, so dass diese keine wirksamen Antigen-präsentierenden Zellen (APCs) für T-Zellen sind. Eine Induktion der B7-Expression würde ermöglichen, dass Tumorzellen wirksamer eine Vermehrung von cytotoxischen T-Lymphozyten und eine Effektorfunktion stimulieren. Eine Co-Stimulierung durch eine Kombination von B7/IL-6/IL-12 zeigte eine Induktion des IFN-gamma- und Thl-Cytokin-Profils in einer T-Zell-Population, was zu einer weiter verstärkten T-Zell-Aktivität führt (Gajewski et al., J. Immunol. 154:5637-5648 (1995)).

Eine vollständige Aktivierung von cytotoxischen T-Lymphozyten und eine vollständige Effektorfunktion erfordert eine Mitwirkung von T-Helferzellen durch die Interaktion zwischen dem CD40-Liganden auf den T-Helferzellen und dem CD40-Molekül, das von dendritischen Zellen exprimiert wird (Ridge et al., Nature 393:474 (1998), Bennett et al., Nature 393:478 (1998), Schönberger et al., Nature 393:480 (1998)). Der Mechanismus dieses co-stimulierenden Signals betrifft wahrscheinlich die Steigerung der B7- und assoziierten IL-6/IL-12-Produktion durch die dendritischen Zellen (Antigen-präsentierenden Zellen). Die CD40-CD40L-Interaktion komplementiert so die Interaktionen des Signals 1 (Antigen/MHC-TCR) und des Signals 2 (B7-CD28).

Die Verwendung von anti-CD40-Antikörpern für eine Stimulierung von dendritischen Zellen würde erwartungsgemäß direkt eine Reaktion gegenüber Tumor-Antigenen verstärken, die normalerweise außerhalb des Bereichs einer entzündlichen Reaktion liegen oder von nichtprofessionellen Antigen-präsentierenden Zellen (Tumorzellen) präsentiert werden. In diesen Situationen werden T-Helfer- und B7-co-stimulierende Signale nicht bereitgestellt. Dieser Mechanismus könnte im Zusammenhang mit Therapien verwendet werden, die auf Antigengepulsten dendritischen Zellen basieren, oder in Situationen, bei denen T-Helfer-Epitope nicht in bekannten TRA-Vorläufern definiert wurden.

Offenbarungsgemäß vorgesehen ist auch eine Verabreichung von Nukleinsäuren, Polypeptiden oder Peptiden. Eine Verabreichung von Polypeptiden und Peptiden kann in an sich bekannter Weise erfolgen. In einer Ausführungsform erfolgt die Verabreichung von Nukleinsäuren durch *ex vivo*-Verfahren, d.h. durch Entfernung von Zellen aus einem Patienten, genetische Veränderung der Zellen, um ein Tumor-assoziiertes Antigen einzubauen, und Wiedereinbringung der veränderten Zellen in den Patienten. Dies umfasst im Allgemeinen das Einbringen einer funktionellen Kopie eines Gens in die Zellen eines Patienten *in vitro* und die Rückführung der genetisch veränderten Zellen in den Patienten. Die funktionelle Kopie des Gens steht unter funktioneller Kontrolle von regulatorischen Elementen, die eine Expression des Gens in den genetisch veränderten Zellen erlauben. Transfektions- und Transduktionsverfahren sind dem Fachmann bekannt. Offenbarungsgemäß vorgesehen ist auch eine Verabreichung von Nukleinsäuren *in vivo* durch die Verwendung von Vektoren wie Viren und zielgesteuerten Liposomen.

In einer bevorzugten Ausführungsform ist ein viraler Vektor für die Verabreichung einer Nukleinsäure, die für ein Tumor-assoziiertes Antigen kodiert, aus der Gruppe ausgewählt bestehend aus Adenoviren, Adeno-assoziierten Viren, Poxviren, einschließlich Vacciniavirus und attenuierten Poxviren, Semliki-Forest-Virus, Retroviren, Sindbis-Virus und Ty-Virusähnlichen Partikeln. Besonders bevorzugt sind Adenoviren und Retroviren. Die Retroviren sind üblicherweise replikationsdefizient (d.h. sie sind unfähig, infektiöse Partikel zu erzeugen).

Verschiedene Verfahren können eingesetzt werden, um Nukleinsäuren in Zellen *in vitro* oder *in vivo* einzubringen. Solche Verfahren umfassen die Transfektion von Nukleinsäure-CaPO₄-Präzipitaten, die Transfektion von Nukleinsäuren, die mit DEAE assoziiert sind, die Transfektion oder Infektion mit den vorstehenden Viren, die die interessierenden Nukleinsäuren tragen, die Liposomen-vermittelte Transfektion und ähnliches. In bestimmten Ausführungsformen ist eine Steuerung der Nukleinsäure an bestimmte Zellen bevorzugt. In solchen Ausführungsformen kann ein Träger, der für die Verabreichung einer Nukleinsäure an eine Zelle (z.B. ein Retrovirus oder ein Liposom) eingesetzt wird, ein gebundenes Zielsteuerungsmolekül aufweisen. Zum Beispiel kann ein Molekül wie ein Antikörper, der für ein Oberflächenmembran-Protein auf der Zielzelle spezifisch ist, oder ein Ligand für einen Rezeptor auf der Zielzelle in den Nukleinsäureträger eingebaut oder daran gebunden werden. Bevorzugte Antikörper umfassen Antikörper, die selektiv ein Tumor-assoziiertes Antigen binden. Falls eine Verabreichung einer Nukleinsäure durch Liposomen erwünscht ist, können Proteine, die an ein Oberflächenmembran-Protein binden, das mit der Endozytose assoziiert ist, in die Liposomenformulierung eingebaut werden, um eine Zielsteuerung und/oder Aufnahme zu ermöglichen. Solche Proteine umfassen Kapsid-Proteine oder Fragmente davon, die für einen bestimmten Zelltyp spezifisch sind, Antikörper gegen Proteine, die internalisiert werden, Proteine, die eine intrazelluläre Stelle ansteuern und ähnliches.

Die offenbarungsgemäßen therapeutischen Zusammensetzungen können in pharmazeutisch verträglichen Zubereitungen verabreicht werden. Solche Zubereitungen können gewöhnlich pharmazeutisch verträgliche Konzentrationen von Salzen, Pufferstoffen, Konservierungsstoffen, Trägern, ergänzenden immunitätssteigernden Stoffen wie Adjuvanzien, CpG und Cytokine und gegebenenfalls andere therapeutische Wirkstoffe enthalten.

Die offenbarungsgemäßen therapeutischen Wirkstoffe können auf jedem herkömmlichen Weg verabreicht werden, einschließlich durch Injektion oder durch Infusion. Die Verabreichung kann beispielsweise oral, intravenös, intraperitoneal, intramuskulär, subkutan oder transdermal erfolgen. Eine therapeutische Verabreichung von Antikörpern erfolgt vorzugsweise durch ein Lungenaerosol. Die Verabreichung von Antisense-Nukleinsäuren erfolgt vorzugsweise durch langsame intravenöse Verabreichung.

Die offenbarungsgemäßen Zusammensetzungen werden in wirksamen Mengen verabreicht. Eine "wirksame Menge" betrifft die Menge, die alleine oder zusammen mit weiteren Dosen eine gewünschte Reaktion oder eine gewünschte Wirkung erzielt. Im Fall einer Behandlung einer bestimmten Erkrankung oder eines bestimmten Zustands, der sich durch die Expression eines oder mehrerer Tumor-assoziierter Antigene auszeichnet, betrifft die gewünschte Reaktion die Hemmung des Krankheitsverlaufs. Dies umfasst die Verlangsamung des Fortschreitens der Erkrankung und insbesondere eine Unterbrechung des Fortschreitens der Erkrankung. Die gewünschte Reaktion bei einer Behandlung einer Krankheit oder eines Zustands kann auch die Verzögerung des Ausbruchs oder eine Verhinderung des Ausbruchs der Krankheit oder des Zustands sein.

Eine wirksame Menge einer Zusammensetzung wird von dem zu behandelnden Zustand, der Schwere der Krankheit, den individuellen Parametern des Patienten, einschließlich Alter, physiologischer Zustand, Größe und Gewicht, der Dauer der Behandlung, der Art einer begleitenden Therapie (falls vorhanden), dem spezifischen Verabreichungsweg und ähnlichen Faktoren abhängen.

Die pharmazeutischen Zusammensetzungen sind vorzugsweise steril und enthalten eine wirksame Menge der therapeutisch wirksamen Substanz für die Erzeugung der gewünschten Reaktion oder der gewünschten Wirkung.

Die Dosen der Zusammensetzungen, die verabreicht werden, können von verschiedenen Parametern wie der Verabreichungsart, dem Zustand des Patienten, dem gewünschten Verabreichungszeitraum, usw. abhängen. Für den Fall, dass eine Reaktion bei einem Patienten bei einer anfänglichen Dosis unzureichend ist, können höhere Dosen (oder effektiv höhere Dosen, die durch einen anderen, stärker lokalisierten Verabreichungsweg erzielt werden) eingesetzt werden.

Im Allgemeinen werden für eine Behandlung oder für eine Erzeugung oder Erhöhung einer Immunreaktion Dosen des Tumor-assoziierten Antigens von 1 ng bis 1 mg, vorzugsweise von 10 ng bis 100 µg formuliert und verabreicht. Falls die Verabreichung von Nukleinsäuren (DNA sowie RNA), die für Tumor-assoziierte Antigene kodieren, erwünscht ist, werden Dosen von 1 ng bis 0,1 mg formuliert und verabreicht.

Die pharmazeutischen Zusammensetzungen werden im Allgemeinen in pharmazeutisch verträglichen Mengen und in pharmazeutisch verträglichen Zusammensetzungen verabreicht. Der Begriff "pharmazeutisch verträglich" betrifft ein nicht-toxisches Material, das nicht mit der Wirkung des aktiven Bestandteils der pharmazeutischen Zusammensetzung wechselwirkt. Solche Zubereitungen können gewöhnlich Salze, Pufferstoffe, Konservierungsstoffe, Träger und gegebenenfalls andere therapeutische Wirkstoffe enthalten. Bei einer Verwendung in der Medizin sollten die Salze pharmazeutisch verträglich sein. Nicht-pharmazeutisch verträgliche Salze können jedoch für die Herstellung pharmazeutisch verträglicher Salze davon verwendet werden und sind erfindungsgemäß umfasst. Solche pharmakologisch und pharmazeutisch verträglichen Salze umfassen in nicht begrenzender Weise diejenigen, die aus den nachstehenden Säuren hergestellt werden: Chlorwasserstoff-, Bromwasserstoff-, Schwefel-, Salpeter-, Phosphor-, Malein-, Essig-, Salicyl-, Citronen-, Ameisen-, Malon-, Bernsteinsäure und ähnliches. Pharmazeutisch verträgliche Salze können auch als Alkalimetall- oder Erdalkalimetallsalze wie Natrium-, Kalium- oder Calciumsalze hergestellt werden.

Eine pharmazeutische Zusammensetzung kann einen pharmazeutisch verträglichen Träger umfassen. Der Begriff "pharmazeutisch verträglicher Träger" betrifft offenbarungsgemäß einen oder mehrere kompatible feste oder flüssige Füllstoffe, Verdünnungsmittel oder Kapselsubstanzen, die für eine Verabreichung an einen Menschen geeignet sind. Der Begriff "Träger" betrifft einen organischen oder anorganischen Bestandteil, natürlicher oder synthetischer Natur, in dem der aktive Bestandteil kombiniert wird, um eine Anwendung zu erleichtern. Die Bestandteile der pharmazeutischen Zusammensetzung sind gewöhnlich derart, dass keine Interaktion auftritt, die die gewünschte pharmazeutische Wirksamkeit wesentlich beeinträchtigt.

Die pharmazeutischen Zusammensetzungen können geeignete Pufferstoffe wie Essigsäure in einem Salz, Citronensäure in einem Salz, Borsäure in einem Salz und Phosphorsäure in einem Salz enthalten.

Die pharmazeutischen Zusammensetzungen können auch gegebenenfalls geeignete Konservierungsstoffe wie Benzalkoniumchlorid, Chlorbutanol, Parabene und Thimerosal enthalten.

Die pharmazeutischen Zusammensetzungen werden gewöhnlich in einer einheitlichen Dosisform dargeboten und können in an sich bekannter Weise hergestellt werden. Erfindungsgemäße pharmazeutische Zusammensetzungen können beispielsweise in Form von Kapseln, Tabletten, Lutschpastillen, Suspensionen, Sirupen, Elixieren oder als Emulsion vorliegen.

Zusammensetzungen, die für eine parenterale Verabreichung geeignet sind, umfassen gewöhnlich eine sterile wässrige oder nicht-wässrige Zubereitung des Wirkstoffs, die vorzugsweise mit dem Blut des Empfängers isotonisch ist. Verträgliche Träger und Lösungsmittel sind beispielsweise Ringer-Lösung und isotonische Natriumchloridlösung. Zusätzlich werden gewöhnlich sterile, fixierte Öle als Lösungs- oder Suspensionsmedium eingesetzt.

Die vorliegende Lehre wird durch die nachstehenden Abbildungen und Beispiele ausführlich beschrieben, die ausschließlich der Erläuterung dienen und nicht begrenzend zu verstehen sind. Dem Fachmann sind aufgrund der Beschreibung und der Beispiele weitere Ausführungsformen zugänglich.

### Abbildungen:

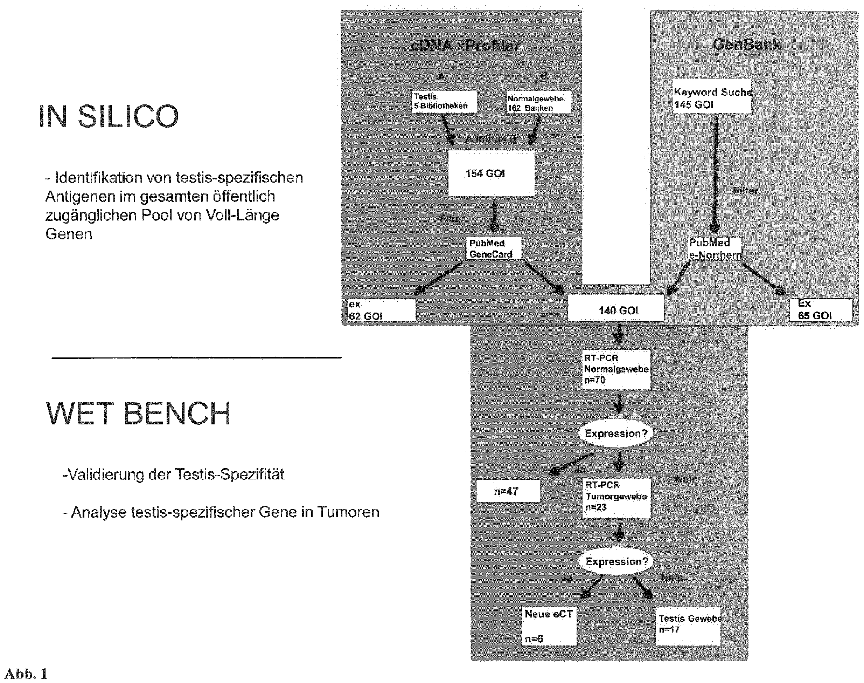

**Abb. 1****: Schematische Darstellung der Klonierung von eCT.** Die Strategie umfasst die Identifizierung von Kandidaten-Genen (GOI = "Genes of interest") in Datenbanken und das Testen dieser Gene durch RT-PCR.

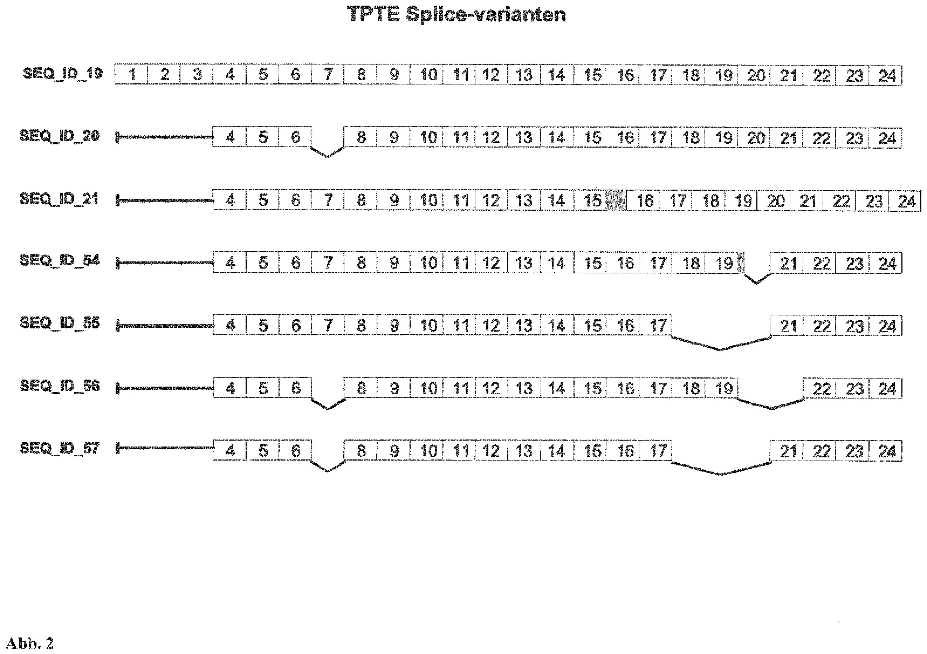

**Abb. 2****: Exon-Zusammensetzung von TPTE-Varianten.** Erfindungsgemäß wurden Splicevarianten identifiziert (SEQ ID NO:20, SEQ ID NO:21, SEQ ID NO:54, SEQ ID NO:55, SEQ ID NO:56, SEQ ID NO:57), die in testikulärem Gewebe und Tumoren exprimiert werden und Leserasterverschiebungen und somit veränderte Sequenzbereiche aufweisen.

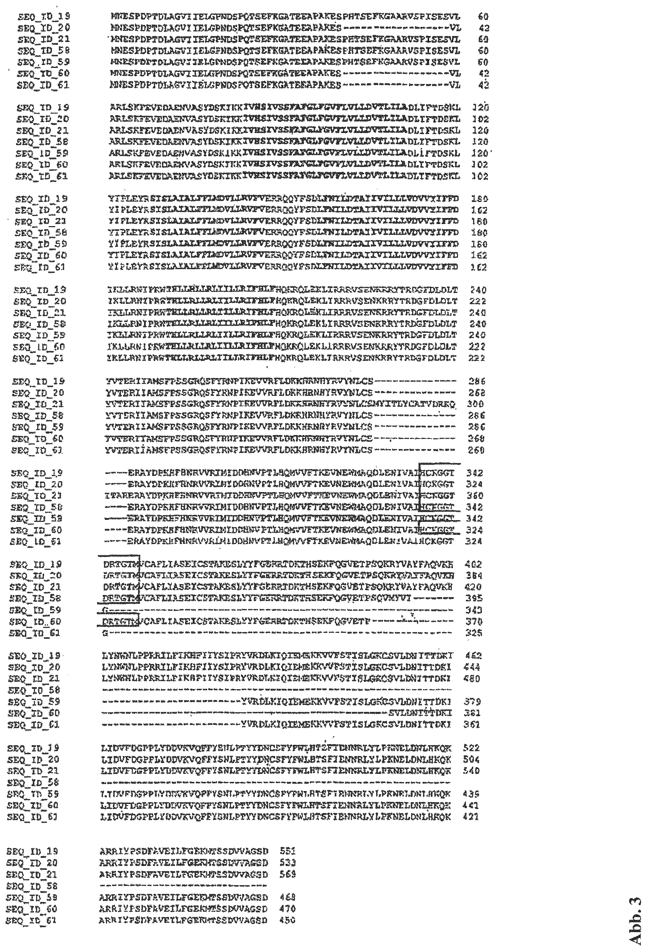

**Abb. 3****: Alignment der möglichen TPTE-Proteine.** Alternative Splicing-Ereignisse resultieren in Veränderungen des kodierten Proteins, wobei das Leseraster grundsätzlich erhalten bleibt. Die putativen transmembrandomänen sind fettgedruckt, die katalytische Domäne ist eingerahmt.

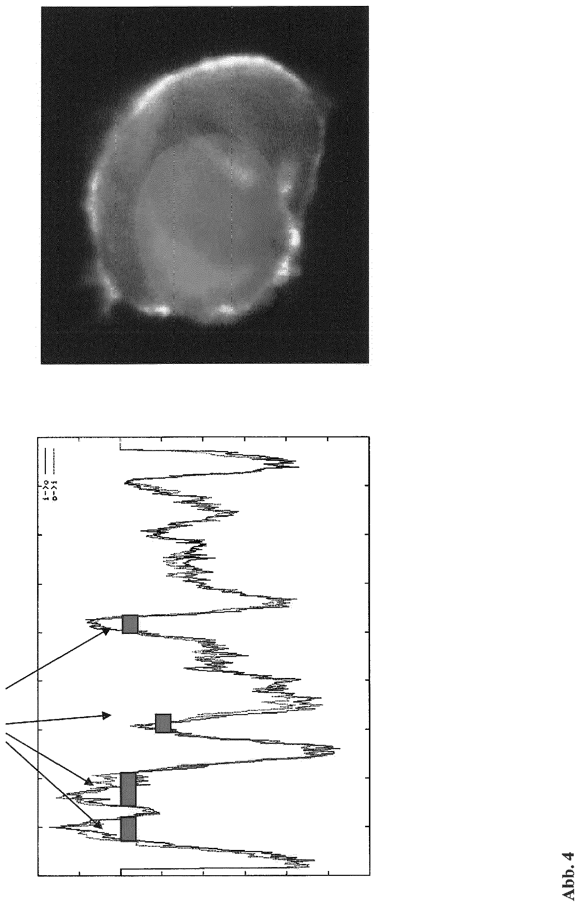

**Abb 4: Prädizierte Topologie von TPTE und subzelluläre Lokalisation an der Zelloberfläche von MCF-7 Zellen.** Das linke Schema zeigt die 4 putativen Trabsmembran-Domänen (Pfeile) von TPTE. MCF-7 Zellen wurden transient mit einem TPTE-Expressions-Plasmid transfiziert. Das Antigen wurde mit TPTE-spezifischen Antikörpern detektiert und zeigte eine deutliche Kolokalisation mit an der Zelloberfläche lokalisierten MHC I Molekülen.

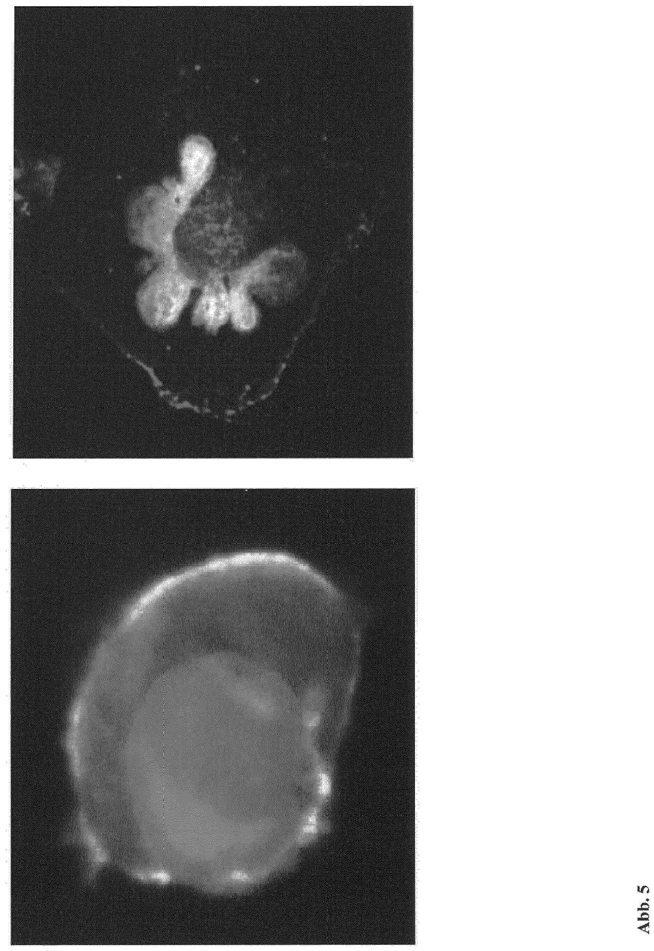

**Abb. 5****: Membranlokalisation von heterolog und endogen exprimierter TPTE in Zellinien.** (Links) NIH3T3 Zellen wurden transient mit einem TPTE-Expressions-Plasmid transfiziert. TPTE wurde mit spezifischen Antikörpern detektiert und zeigte eine deutliche Kolokalisation mit an der Zelloberfläche lokalisierten MHC I Molekülen. (Rechts) Endogene TPTE in SK-Mel 37 Zellen wurde mit einem spezifischen Antikörper detektiert und zeigt deutliche Membranlokalisation.

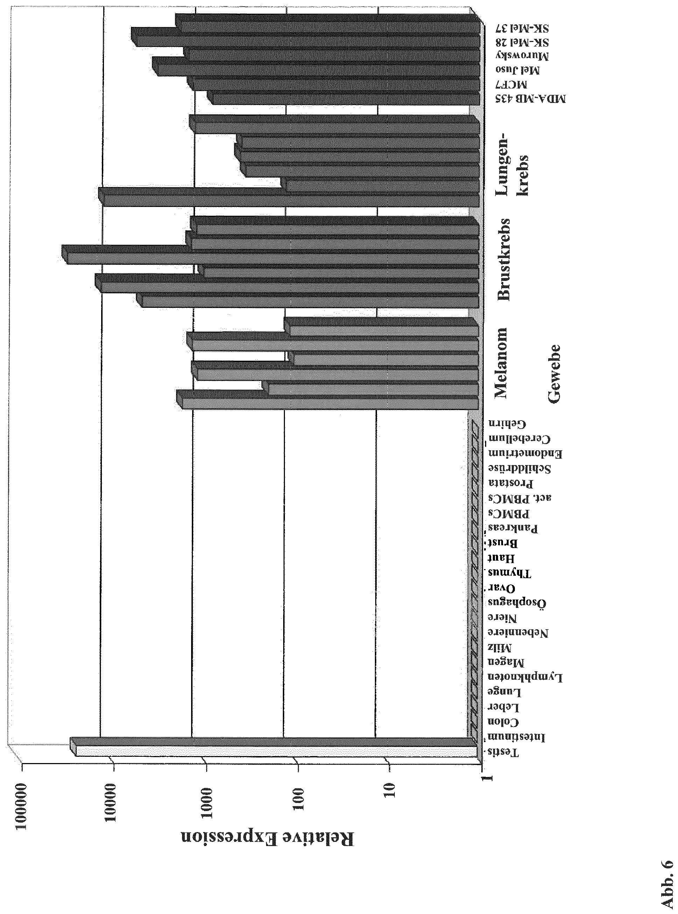

**Abb. 6****: Quantifizierung der TPTE mRNA Expression in verschiedenen Geweben durch Real-Time PCR.** Expression in Normalgeweben ist nur in Testis nachweisbar. Signifikante Expressionslevel finden sich auch in Tumoren und Tumorzellinien.

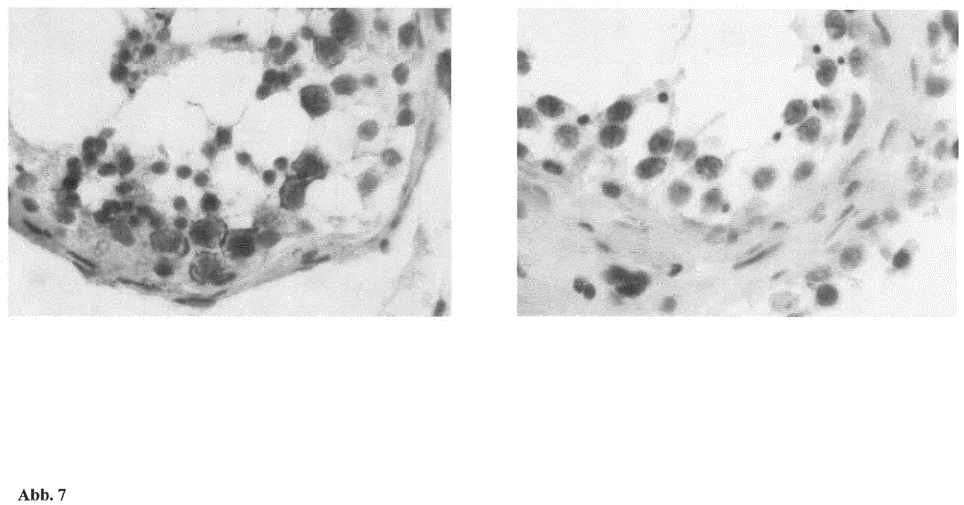

**Abb. 7****: Nachweis der Antiköper-Spezifität.** Schnitte von Testisgewebe wurden mit TPTE-spezifischem Antikörper gefärbt. Der spezifische Nachweis von TPTE wird durch Blocken (rechts) des Antikörpers mit dem zur Immunisierung eingesetzten Peptid verhindert.

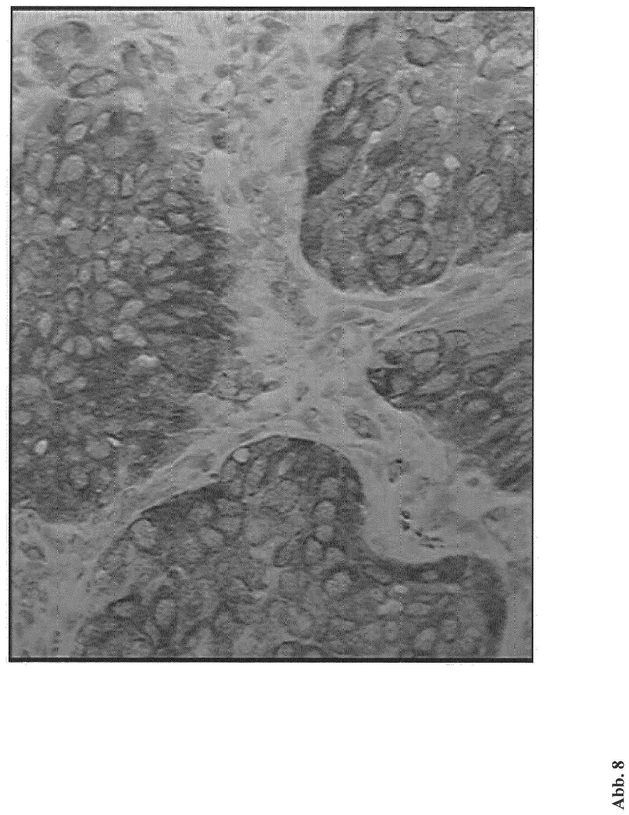

**Abb. 8****: Immunhistochemischer Nachweis von TPTE in Bronchialkarzinomen.** Schnitte von Bronchialkarzinomen wurden mit TPTE-spezifischem Antikörper gefärbt. TPTE wird homogen im gesamten Tumor exprimiert und ist an der Zellmembran lokalisiert.

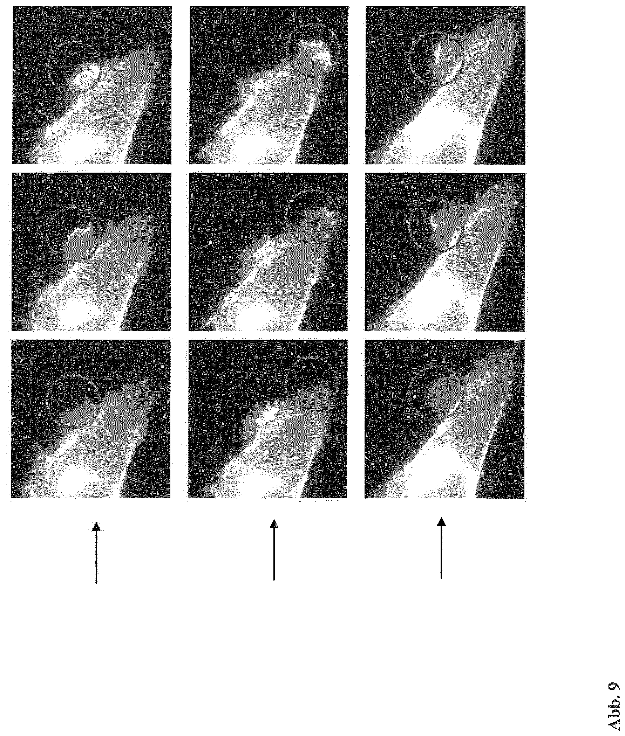

**Abb. 9****: Nachweis der TPTE-Lokalisation in vitalen Zellen.** NIH3T3 Zellen wurden transient mit einem TPTE-Expressions-Plasmid transfiziert und mittels Time-Lapse Mikroskopie analysiert. Die Lokalisation von TPTE an die Membran von Zellprotrusionen und Pseudoposien hat die unmittelbare Retraktion der betreffenden Membranbereiche zur Folge.

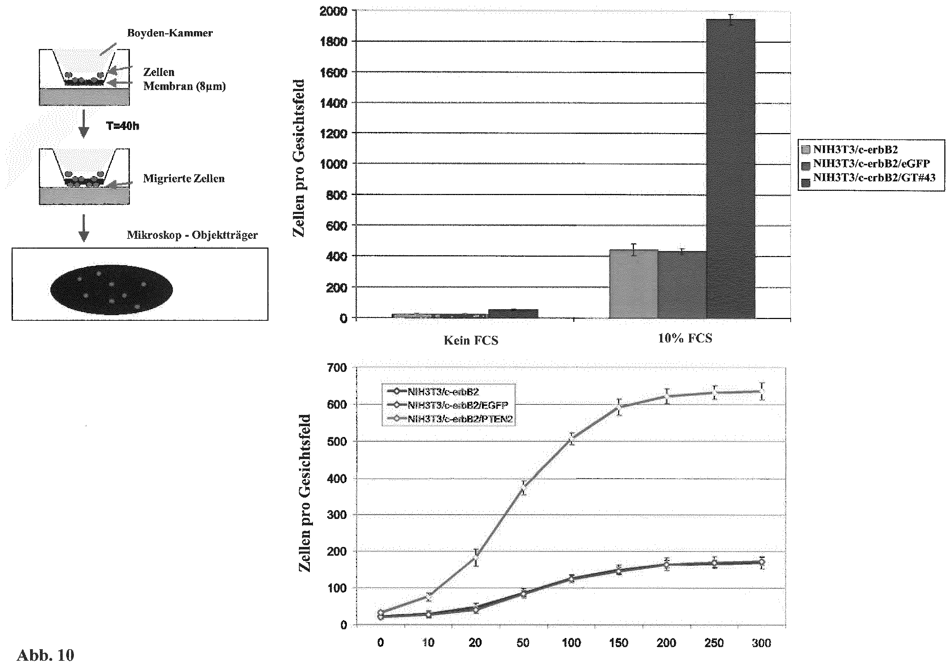

**Abb. 10****: TPTE verstärkt Zellmigration in chemotaktischen Gradienten.** (Links) Schematische Darstellung des Boyden-Kammer Assays. NIH3T3/c-erbB-2 Zellen wurden mit TPTE-eGFP transfiziert und die Migration der Zellen im Vergleich mit nicht-transfizierten und mit Leer-pEGFP-Vektor transfizierten Zellen bestimmt. (Rechts oben) Die Expression von TPTE führt zu einer um den Faktor 4-5 erhöhten Migration der Zellen bei Einsatz von 10%FCS als Chemotaktikum. (Recht unten) Die Expression von TPTE führt schon bei sehr geringen Konzentrationen von PDGF zu einer signifikanten Steigerung der Migration.

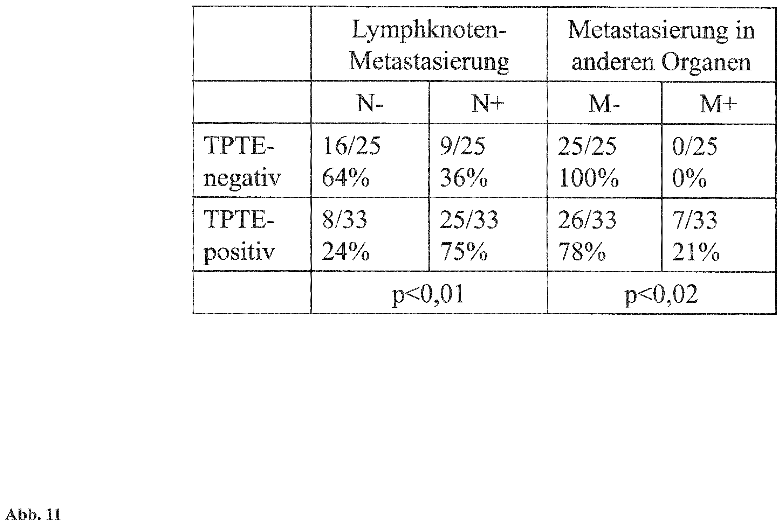

**Abb. 11****: Expression TPTE in Tumoren ist assoziiert mit Metastasierung.** Statistische Auswertung der TNM-Stadien von 58 Tumorproben in Korrelation mit der Expression von TPTE zeigt, dass TPTE positive Tumoren signifankt häufiger lymphogen und hämatogen metastasieren.

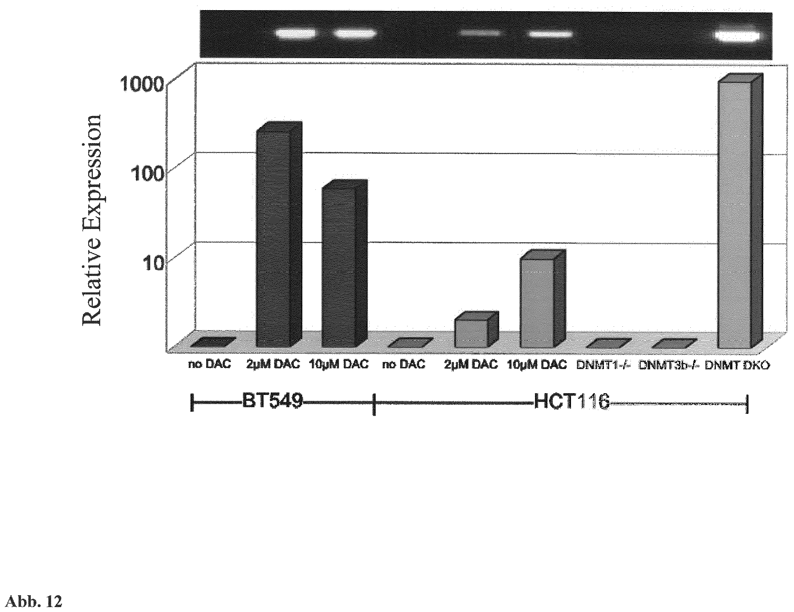

**Abb. 12****: Nachweis der Induzierbarkeit von TPTE durch genomische Demethylierung.** Real-Time RT-PCR Analyse der TPTE-Expression in nicht-exprimierenden Zellinien BT549 und HCT116 nach Behandlung der Zellen mit 2µM bzw. 10µM 5-Aza-2'-deoxycytidin und in DNA-Methyltransferase defizienten HCT116 Zellen.

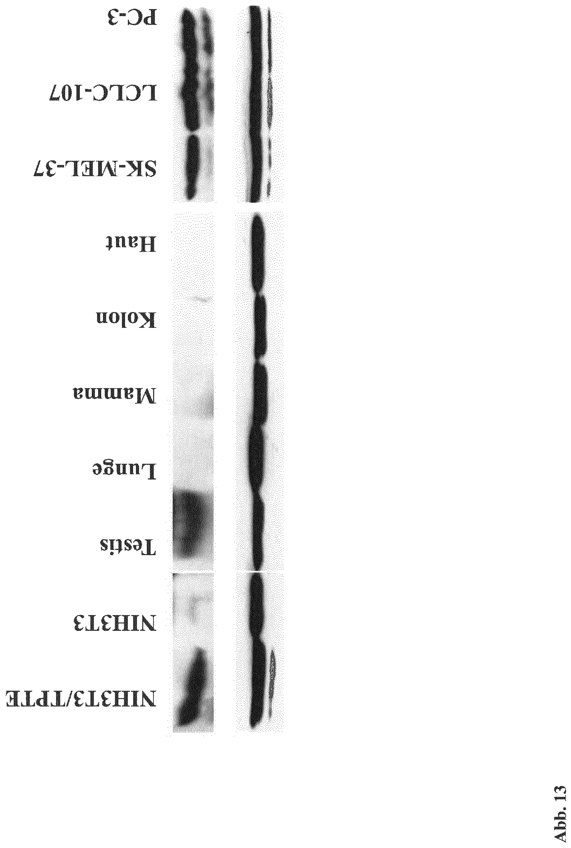

**Abb. 13****: Western Blot Nachweis von TPTE in Normalgeweben und Tumorzellinien.** Die Expression von TPTE ist nur in Testis und Tumorzellinien (SK-Mel-37, LCLC-107, PC-3) nachweisbar, alle anderen getesteten Normalgewebe sind negativ. Die Spezifität des Antikörpers wird durch den Nachweis von TPTE in transfizierten NIH3T3 Zellen (TPTEpcDNA3.1) bestätigt.

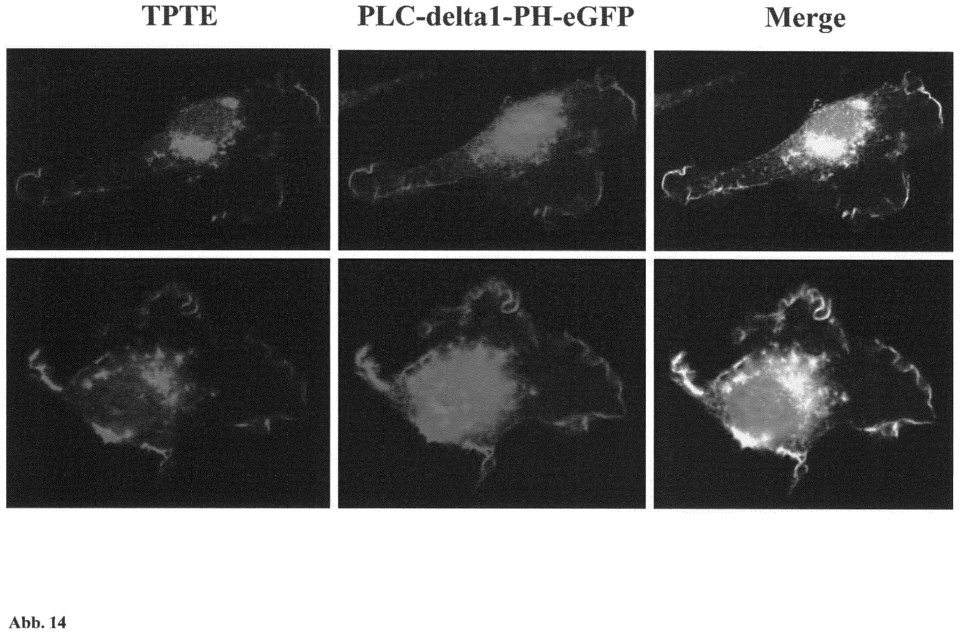

**Abb. 14****: TPTE kolokalisiert mit PIP4,5.** TPTE transfizierte NIH3T3/c-erb-2 Zellen wurden mit PH-eGFP von PLC-deltal kotransfiziert und mit TPTE-spezifischem Antikörper gefärbt. In der Überlagerung zeigt sich eine eindeutige Kolokalisation von TPTE mit PLC-deltal-PH-eGFP als Marker für PIP4,5.

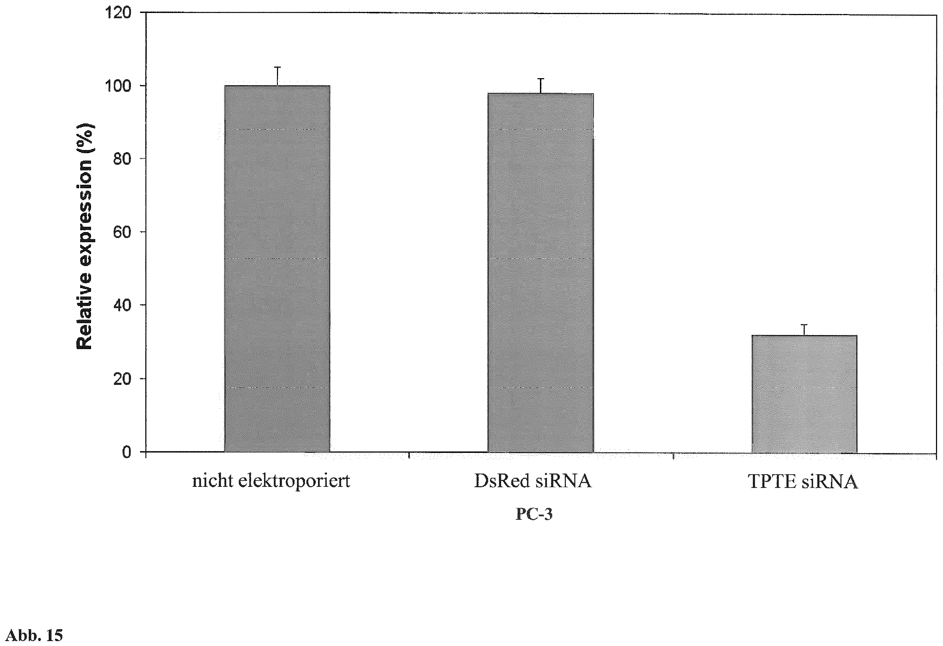

**Abb. 15****: Knock-down der TPTE-Expression in PC-3 Zellen durch RNAi. PC-3 Zellen** wurden mit 1µM siRNA spezifisch für TPTE elektroporiert. Nach 24h wurde die mRNA-Expression mittels Real-Time RT-PCR quantifiziert. Als Kontrolle dienten nichtelektroporierte Zellen und Zellen, die mit DsRed siRNA elektroporiert wurden.

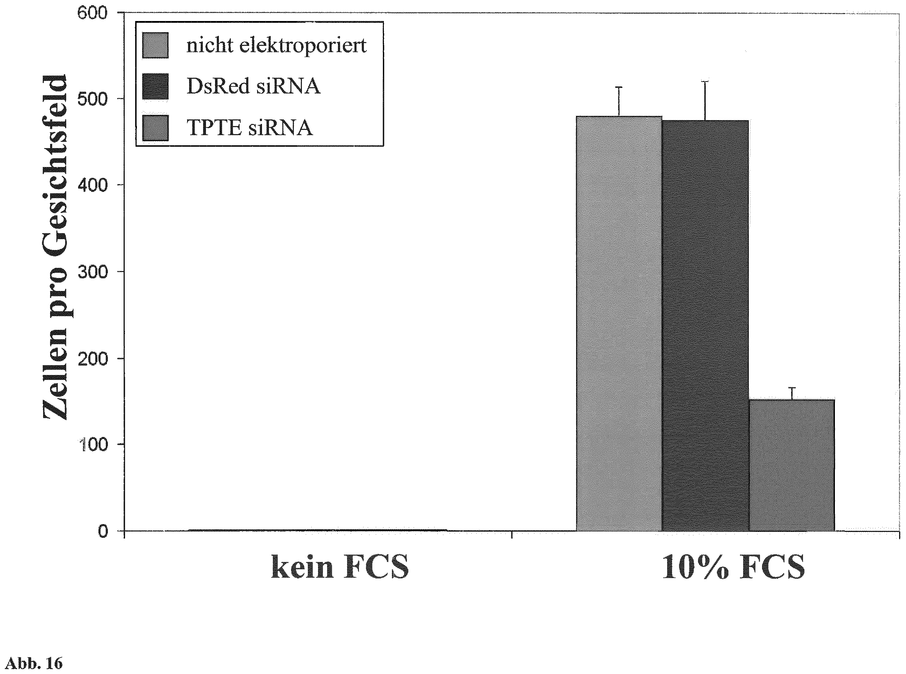

**Abb. 16****: Verminderung der Zellmigration von PC-3 Zellen nach Elektroporation mit TPTE-siRNA.** 48h nach Elektropration mit 1µM TPTE-siRNA zeigt sich eine relevante Abnahme der Zellmigration im Boyden-Kammer Assay.

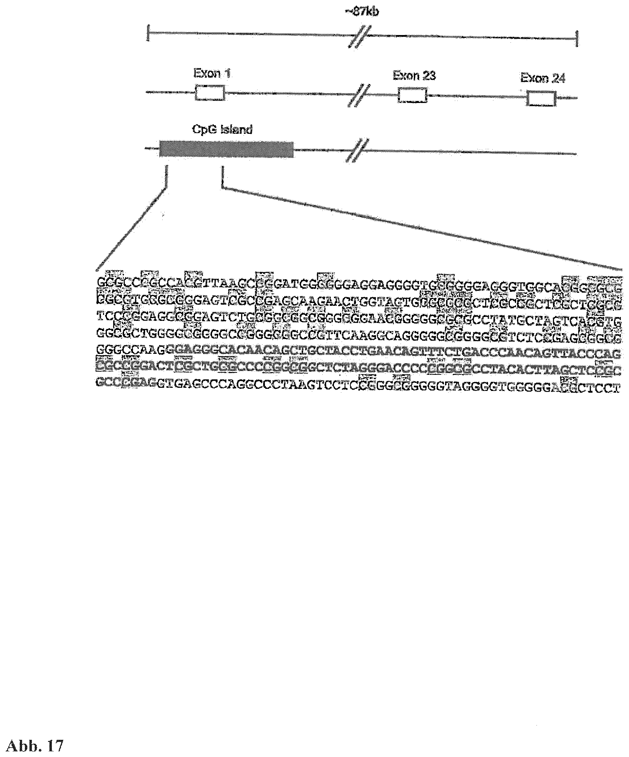

**Abb. 17****: Region von Position 121 bis 540 der in SEQ ID NO: 822 des Sequenzprotokolls gezeigten Sequenz.**

### Beispiele:

### Material und Methoden

Die Begriffe *"in silico",* "elektronisch" und "virtuell klonieren" beziehen sich rein auf die Nutzung von auf Datenbanken beruhenden Verfahren, mit denen auch Labor-experimentelle Vorgänge simuliert werden können.

Alle anderen Begriffe und Termini sind, falls nicht explizit anders definiert, so verwendet, wie sie der Fachmann versteht. Die genannten Techniken und Methoden erfolgen in an sich bekannter Weise und sind z.B. in Sambrook et al., Molecular Cloning: A Laboratory Manual, 2. Auflage (1989) Cold Spring Harbor Laboratory Press, Cold Spring Harbor, N. Y beschrieben. Alle Verfahren, die die Verwendung von Kits und Reagenzien einschließen, sind entsprechend den Angaben der Hersteller durchgeführt.

### Datamining-basierte Strategie zur Ermittlung von eCT (elektronisch klonierte Cancer/Testis-Gene)

Zwei *in silico* Strategien nämlich GenBank-Schlagwort-Suche und der cDNAxProfiler wurden kombiniert (Abb. 1). Es wurde unter Nutzung des ENTREZ Search and Retrieval Systems des NCBI (http://www.ncbi.nlm.nih.gov/Entrez) eine Suche nach Kandidaten-Genen in der GenBank durchgeführt, die annotiert sind als spezifisch exprimiert in testikulärem Gewebe (Wheeler et al., Nucleic Acids Research 28:10-14, 2000).

Durch Suchabfragen mit den Schlagworten "testis-specific gene", "sperm-specific gene", "spermatogonia-specific gene" wurden Kandidatengene (GOI, genes of interest) aus den Datenbanken herausextrahiert. Die Suche wurde auf einen Teil der Gesamtinformation dieser Datenbanken eingeschränkt, indem als Limits "homo sapiens" für den Organismus und "mRNA" für die Molekülart eingesetzt wurden.

Die Liste der gefundenen GOI wurde kuratiert, indem unterschiedliche Bezeichnungen für dieselbe Sequenz ermittelt und solche Redundanzen behoben wurden.

Alle Kandidatengene, die sich durch die Schlagwort-Suche ergaben, wurden wiederum durch das Verfahren des "electronic Northern" (eNorthern) bezüglich ihrer Gewebeverteilung untersucht. Der eNorthern basiert darauf, dass die Sequenz eines GOI gegenüber einer EST-(expressed sequence tag) Datenbank (Adams et al., Science 252:1651, 1991) abgeglichen wird (http://www.ncbi.nlm.nih.gov/BLAST). Zu jedem EST, das sich als homolog zum eingegebenen GOI ergibt, lässt sich die Gewebeherkunft ermitteln und durch die Summe aller ESTs auf diese Weise eine vorläufige Einschätzung der Gewebeverteilung des GOI erreichen. Nur diejenigen GOI wurden weiteren Untersuchungen zugeführt, die keine Homologien zu EST aus nicht-testikulären Normalgeweben mit Ausnahme von Plazenta und fetalem Gewebe hatten. Für dies Beurteilung wurde auch berücksichtigt, dass es falsch-annotierte cDNA Banken in der öffentlichen Domäne gibt (Scheurle et al., Cancer Res. 60:4037-4043, 2000) (www.fau.edu/cmbb/publications/cancergenes6.htm).

Als zweites Datamining-Verfahren wurde der cDNA xProfiler des Cancer Genome Anatomy Projekts des NCBI (http://cgap.nci.nih.gov/Tissues/xProfiler) genutzt (Hillier et al., Genome Research 6:807-828, 1996; Pennisi, Science 276:1023-1024, 1997). Dieser erlaubt Pools von in Datenbanken abgelegten Transkriptomen durch logische Operatoren in Beziehung zueinander zu setzen. Wir haben einen Pool A definiert, dem alle aus Testis hergestellten Expressionsbibliotheken, unter Ausschluss von gemischten Bibliotheken zugeordnet wurden. Dem Pool B wurden alle cDNA-Bibliotheken zugeordnet, die von Normalgeweben mit Ausnahme von Testis, Ovar und Fetalgewebe hergestellt waren. Generell wurden alle cDNA-Banken unabhängig vom zugrundeliegenden Herstellungsverfahren genutzt, allerdings lediglich solche mit einer Mächtigkeit > 1000 zugelassen. Mittels des BUT NOT Operators wurde Pool B digital von Pool A subtrahiert. Auch das Set der auf diese Weise gefundenen GOI wurde eNorthern-Studien unterzogen, sowie durch eine Literaturrecherche abgesichert. Dieses kombinierte Datamining schließt alle etwa 13 000 Vollänge-Gene in der öffentlichen Domäne ein und prädiziert aus diesen insgesamt 140 Gene mit potentieller Testis-spezifischer Expression. Unter diesen waren 25 bereits bekannte Gene der CT-Genklasse, was die Effizienz unserer Strategie unterstreicht.

Alle anderen Gene wurden zunächst durch spezifische RT-PCR in Normalgeweben evaluiert. Alle GOI, die sich als in nicht-testikulären Normalgeweben exprimiert erwiesen, hatten als Falsch-Positive zu gelten und wurden aus weiteren Untersuchungen ausgeschlossen. Die verbliebenen wurden in einem großen Panel an verschiedensten Tumorgeweben untersucht. Die unten dargestellten Antigene erwiesen sich dabei als ektop in Tumorzellen aktiviert.

### RNA-Extraktion, Herstellung von poly-d(T) geprimter cDNA und RT-PCR Analyse

Gesamt-RNA aus nativem Gewebematerial wurde unter Verwendung von Guanidiumisothiocyanate als chaotrophem Agens extrahiert (Chomczynski & Sacchi, Anal. Biochem. 162:156-9, 1987). Nach Extraktion mit saurem Phenol und Fällung mit Isopropanol wurde die RNA in DEPC-behandeltem Wasser gelöst.

Aus 2-4 µg Gesamt-RNA wurde in einem 20µl Reaktionsansatz mittels Superscript II (Invitrogen) entsprechend den Angaben des Herstellers eine Erststrang-cDNA-Synthese durchgeführt. Als Primer wurde ein dT(18) Oligonukleotid verwendet. Integrität und Qualität der cDNA wurden durch Amplifikation von p53 in einer 30 Zyklen-PCR (sense CGTGAGCGCTTCGAGATGTTCCG (SEQ ID NO: 52), antisense CCTAACCAGCTGCCCAACTGTAG (SEQ ID NO: 53), Hybridisierungstemperatur 67°C) überprüft.

Es wurde ein Archiv aus Erststrang-cDNAs aus einer Reihe von Normalgeweben und Tumorentitäten hergestellt. Für Expressionsstudien wurden 0,5 µ1 dieser cDNAs in einem 30µl Reaktionsansatz mit GOI-spezifischen Primern (siehe unten) und 1 U HotStarTaq DNA Polymerase (Qiagen) amplifiziert. Der Reaktionsansatz enthielt jeweils 0,3 mM dNTPs, je 0,3 µM jeden Primers und 3 µ1 10 x Reaktionspuffer.

Die Primer wurden so ausgewählt, dass sie in 2 verschiedenen Exons liegen und die Beseitigung der Interferenz durch kontaminierende genomische DNA als Grund für falsch positive Resultate wurde durch Testen von nicht revers transkribierter DNA als Matritze bestätigt. Nach 15 Minuten bei 95°C zur Aktivierung der HotStarTaq DNA Polymerase wurden 35 Zyklen PCR durchgeführt (1 min 94°C, 1 min jeweilige Hybridisierungstemperatur, 2 min 72°C und abschließende Elongation bei 72°C für 6 min). 20µl dieser Reaktion wurden auf einem mit Ethidiumbromid gefärbten Agarosegel aufgetrennt und analysiert.

Folgende Primer wurden für die Expressionsanalyse der entsprechenden Antigene bei der angegebenen Hybridisierungstemperatur verwendet.

### TPTE (64°C)

sense TGGATGTCACTCTCATCCTTG (SEQ ID NO: 27),
antisense CCATAGTTCCTGTTCTATCTG (SEQ ID NO: 28)

### Herstellung von Random-Hexamer-geprimter cDNA und quantitative Real-Time-PCR

Die Expression von LDHC wurde mittels Real-Time-PCR quantifiziert.

Das Prinzip der quantitativen Real-Time-PCR mit dem ABI PRISM Sequence Detection System (PE Biosystems, USA) nutzt die 5'-3' Exonuklease-Aktivität der Taq DNA Polymerase zur direkten und spezifischen Detektion von PCR-Produkten durch Freisetzung von Fluoreszenz-Reporterfarbstoffen. Neben Sense- und Antisense-Primern wird bei der PCR eine doppelt fluoreszenz-markierte Sonde (TaqMan-Probe) eingesetzt die an eine Sequenz des PCR-Produkts hybridisiert. Die Sonde ist 5' mit einem Reporterfarbstoff (z.B. FAM) und 3' mit einem Quencherfarbstoff (z.B. TAMRA) markiert. Ist die Sonde intakt, supprimiert die räumliche Nähe von Reporter zu Quencher die Emission der Reporterfluoreszenz. Hybridisiert die Sonde während der PCR an das PCR-Produkt, wird sie durch die 5'-3' Exonuklease-Aktivität der Taq DNA Polymerase gespalten und die Suppression der Reporterfluorszenz aufgehoben. Das Ansteigen der Reporterfluoreszenz als Folge der Zielamplifikation wird nach jedem PCR-Zyklus gemessen und zur Quantifizierung genutzt. Die Expressionsquantifizierung des Zielgens erfolgt absolut oder relativ zur Expression eines Kontrollgens mit konstanter Expression in den zu untersuchenden Geweben. Die Expression von LDHC wurde nach Normalisierung der Proben gegen 18s RNA als sog. Housekeeping-Gen mittels der ΔΔ-Cₜ Methode (PE Biosystems, USA) berechnet. Die Reaktionen wurden in Duplex-Ansätzen durchgeführt und in Duplikaten bestimmt. Die Synthese der cDNA erfolgte mit dem High Capacity cDNA Archive Kit (PE Biosystems, USA) unter Verwendung von Hexamer-Primern nach Angaben des Herstellers. Jeweils 5 µl der verdünnten cDNA wurden in 25 µl Gesamtvolumen für die PCR eingesetzt: sense-Primer (GGTGTCACTTCTGTGCCTTCCT) 300 nM; antisense-Primer (CGGCACCAGTTCCAACAATAG) 300 nM; TaqMan-Probe (CAAAGGTTCTCCAAATGT) 250 nM; sense-Primer 18s RNA 50 nM; antisense-Primer 18s RNA 50 nM; 18 s RNA-Probe 250 nM; 12,5 µl TaqMan Universal PCR Master Mix; anfängliche Denaturierung 95°C (10 min); 95°C (15 sec); 60°C (1 min); 40 Zyklen. Durch die Amplifikation eines 128 bp-Produkts über die Grenze von Exon 1 und Exon 2 wurden alle beschriebenen Splicevarianten von LDHC in die Quantifizierung einbezogen.

Die Expression von TPTE wurde ebenfalls mittels Real-Time-PCR quantifiziert, jedoch wurden die PCR-Produkte mit SYBR-Green als Reporterfarbstoff detektiert. Die Reporterfluoreszenz von SYBR-Green ist in Lösung supprimiert und erst nach Bindung an dopplesträngige DNA-Fragmente ist der Farbstoff aktiv. Das Ansteigen der SYBR-Green Fluoreszenz als Folge der spezifischem Amplifikation mittels GOI-spezifische Primern nach jedem PCR Zyklus wird zur Quantifizierung genutzt. Die Expressionsquantifizierung des Zielgens erfolgt absolut oder relativ zur Expression eines Kontrollgens mit konstanter Expression in den zu untersuchenden Geweben. Die Expression von LDHC wurde nach Normalisierung der Proben gegen 18s RNA als sog. Housekeeping-Gen mittels der ΔΔ-Cₜ Methode (PE Biosystems, USA) berechnet. Die Reaktionen wurden in Duplex-Ansätzen durchgeführt und in Triplikaten bestimmt. Verwendet wurde das QuantiTect SYBR-Green PCR Kit (Qiagen, Hilden) nach Angaben des Herstellers. Die Synthese der cDNA erfolgte mit dem High Capacity cDNA Archive Kit (PE Biosystems, USA) unter Verwendung von Hexamer-Primern nach Angaben des Herstellers. Jeweils 5 µl der verdünnten cDNA wurden in 25 µl Gesamtvolumen für die PCR eingesetzt: sense-Primer 300nM, antisense-Primer 300nM; initiale Denaturierung 95° 15 min; 95° 30 sec; Annealing 30 sec; 72° 30 sec; 40 Zyklen.

### TPTE (62°)

sense GAGTCTACAATCTATGCAGTG
antisense CCATAGTTCCTGTTCTATCTG

### Klonierung und Sequenzanalyse

Klonierung von Vollängen bzw. Genfragmenten erfolgte nach gängigen Methoden. Zur Ermittlung der Sequenz wurden entsprechende Antigene mittels der Proofreading-Polymerase pfu (Stratagene) amplifiziert. Nach Beendigung der PCR wurde Adenosin mittels HotStarTaq DNA Polymerase an die Enden des Amplikons ligiert, um die Fragmente entsprechend den Angaben des Herstellers in den TOPO-TA Vektor zu klonieren. Die Sequenzierung wurde durch einen kommerziellen Service durchgeführt. Die Sequenzen wurden mittels gängiger Prädiktionsprogramme und Algorithmen analysiert.

### Western blot

Zellen aus Zellkultur (endogene Expression des Zielgens oder Synthese des Zielproteins nach Transfektion eines Expressionsvektors, der das Zielprotein kodiert) oder Gewebeproben, die das Zielprotein enthalten könnten, werden in einer 1%igen SDS Lösung lysiert. Das SDS denaturiert dabei die im Lysat enthaltenen Proteine. Die Lysate eines experimentellen Ansatzes werden abhängig von der erwarteten Proteingröße auf 8-15 %igen denaturierenden Polyacrylamidgelen (enthalten 1% SDS) der Größe nach elekrophoretisch aufgetrennt (SDS-Polyacrylamid Gelelektrophorese, SDS-PAGE). Anschließend werden die Proteine durch das semi-dry Elektroblot Verfahren (Biorad) auf Nitrozellulose Membran (Schleicher & Schüll) transferiert, auf der das gewünschte Protein nachgewiesen werden kann. Dazu wird die Membran zunächst blockiert (z.B. mit Milchpulver) und anschließend mit dem spezifischen Antikörper in einer Verdünnung von 1:20-1:200 (je nach Spezifität des Antikörpers) für 60 Minuten inkubiert. Nach einem Waschschritt wird die Membran mit einem zweiten, mit einem Marker (z.B. Enzyme wie Peroxidase oder alkalische Phosphatase) gekoppelten Antikörper inkubiert, der den ersten Antikörper erkennt. Nach einem weiteren Waschschritt wird anschließend das Zielprotein in einer Farb- oder Chemilumineszenz-Reaktion auf der Membran mittels einer Enzymreaktion sichtbar gemacht (z.B. ECL, Amersham Bioscience). Das Ergebnis wird durch Aufnahme mit einer geeigneten Kamera dokumentiert.

### Immunfluoreszenz

Es werden Zellen etablierter Zelllinien benutzt, die entweder das Zielprotein endogen synthetisieren (Nachweis der RNA in der RT-PCR oder des Proteins im Western blot) oder aber vor der IF mit Plasmid-DNA transfiziert worden sind. Zur Transfektion von Zelllinien mit DNA sind die verschiedensten Methoden (z.B. Elektroporation, Liposomen basierte Transfektion, Calciumphosphatpräzipitation) gut etabliert (z.B. Lemoine et al. Methods Mol Biol. 1997; 75: 441-7). Das transfizierte Plasmid kann bei der Immunfluoreszenz das unmodifizierte Protein kodieren oder aber auch unterschiedliche Aminosäuremarker an das Zielprotein koppeln. Die wichtigsten Marker sind z.B. das fluoreszierende "green fluorescent protein" (GFP) in seinen verschiedenen differentiell fluoreszierenden Formen, kurze Peptidsequenzen von 6-12 Aminosäuren, für die hoch affine und spezifische Antikörper zur Verfügung stehen. Zellen, die das Zielprotein synthetisieren, werden mit Paraformaldehyd; Saponin oder Methanol fixiert. Anschließend können die Zellen bei Bedarf durch Inkubation mit Detergenzien (z.B. 0,2% Triton X-100) permeabilisiert werden. Nach der Fixierung/Permeabilisierung werden die Zellen mit einem primären Antikörper inkubiert, der gegen das Zielprotein oder gegen einen der gekoppelten Marker gerichtet ist. Nach einem Waschschritt wird der Ansatz mit einem zweiten, mit einem fluoreszierenden Marker (z.B. Fluorescin, Texas Red, Dako) gekoppelten Antikörper inkubiert, der an den ersten Antikörper bindet. Anschließend werden die so markierten Zellen mit Glycerin überschichtet und mit Hilfe eines Fluoreszenzmikroskops nach den Angaben des Herstellers analysiert. Spezifische Fluoreszenzemissionen werden dabei, abhängig von den eingesetzten Substanzen, durch spezifische Anregung erreicht. Die Analyse erlaubt in der Regel die sichere Lokalisation des Zielproteins, wobei zur Bestätigung der Antikörperqualität und des Zielproteins in Doppelfärbungen zusätzlich zum Zielprotein auch die gekoppelten Aminosäuremarker oder andere Markerproteine angefärbt werden, deren Lokalisation bereits in der Literatur beschrieben ist. Ein Sonderfall stellt das GFP und seine Derivate dar, dass direkt angeregt werden kann und selbst fluoresziert, so dass zum Nachweis keine Antikörper benötigt werden.

### Immunhistochemie

Die IHC dient im einzelnen dazu, um (1) die Menge an Zielprotein in Tumor und Normalgeweben abschätzen zu können, (2) zu analysieren, wie viele Zellen in Tumor und gesundem Gewebe das Zielgen synthetisieren, (3) den Zelltyp in einem Geweben (Tumor, gesunde Zellen) zu definieren, in dem das Zielprotein nachweisbar ist.

Je nach individuellem Antikörper müssen unterschiedliche Protokolle verwendet werden (z.B. "Diagnostic Immunohistochemistry by David J., MD Dabbs ISBN: 0443065667" oder in "Microscopy, Immunohistochemistry, and Antigen Retrieval Methods: For Light and Electron Microscopy ISBN: 0306467704").

Formalin fixierte (andere Fixierungen: z.B. Methanol) und in Paraffin eingebettete Gewebestücke mit einer Dicke von ca. 4µm werden auf einem Glasträger aufgebracht und z.B. mit Xylene deparaffiniert. Die Proben werden mit TBS-T gewaschen in Serum blockiert. Anschließend erfolgt die Inkubation mit dem ersten Antikörper (Verdünnung: 1:2 bis 1:2000) für 1-18 Stunden, wobei in der Regel affinitätsgereinigete Antikörper verwendet werden. Nach einem Waschschritt erfolgt eine ca. 30-60 minütige Inkubation mit einem zweiten mit Alkalischer Phosphatase (alternativ: z.B. Peroxidase) gekoppelten Antikörper, der gegen den ersten Antikörper gerichtet ist. Anschließend erfolgt eine Farbreaktion unter Verwendung der Alkalischen Phosphatase (Referenzen: Shi et al. J Histochem Cytochem 1991 39: 741-748; Shin et al. Lab Invest. 1991 64: 693-702). Zum Nachweis der Antikörperspezifität kann die Reaktion durch vorherige Zugabe des Immunogens blockiert werden.

### Immunisierung

(Siehe auch Monoclonal Antibodies: A Practical Approach by Philip Shepherd, Christopher Dean isbn 0-19-963722-9; Antibodies: A Laboratory Manualby Ed Harlow, David Lane ISBN: 0879693142; Using Antibodies: A Laboratory Manual: Portable Protocol NO. by Edward Harlow, David Lane, Ed Harlow ISBN: 0879695447).

Im Folgenden wird der Herstellungsprozess von Antikörpern kurz beschrieben, Details sind den zitierten Publikationen zu entnehmen. Zunächst werden Tiere (z.B. Kaninchen) durch eine erste Injektion des gewünschten Zielproteins immunisiert. Durch eine zweite oder dritte Immunisierung innerhalb eines definierten Zeitraums (ca. 2-4 Wochen nach der vorangegangenen Immunisierung) lässt sich die Immunantwort des Tieres gegen das Immunogen verstärken. Wiederum nach verschiedenen definierten Zeitabständen (1. Blutung nach 4 Wochen, anschließend ca. alle 2 Wochen mit insgesamt bis zu 5 Entnahmen) wird den Tieren Blut entnommen und daraus ein Immunserum gewonnen.

Die Immunisierung der Tiere erfolgt in der Regel über eines von vier gut etablierte Verfahren, wobei auch andere Verfahren existieren. Immunisiert werden kann dabei mit Peptiden, die für das Zielprotein spezifisch sind, dem gesamten Protein, mit extrazellulären Teilsequenzen eines Proteins, das experimentell oder über Prediktionsprogramme identifiziert werden kann.
(1) Im ersten Fall werden an KLH (keyhole limpet hemocyanin) konjugierte-Peptide (Länge: 8-12 Aminosäuren) über ein standardisiertes in vitro Verfahren synthetisiert und diese Peptide zur Immunisierung verwendet. In der Regel erfolgen 3 Immunisierungen mit einer Konzentration von 5-1000 µg/Immunisierung. Die Durchführung der Immunisierung kann auch als Service von Dienstleistern erfolgen.
(2) Alternativ kann die Immunisierung durch rekombinante Proteine erfolgen. Dazu wird die klonierte DNA des Zielgens in einen Expressionsvektor kloniert und das Zielprotein analog den Bedingungen des jeweiligen Herstellers (z.B. Roche Diagnostics, Invitrogen, Clontech, Qiagen) z.B. zellfrei in vitro, in Bakterien (z.B. E. coli), in Hefe (z.B. S. pombe), in Insektenzellen oder in mammalen Zellen synthetisiert. Nach Synthese in einem der Systeme wird das Zielprotein aufgereinigt, die Aufreinigung erfolgt dabei in der Regel über standardisierte chromatografische Methoden. Dabei können auch Proteine für die Immunisierung verwendet werden, die über einen molekularen Anker als Hilfsmittel zur Reinigung verfügen (z.B. His-Tag, Qiagen; FLAG-Tag, Roche Diagnostics; Gst Fusionsproteine). Eine Vielzahl von Protokollen befinden sich z.B. in den "Current Protocols in Molecular Biology (John Wiley & Sons Ltd, Wiley InterScience).
(3) Falls eine Zelllinie zur Verfügung steht, die das gewünschte Protein endogen synthetisiert, kann auch diese Zelllinie zur Herstellung des spezifischen Antiserums verwendet werden. Die Immunisierung erfolgt dabei in 1-3 Injektionen mit jeweils ca. 1-5 x 10⁷ Zellen.
(4) Die Immunisierung kann auch durch Injektion von DNA (DNA Immunisierung) erfolgen. Dazu wird das Zielgen zunächst in einen Expressionsvektor kloniert, so dass die Zielsequenz unter der Kontrolle eines starken eukaryontischen Promotors steht (z.B. CMV Promotor). Anschließend werden 5-100 µg DNA als Immunogen mit einer "gene gun" in stark durchblutete, kapillare Bereiche eines Organismus transferiert (z.B. Maus, Kaninchen). Die transferierte DNA wird von Zellen des Tieres aufgenommen, das Zielgen wird exprimiert und das Tier entwickelt schließlich eine Immunantwort gegen das Zielgen (Jung et al., Mol Cells 12: 41-49, 2001; Kasinrerk et a., Hybrid Hybridomics 21: 287-293, 2002).

### Affinitätsreinigung

Die Reinigung der polyklonalen Seren erfolgte im Fall der Peptidantikörper gänzlich oder im Fall der Antikörper gegen rekombinante Proteine teilweise als Service durch die beauftragten Firmen. Hierzu wurde in beiden Fällen das entsprechende Peptid bzw. rekombinante Protein kovalent an eine Matrix gebunden, diese nach der Kopplung mit einem nativen Puffer (PBS: phosphate buffered saline) äquilibriert und im Anschluß mit dem Rohserum inkubiert. Nach einem weiteren Waschschritt mit PBS wurde der Antikörper mit 100 mM Glycin pH 2,7 eluiert und das Eluat sogleich in 2 M TRIS pH 8 neutralisiert. Die so gereinigten Antikörper konnten dann zur spezifischen Detektion der Zielproteine sowohl durch Westernblotting als auch durch Immunfluoreszenz eingesetzt werden.

### Boydenkammer Migrationsassay

Die Boydenkammer dient der Quantifizierung der Zellmigration in Reaktion auf chemotaktische Stimuli. Die Kammer besteht aus zwei Kompartimenten die durch eine Micropore-Membran voneinander getrennt sind. In das obere Kompartment gibt man die zu untersuchenden Zellen in Minimalmedium, währen das untere Kompartment mit Medium gefüllt wird welches das entsprechende Chemotaktikum enthält. Die Zellen wandern entsprechend des Gradienten durch die Membran und adhärieren an die Unterseite der Membran. Nach Fixation können die transmigrierten Zellen unter dem Mikroskop ausgezählt werden.

Der Migrationsassay wurde zur Bestimmung des promigratorischen Potentials von TPTE durchgeführt. Eingesetzt wurden mit TPTE-eGFP transfizierte, c-erbB2 transformierte NIH3T3 Fibroblasten. Als Kontrollzellen wurden nicht-transfizierte Zellen und mit eGFP-N3 Leervektor transfizierte Zellen eingesetzt. Für den Assay wurden Transwell Kammern (Becton Dickinson) mit einer Porengrösse von 8,0 µM in der Membran verwendet. Jeweils 4x10⁴ Zellen in 400 µl Serum-freiem DMEM Medium wurden in das obere Kompartiment gegeben. Das untere Kompartiment wurde mit 800 µl DMEM Medium gefüllt, dem 10%FCS oder PDGF-BB in aufsteigenden Konzentrationen (10-300ng/µl) zugefügt wurde. Die Kammern wurden für 40h bei 37° inkubiert. Anschliessend wurden die transmigrierten Zellen in eiskaltem Methanol fixiert, die Membranen wurden ausgeschnitten und auf Objektträgern mit Hoechst Kernfarbstoff (DAKO) zur Fluoreszenzmikroskopie eingedeckt. Zellen in fünf Gesichtsfeldern (Vergrösserung 20fach) pro Membran wurden ausgezählt. Alle Experimente wurden in Triplikaten durchgeführt.

### RNA-Interferenz (RNAi)

Die siRNA-Oligos wurden entsprechend der Tuschl-Kriterien ausgewählt (Elbashir et al., Nature 411(6836):428-9, 2001). TPTE siRNA-Oligos (sense 5'-CCCUGCCACAUGUUCAUAUdTdT-3'; antisense 5'-AUAUGAACAUGUGGCAGGGdTdT-3') targetierten die Nukleotide 2043-2061 der TPTE mRNA-Sequenz (NM_013315). Als Kontrolle wurden siRNA-Oligos spezifisch für das irrelevante DsRed-Fluoreszenzprotein (AF506025) eingesetzt (sense 5'-AGUUCCAGUACGGCUCCAAdTdT-5'; antisense 5'-UUGGAGCCGUACUGGAACUdTdT-3'). Zur Generierung der siRNA-Duplexe wurden jeweils 200µM der entsprechenden sense- und antisense-Oligos in Hybridisierungspuffer (30mM HEPES (pH 7,4), 100mM Natrium-Acetat, 2mM Magnesium-Acetat) nach initialer Denaturierung (1 min. bei 90°C) für 1h bei 37°C inkubiert.

### siRNA-Elektroporation

5x10⁶ Zellen wurden in 250µl Serum-freiem X-VIVO 15 Medium aufgenommen und mit 1µM der entsprechenden siRNA-Duplexe elektroporiert (200V, 250µF). Die TPTE mRNA-Expression wurde 24h später mittels Real-Time RT-PCR quantifiziert.

### Methylierungsstudien

Zur Untersuchung der Induzierbarkeit der TPTE-Expression durch genomische Demethylierung wurden die TPTE-negative Zellinien BT549 (Mamma-Ca) und HCT116 (Kolon-Ca) für 72h mit 2µM bzw. 10µM 5-Aza-2'-deoxycytidin kultiviert. Anschliessend wurde die TPTE-Expression mittels Real-Time RT-PCR quantifiziert. Desweiteren wurde die TPTE-Expression in DNA-Methyltransferase (DNMT) defizienten HCT116 Zellen quantifiziert. Während DNMT1 (HCT116^{DNMT1-/-}) bzw. DNMT3b (HCT116^{DNMT3b-/-}) defiziente Zellen ein nahezu unverändertes Methylierungsmuster im Vergleich zur parentalen Zellinie aufweisen (HCT116^{par}), sind Zellen in denen beide DNMTs gemeinsam deletiert sind (HCT116^{DKO}) durch fast vollständige Demethylierung der genomischen DNA gekennzeichnet.

### Time-Lapse Mikroskopie

Die Membranlokalisation von TPTE und die Beteilung an der Regulation von Membrandynamiken in vitalen Zellen wurde mittels Time-Lapse Mikroskopie untersucht. Hierfür wurden TPTE-eGFP transfizierte Zellen für 12h in serum-freiem DMEM Medium inkubiert. Zur Induktion von spontanen Zellmemdynamiken in Form von Membranprotrusionen, Pseudopodien und Filopodien wurden die Zellen vor der Analyse durch Zugabe von FCS stimuliert. Bilder der vitalen Zellen wurden in 30sec Abständen mit einem Invert Olympus Mikroskop (IX70) mit einer TILL IMAGO-VGA CCD Kamera aufgenommen.

### Herstellung EGFP-Transfektanten

Für die Immunfluoreszenz-Mikroskopie von heterolog exprimierter TPTE wurde der komplette ORF von TPTE in pEGFP-Cl und pEGFP-N3 Vektoren (Clontech) kloniert. Auf Objekträgern kultivierte CHO und NIH3T3 Zellen wurden mit den entsprechenden Plasmidkonstrukten unter Verwendung von Fugene Transfektionsreagenz (Roche) nach Herstellerangaben transfiziert und nach 12-24h mitels Immunfluoreszenz-Mikroskopie anlysiert.

### Prädiktion von Peptidepitopen für MHC Klasse I

Peptidepitope aus jeder der Proteinsequenzen mit Bindungseigenschaften für die polymorphen HLA-Allele A*0201, A*2402, A*0101, A*0301, B*0702 wurden unter Nutzung des unter http://syfpeithi.bmi-heidelberg.com/ abgelegten Prädiktionalgorithmus ermittelt. Als Peptidlänge wurden 8, 9 und 10mere vorgegeben und ein Cutoff bei einem Score von 15 gesetzt. Ergab die Suche weniger als 10 Peptide für die jeweilige Länge, wurden alternativ die 10 besten Peptide ausgewählt.

### Beispiel 1: Identifizierung von TPTE als neues Tumorantigen

Die Sequenzen des Transkripts von TPTE (SEQ ID NO:19) und seines Translationsprodukt (SEQ ID NO:22) sind in GenBank unter der Zugangsnummer NM_013315 veröffentlicht (Walker, S.M. et al., Biochem. J. 360(Pt 2):277-83, 2001; Guipponi M. et al., Hum. Genet. 107(2):127-31, 2000; Chen H. et al., Hum. Genet. 105(5):399-409, 1999). TPTE wurde als Gen, das für eine mögliche transmembrane Tyrosinphosphatase kodiert, mit Testisspezifischer Expression beschrieben, die in der perizentromeren Region der Chromosomen 21, 13, 15, 22, and Y liegt (Chen, H. et al., Hum. Genet. 105:399-409, 1999). Chen et al. beschreibt auch die biologischen Sequenzen gemäß SEQ ID NOs: 19 und 22. Erfindungsgemäße Abgleichuntersuchungen zeigen zusätzlich homologe genomische Sequenzen auf den Chromosomen 3 und 7. WO02/10363 beschreibt eine Nukleinsäure, die für eine humane Proteinphosphatase 1 (PP1) kodiert und die über einen Bereich von 972 Nukleotiden im Vergleich zu SEQ ID NO: 20 eine Sequenzidentität von 99% aufweist. Darüberhinaus erwähnt Dong et al. 2003, dass TPTE-Expression u.a. bei bestimmten Leberkarzinomen nachweisbar ist (Br J Cancer, 89: 291-297).

Die Membranlokalisation, die z.B. für die Erreichbarkeit von therapeutischen Antikörpern Voraussetzung ist, konnte zweifelsfrei nachgewiesen werden durch Transfektion von TPTE-negativen Zellen mit einem Fusionskonstrukt aus TPTE und Green-Fluorescent-Protein (Abb. 18, rechts). Dieses reichert sich an der Membranoberfläche an und lässt sich dort als kolokalisierend mit anderen bekannten Membranmarkern wie HLA-Molekülen nachweisen.

Basierend auf der Sequenz von TPTE (SEQ ID NO:19) wurden erfindungsgemäß PCR-Primer generiert (5'-TGGATGTCACTCTCATCCTTG-3' (SEQ ID NO: 27) and 5'-CCATAGTTCCTGTTCTATCTG-3' (SEQ ID NO: 28)) und für RT-PCR-Analysen (95° 15min; 94° 1 min; 63° 1 min; 72° 1 min; 35 Zyklen) in einer Reihe von humanen Geweben eingesetzt. Es zeigte sich, dass die Expression in Normalgeweben auf Testis beschränkt ist. Wie für die anderen eCT beschrieben, konnte erfindungsgemäß gezeigt werden, dass TPTE-Varianten ektop in einer Reihe von Tumorgeweben aktiviert werden; vgl. Tabelle 1.

**Tabelle 1. Expression von TPTE in Tumoren**

| **Gewebe** | **insgesamt getestet** | **Positiv** | **%** |
|---|---|---|---|
| **Melanom** | **18** | **9** | **50** |
| **Mammakarzinome** | **36** | **17** | **47** |
| **Kolorektale Tumoren** | **20** | **0** | **0** |
| **Prostatakarzinome** | **8** | **3** | **38** |
| **Bronchialkarzinome** | **45** | **25** | **55** |
| **Nierenzellkarzinome** | **7** | **2** | **29** |
| **Ovarialkarzinome** | **7** | **2** | **29** |
| **Schilddrüsenkarzinome** | **4** | **0** | **0** |
| **Zervixkarzimome** | **6** | **1** | **17** |
| **Melanom Zellinien** | **8** | **5** | **62** |
| **Bronchialkarzinom Zellinien** | **6** | **2** | **33** |
| **Mammakarzinom Zellinien** | **5** | **4** | **80** |

Es wurde eine quantitative Realtime-PCR (40 Zyklen, initiale Denaturierung 15 min 95°C, 30 sek 94°C, 30 sek 62°C, und 30 sek 72°C) mit den spezifischen Primern (sense GAGTCTACAATCTATGCAGTG; antisense CCATAGTTCCTGTTCTATCTG) durchgeführt (Abb. 6). Diese bestätigt nicht nur das Fehlen von TPTE in nicht-testikulärem Normalgewebe und die ektope Expession in Tumoren, sondern deckt hohe Transkriptmengen in den Tumoren auf, die mit der physiologischen expression in Testisgewebe durchaus vergleichbar sind. Die ektope Expression vieler bekannter CT-Gene in Tumoren wird durch Promotordemethylierung induziert. Zur Untersuchung ob dieser Mechanismus auch für die ektope Aktivierung von TPTE effektiv ist, wurden die nicht-exprimierenden Zellinien BT549 (Mamma-Ca) und HCT116 (Kolon-Ca) mit 5-Aza-2'-deoxycytidin, einem pharmakologischen Inhibitor der DNA-Methylierung, kultiviert. In beiden Fällen resultierte die Demethylierung der DNA in einer starken Induktion der TPTE-Expression (Abb. 12). Der gleiche Effekt zeigt sich auch in DNA-Methyltransferase DNMT1 und DNMT3b defizienten HCT116 Zellen, die durch fast vollständigen Verlust der DNA-Methylierung charakterisiert sind. Diese Ergebnisse belegen dass die Promoterdemethylierung des Genlokus auch für die ektope Induktion der TPTE-Expression effektiv ist.

Zum Nachweis von TPTE Protein wurden Antikörper durch Immunisieren von Kaninchen hergestellt. Folgende Peptide bzw. ein rekombinant in E. coli exprimiertes und gereinigtes Protein wurden zur Propagierung dieser Antikörper genutzt:
FTDSKLYIPLEYRSC (aa 116 - 128, SEQ ID NO: 103)
CFDIKLLRNIPRWT (aa 179 - 191, SEQ ID NO: 104)
MNESPDPTDLAGVIIELGPNDSPQTSEFKGATEEAPAKESPHTSEFKGAARVSP (rek.Prot. aa 1-54, SEQ ID NO: 105)

Beispielhaft sollen die Daten dargestellt werden für Antikörper, die gegen SEQ ID NO:105 gerichtet sind. Der spezifische Antikörper lässt sich unter verschiedenen Fixationsbedigungen für Immunfluoreszenz-Untersuchungen nutzen. Bei vergleichenden Färbungen von RT-PCR positiven wie auch negativen Zellinien ist das entsprechende Protein in gut nachweisbarer Menge spezifisch in den als positiv typisierten Zellinien detektierbar (Abb. 5, rechts). Das endogene Protein ist membranlokalisiert. Dieser Antikörper wurde desweiteren eingesetzt zur immunhistochemischen Färbung von Gewebsschnitten. Wie erwartet färbt der Antikörper Testisgewebe spezifisch an. Insbesondere sind es Keimzellen, die angefärbt werden (Abb. 7). Auch Gewebeschnitte von Lungenkarzinomen lassen sich anfärben. TPTE Protein lässt sich dabei homogen und in grosser Menge in allen Zellen eines Tumors detektieren. Auch hier bestätigt sich die Lokalisation des Proteins an der Membran der Zellen (Abb. 8). Die Spezifität der Färbung wurde durch Kompetitionsexperimente bestätigt. Der spezifische Antikörper wurde zunächst mit rekombinantem TPTE Protein vor-inkubiert und danach auf die Gewebeschnitte (z.B. Testis als Positivkontrolle, Abb. 7) verbracht. Hierdurch lässt sich die Reaktivität erfolgreich blockieren. Mit diesem Antikörper lassen sich viele verschiedene primäre Tumortypen (auch Brusttumoren, Melanome etc.) aber nicht das dazugehörige Normalgewebe erfolgreich anfärben. Beispielhaft ist auch dargestellt eine Färbung von Prostatagewebe. Normales Prostatagewebe ist negativ, während invasive wie auch noch nicht invadierte Prostatatumoren positiv für dieses Molekül sind. Auch eine einfache Hypertrophie von Prostata-Epithel färbt sich nicht an, allerdings innerhalb dieser gutartigen Veränderung bereits vorliegende premaligne Bereiche, die in der Folge transformieren werden sind TPTE positiv. Entsprechend kann ein solcher spezifischer Antikörper gegen TPTE dazu dienen, diagnostisch eine bestehende Anlage für Prosatakarzinome früh zu detektieren. Desweiteren wurde der Antikörper zum Expressionsnachweis von TPTE im Western-Blot eingesetzt. Proteinexpression konnte in Lysaten aus Testisgewebe und spontan exprimierenden Zellinien nachgewiesen werden, Normalgewebe zeigten wie erwartet keine Expression (Abb. 13).

Erfindungsgemäß wurden für TPTE weitere Splicevarianten identifiziert (SEQ ID NO:20, SEQ ID NO:21, SEQ ID NO:54, SEQ ID NO:55, SEQ ID NO:56, SEQ ID NO:57), die in testikulärem Gewebe und Tumoren exprimiert werden und Leserasterverschiebungen und somit veränderte Sequenzbereiche aufweisen (Abb. 2).

Die genomische Sequenz von TPTE besteht aus 24 Exons (Zugangsnummer NT_029430). Das in SEQ ID NO:19 gezeigte Transkript enthält alle diese Exons. Die in SEQ ID NO:20 gezeigte Splicevariante entsteht durch heraussplicen von Exon 7. Die in SEQ ID NO:21 gezeigte Splicevariante zeigt eine partielle Inkorporation eines Introns, das auf Exon 15 folgt. Wie aus den Varianten SEQ ID NO:54, SEQ ID NO:55, SEQ ID NO:56, SEQ ID NO:57 ersichtlich, können alternativ auch Exons 18, 19, 20 und 21 herausgesplict werden. Diese alternativen Splicing-Ereignisse resultieren in Veränderungen des kodierten Proteins, wobei das Leseraster grundsätzlich erhalten bleibt (Abb. 3). Beispielsweise weist das Translationsprodukt, das von der in SEQ ID NO:20 gezeigten Sequenz kodiert wird (SEQ ID NO:23) eine Deletion von 13 Aminosäuren im Vergleich zu der in SEQ ID NO:22 gezeigten Sequenz auf. Das Translationsprodukt, das von der in SEQ ID NO:21 gezeigten Sequenz kodiert wird (SEQ ID NO: 24) trägt eine zusätzliche Insertion im zentralen Bereich des Moleküls und unterscheidet sich dadurch um 14 Aminosäuren von den anderen Varianten. Die Translationsprodukte der Varianten SEQ ID NO:54, SEQ ID NO:55, SEQ ID NO:56, SEQ ID NO:57, nämlich die Proteine SEQ ID NO:58, SEQ ID NO:59, SEQ ID NO:60, SEQ ID NO:61, sind ebenfalls verändert. Analysen zur Prädiktion funktioneller Domänen ergeben für SEQ ID NO:22, SEQ ID NO:23, SEQ ID NO:24, SEQ ID NO:58, SEQ ID NO:60, aber nicht für SEQ ID NO:59, SEQ ID NO:61, das Vorhandensein einer Tyrosinphosphatase-Domäne. Für alle Varianten werden 3-4 Transmembrandomänen prädiziert (Abb. 3).

Die Analyse der TPTE-Antigen-Expression mit spezifischen Antikörpern bestätigte die selektive Expression in Testis sowie in einer Reihe von unterschiedlichen Tumoren. Durch Kolokalisationsuntersuchungen zeigte sich zudem, dass TPTE erfindungsgemäß mit Klasse I Histokompatibilitätsantigenen an der Zelloberfläche von Tumorzellen lokalisiert ist. Bisher war TPTE lediglich als Golgi-assoziiertes Protein beschrieben worden. Aufgrund der Expression von TPTE auf der Zelloberfläche von Tumorzellen eignet sich dieses Tumorantigen erfindungsgemäß als hervorragendes Target für die Entwicklung diagnostischer und therapeutischer monoklonaler Antikörper. Aufgrund der prädizierten Membrantopologie von TPTE eignen sich für diesen Zweck erfindungsgemäß insbesondere die extrazellulär exponierten Bereiche. Dies umfasst erfindungsgemäß die Peptide FTDSKLYIPLEYRS (SEQ ID NO:81) und FDIKLLRNIPRWT (SEQ ID NO:82). Darüber hinaus konnte gezeigt werden, dass TPTE die Migration von Tumorzellen fördert.

Zellen die für mehrere Stunden in Minimalmedium ohne FCS kultiviert werden neigen zur spontanen Ausbildung von Pseudopodien und Membranprotrusionen. Sowohl in heterolog exprimierenden Zellen (TPTE-eGFP) als auch in spontan exprimierenden Zellinien (AK-Färbung) konnte eine ausgeprägte Anreicherung des Proteins an der Membran dieser Struktur nachgewiesen werden. Ferner ergab eine Ko-Färbung mit Rhodamine-Phalloidin eine eindeutige Ko-Lokalisation von TPTE mit F-Aktin in diesen Bereichen. Die durch Second Messenger wie Phosphoinisitide und Kalzium vermittelte Aktin-Polymerisierung ist ein kritischer Faktor für die Initiierung der Zellmigration in Reaktion auf extrazelluläre Gradienten von Chemotaktika. Die Signaltransduktion über diese Second Messenger wird hauptsächlich durch Rezeptor-Tyrosinkinasen (RTK) initiiert. Einer der wichtigsten Faktoren in der Regulation des Second Messenger Signalling über Phosphoinositide und der daraus folgenden Modulation des Aktin-Zytoskeletts ist die RTK c-erbB-2 (HER2), die in verschiedenen Tumortypen frequent überexprimiert wird. Diese Ergebnisse führten zur Überlegung dass TPTE als zellmembranständige Lipidphosphatase mit Substratspezifität für PIP_{3,4,5} eine regulierende Funktion auf die RTK-vermittelte Signaltransduktion hat und somit modulierende Effekt auf die Membrandynamik in Tumorzellen ausübt. Für eine detailierte Analyse wurde TPTE-eGFP in NIH3T3 Fibroblasten exprimiert die durch Transformation mit c-erbB-2 ein konstitutiv aktiviertes PI-3 Kinase Signalling und somit eine Überproduktion des Second Messengers PIP_{3,4,5} aufweisen. Time-Lapse Mikroskopie dieser Zellen nach mehrstündiger Kultivierung in Minimalmedium zeigte, dass die Lokalisation von TPTE an der Membran von Pseudopodien und Protrusionen unverzüglich zur Retraktion des entsprechenden Memranbereichs führt. Eine Erklärung für diese Beobachtung bietet der PI-3 Kinase antagonistische Effekt der TPTE der durch die Termination der RTK-vermittelten Signaltransduktion die Aktin-Polimerisation unter dem betroffene Membranbereich beendet. Diese PI-3K antagonistischen Effekte von TPTE werden durch Dephosphorylierung des Second Messengers PIP_{3,4,5} zu PIP_{4,5} vermittelt. PIP_{4,5} reguliert die kortikale Aktinpolymerisation und vermittelt die Adhäsion des Zytoskeletts an die Plasmamembran. Zum Nachweis der Kolokalisation von TPTE und PIP_{4,5} and wurden TPTE transfizierte NIH3T3/c-erbB2 Zellen mit der eGFP gekoppelten PH-Domäne von Phospholipase C-δ1 (PLC-δ1) kotransfiziert. Die PH-Domäne vermittelt die spezifische Bindung von PLC-δ1 an ihr physiologisches Substrat PIP_{4,5}, daher ist sie für die Darstellung der PIP_{4,5} Verteilung and der Plasmamembran hervorragend geeignet. Erwartungsgemäss zeigte sich in den kotransfizierten Zellen eine eindeutige Kolokalisation von TPTE und PIP_{4,5} an der Plasmamembran (Abb. 14). Dies konnte in weiteren Untersuchungen durch Kofärbung mit einem PIP_{4,5}-spezifischen Antikörper nochmals bestätigt werden. Kürzlich wurde gezeigt, dass das zu TPTE homologe PTEN das migratorische Potential von Zellen positiv reguliert. Zellmigration ist ein direktionaler Prozess für den die Wahrnehmung extrazellulärer Gradienten von Chemotaktika von essentieller Bedeutung ist. Gerichtete Zellmigration bedingt, dass die Zelle nach Detektion des Chemotaktikums in Richtung des Gradienten polarisiert. PTEN vermittelt die Polarisation der Zelle durch Ausbildung eines intrazellulären Gradienten von Second Messenger PIP_{3,4,5} in Richtung des extrazellulären Gradienten und somit eine gerichtete Aktin-Polimerisation und Migration. Zur Untersuchung, ob TPTE ähnliche promigratorische Eigenschaften besitzt wurde ein Transwell-Migrationsassay durchgeführt. TPTE transfizierte NIH3T3/c-erbB-2 Zellen und entsprechende Kontrollzellen (nicht-transfizierte NIH3T3/c-erbB-2 Zellen und mit Leer-peGFP-Vektor transfizierte NIH3T3/c-erbB-2 Zellen) wurden mit verschiedenen Mengen an chemischen Lockstoffen getestet. Zellen, die TPTE exprimierten, zeigten eine signifikant erhöhte Migrationsrate von mehr als vierfach im Vergleich zu den Kontrollzellen in Reaktion auf 10% FCS (Abb. 10). Durch den Einsatz von RNA-Interferenz (RNAi) konnte der Nachweis erbracht werden dass auch die spontane Expression von TPTE in Tumorzellinien die Chemotaxis dieser Zellen verstärkt. Hierzu wurden TPTE-positive PC-3 Prostatakarzinomzellen mit TPTE-spezifischen doppelsträngigen RNA-Oligos (sense 5'-CCCUGCCACAUGUUCAUAUdTdT-3' und antisense 5'-AUAUGAACAUGUGGCAGGGdTdT-3') elektroporiert. Nach 24 Stunden konnte mittels quantitativer Real-Time RT-PCR eine Abnahme der TPTE-Expression um 70% nachgewiesen werden (Abb. 15). Im Transwell-Migrationsassay zeigte sich eine deutliche Reduktion der Migrationsrate (70%) im Vergleich zu den entsprechenden Kontrollen (unbehandelte PC-3 Zellen und PC-3 Zellen die mit irrelevanter siRNA elektroporiert wurden) (Abb. 16). Während PTEN ein cytoplasmatisches Protein ist, das mittels PH-Domänen-rekrutierenden Second Messengern wie PIP_{3,4,5} zu der Plasmamembran gelenkt werden kann, könnte TPTE im Gegensatz dazu eine Wahrnehmung von chemotaktischen Gradienten direkt an der Membran durch eine Blockierung der Signaltransduktion von aktivierten RTKs modulieren, die häufig in Krebszellen überexprimiert sind, und dadurch eine Wahrnehmung eines räumlichen Gradienten vermitteln.

Phosphatasen sind oft bei der Zellmotilität und Migration involviert. Um die Bedeutung der TPTE bei Zellbewegung zu prüfen, wurde TPTE als Fusionsprotein mit Green-Fluorescence Protein in eine TPTE-negative Zellinie eingebracht und die Verteilung dieses Proteins in vivo mittels Real-time Mikroskopie beobachtet. Zellen bilden Membranausläufer (Protrusionen) als Vorstufe zur Migration. Nach Ausbildung einer solchen Protrusion sammelt sich TPTE an diesem Membranbereich an (Abb. 9). Bei beginnender Abflachung der Protrusion wird TPTE von der Membran zurückgezogen und internalisiert und ist offensichtlich in den Ablauf von Motilitätsereignissen der Zelle involviert. Gleichsam ergibt der Vergleich der Wandereigenschaften der mit TPTE- versus Kontrollplasmid-transfizierten Zellen in der Migrationskammer (BOYDEN Chamber), dass TPTE zu einer signifikanten Erhöhung der Migration entlang chemotaktischer Gradienten von Wachstumsfaktoren (hier z.B. PDGF=Platelet-derived Growth Factor, oder FCS=Fetales Kalbsserum) führt (Abb. 10).

Da TPTE einer Zelle offensichtlich promigratorische Eigenschaften vermittelt, wurden Brust- bzw. Lungentumoren von insgesamt 58 Patienten auf Expression von TPTE untersucht und statistisch mit derm Krankheitsverlauf der Patienten verglichen. Diejenigen Tumoren, die TPTE exprimieren, neigen signifikant häufiger zu Lymphknoten- aber auch zu Fernmetastasen als die TPTE-negativen (Abb. 11).

Diese funktionellen Daten deuten daraufhin, dass TPTE bei der Metastasierung von Tumoren eine wesentliche Rolle spielt. Entsprechend kann TPTE als Marker für prognostisch schlechteren Verlauf und Metastasenneigung gelten. Ferner können Verfahren, die erfindungsgemäß die endogene Aktivität von TPTE in Tumorzellen inhibieren, z.B. durch den Einsatz von Antisene RNA, unterschiedlicher Methoden der RNA-Interferenz (RNAi) mittels Expressionsvektoren oder Retroviren, sowie durch den Einsatz von kleinen Moleküle, zu einer reduzierten Meatstasierung führen und somit therapeutisch sehr bedeutsam sein.

## Patentansprüche

1. Pharmazeutische Zusammensetzung zur Anwendung in einem Verfahren zur Behandlung einer Krebserkrankung, die sich durch die Expression eines Tumor-assoziierten Antigens auszeichnet,
wobei die pharmazeutische Zusammensetzung einen oder mehrere Bestandteile umfasst, die aus der Gruppe ausgewählt sind, bestehend aus:
(i) einem Teil eines Tumor-assoziierten Antigens,
(ii) einer Nukleinsäure, die für einen Teil des Tumor-assoziierten Antigens kodiert, und
(iii) einer Wirtszelle, die einen Teil des Tumor-assoziierten Antigens exprimiert,
wobei der Teil ein Octamer, Nonamer oder Decamer ist, das die Eigenschaft aufweist, einen Komplex mit HLA einzugehen und eine Immunreaktion zu erzeugen, und wobei der Teil für das Tumor-assoziierte Antigen spezifisch ist und wobei der Teil eine Aminosäuresequenz aufweist, die aus der Gruppe bestehend aus SEQ ID NOs: 383-821 ausgewählt ist,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 19-21 und 54-57 ausgewählt ist,
und
(b) einer Nukleinsäure, die mindestens 90% Sequenzidentität zu der Nukleinsäure unter (a) aufweist,
wobei die Krebserkrankung ein Melanom, ein Mammakarzinom, die Metastase eines Mammakarzinoms, ein Nierenzellkarzinom, ein Ovarialkarzinom, oder ein Zervixkarzinom ist.

2. Pharmazeutische Zusammensetzung zur Anwendung gemäß Anspruch 1, wobei der Teil des Tumor-assoziierten Antigens in einem Komplex mit einem HLA-Molekül vorliegt, vorzugsweise HLA-A*0201, HLA-A*2402, HLA-A*01, HLA-A*03 oder HLA-B*0702.

3. Pharmazeutische Zusammensetzung zur Anwendung gemäß einem der Ansprüche 1 oder 2, wobei die Nukleinsäure unter (ii) in einem Expressionsvektor vorliegt.

4. Pharmazeutische Zusammensetzung zur Anwendung gemäß einem der Ansprüche 1 bis 3, wobei die Nukleinsäure unter (ii) funktionell mit einem Promotor verbunden ist.

5. Pharmazeutische Zusammensetzung zur Anwendung gemäß Anspruch 1, wobei die Wirtszelle den Teil des Tumor-assoziierten Antigens sekretiert.

6. Pharmazeutische Zusammensetzung zur Anwendung gemäß Anspruch 1, wobei die Wirtszelle zusätzlich ein HLA-Molekül exprimiert, das an den Teil des Tumor-assoziierten Antigens bindet.

7. Pharmazeutische Zusammensetzung zur Anwendung gemäß Anspruch 6, wobei die Wirtszelle das HLA-Molekül und/oder das Tumor-assoziierte Antigen oder den Teil davon rekombinant exprimiert.

8. Pharmazeutische Zusammensetzung zur Anwendung gemäß Anspruch 7, wobei die Wirtszelle das HLA-Molekül endogen exprimiert.

9. Pharmazeutische Zusammensetzung zur Anwendung gemäß einem der Ansprüche 1 oder 5 bis 8, wobei die Wirtszelle eine Antigen-präsentierende Zelle ist.

10. Pharmazeutische Zusammensetzung zur Anwendung gemäß Anspruch 9, wobei die Antigen-präsentierende Zelle eine dendritische Zelle oder ein Makrophage ist.

11. Pharmazeutische Zusammensetzung zur Anwendung gemäß einem der Ansprüche 1 oder 5 bis 10, wobei die Wirtszelle nicht-proliferativ ist.

12. Pharmazeutische Zusammensetzung zur Verwendung gemäß einem der Ansprüche 1 bis 11, wobei das Verfahren die Reduktion oder Hemmung der Migration oder Metastasierung von Tumorzellen, die sich durch die Expression eines Tumor-assoziierten Antigens auszeichnen, umfasst.

## Claims

1. A pharmaceutical composition for use in a method of treating a cancer disease **characterized by** the expression of a tumor-associated antigen,
wherein the pharmaceutical composition comprises one or more components selected from the group consisting of:
(i) a part of a tumor-associated antigen,
(ii) a nucleic acid which codes for a part of the tumor-associated antigen, and
(iii) a host cell which expresses a part of the tumor-associated antigen,
wherein the part is an octamer, nonamer or decamer which has the property to form a complex with HLA and generate an immune reaction, and wherein the part is specific for the tumor-associated antigen and wherein the part has an amino acid sequence selected from the group consisting of SEQ ID NOs: 383-821,
wherein the tumor-associated antigen has a sequence which is encoded by a nucleic acid selected from the group consisting of:
(a) a nucleic acid which comprises a nucleic acid sequence selected from the group consisting of SEQ ID NOs: 19-21 and 54-57,
and
(b) a nucleic acid which has at least 90% sequence identity to the nucleic acid of (a),
wherein the cancer disease is a melanoma, a mamma carcinoma, the metastasis of a mamma carcinoma, a renal cell carcinoma, an ovarian carcinoma, or a cervical carcinoma.

2. The pharmaceutical composition for use according to claim 1, wherein the part of the tumor-associated antigen is present in a complex with an HLA molecule, preferably HLA-A*0201, HLA-A*2402, HLA-A*01, HLA-A*03 or HLA-B*0702.

3. The pharmaceutical composition for use according to any one of claims 1 or 2, wherein the nucleic acid of (ii) is present in an expression vector.

4. The pharmaceutical composition for use according to any one of claims 1 to 3, wherein the nucleic acid of (ii) is functionally linked to a promoter.

5. The pharmaceutical composition for use according to claim 1, wherein the host cell secretes the part of the tumor-associated antigen.

6. The pharmaceutical composition for use according to claim 1, wherein the host cell additionally expresses an HLA molecule which binds to the part of the tumor-associated antigen.

7. The pharmaceutical composition for use according to claim 6, wherein the host cell recombinantly expresses the HLA molecule and/or the tumor-associated antigen or the part thereof.

8. The pharmaceutical composition for use according to claim 7, wherein the host cell endogenously expresses the HLA molecule.

9. The pharmaceutical composition for use according to any one of claims 1 or 5 to 8, wherein the host cell is an antigen-presenting cell.

10. The pharmaceutical composition for use according to claim 9, wherein the antigen-presenting cell is a dendritic cell or a macrophage.

11. The pharmaceutical composition for use according to any one of claims 1 or 5 to 10, wherein the host cell is nonproliferative.

12. The pharmaceutical composition for use according to any one of claims 1 to 11, wherein the method comprises the reduction or inhibition of the migration or metastasis of tumor cells **characterized by** the expression of a tumor-associated antigen.

## Revendications

1. Composition pharmaceutique pour une utilisation dans un procédé de traitement d'une maladie cancéreuse, qui se **caractérise par** l'expression d'un antigène associé à une tumeur,
la composition pharmaceutique comprenant un ou plusieurs constituants, qui sont choisis dans le groupe consistant en :
(i) une partie d'un antigène associé à une tumeur,
(ii) un acide nucléique qui code pour une partie de l'antigène associé à une tumeur, et
(iii) une cellule hôte, qui exprime une partie de l'antigène associé à une tumeur,
dans laquelle la partie est un octamère, un nonamère ou un décamère, qui présente la propriété de former un complexe avec le HLA et de produire une réaction immunitaire, la partie étant spécifique de l'antigène associé à une tumeur, et la partie présentant une séquence d'acides aminés qui est choisie dans le groupe consistant en SEQ ID NO: 383-821,
l'antigène associé à une tumeur présentant une séquence qui est codée par un acide nucléique qui est choisi dans le groupe consistant en :
(a) un acide nucléique qui comprend une séquence d'acide nucléique qui est choisie dans le groupe consistant en SEQ ID NO: 19-21 et 54-57, et
(b) un acide nucléique qui présente une identité de séquence d'au moins 90 % avec l'acide nucléique de (a),
la maladie cancéreuse étant un mélanome, un carcinome mammaire, la métastase d'un carcinome mammaire, un carcinome à cellules rénales, un carcinome ovarien ou un carcinome du col de l'utérus.

2. Composition pharmaceutique pour l'utilisation selon la revendication 1, dans laquelle la partie de l'antigène associé à une tumeur est présente dans un complexe avec une molécule de HLA, de préférence HLA-A*0201, HLA-A*2402, HLA-A*01, HLA-A*03 ou HLA-B*0702.

3. Composition pharmaceutique pour l'utilisation selon l'une des revendications 1 ou 2, dans laquelle l'acide nucléique de (ii) est présent dans un vecteur d'expression.

4. Composition pharmaceutique pour l'utilisation selon l'une des revendications 1 à 3, dans laquelle l'acide nucléique de (ii) est fonctionnellement lié à un promoteur.

5. Composition pharmaceutique pour l'utilisation selon la revendication 1, dans laquelle la cellule hôte sécrète la partie de l'antigène associé à une tumeur.

6. Composition pharmaceutique pour l'utilisation selon la revendication 1, dans laquelle la cellule hôte exprime en outre une molécule de HLA qui se lie à la partie de l'antigène associé à une tumeur.

7. Composition pharmaceutique pour l'utilisation selon la revendication 6, dans laquelle la cellule hôte exprime d'une manière recombinante la molécule de HLA et/ou l'antigène associé à une tumeur ou la partie de celui-ci.

8. Composition pharmaceutique pour l'utilisation selon la revendication 7, dans laquelle la cellule hôte exprime la molécule de HLA d'une manière endogène.

9. Composition pharmaceutique pour l'utilisation selon l'une des revendications 1 ou 5 à 8, dans laquelle la cellule hôte est une cellule présentant l'antigène.

10. Composition pharmaceutique pour l'utilisation selon la revendication 9, dans laquelle la cellule présentant l'antigène est une cellule dendritique ou un macrophage.

11. Composition pharmaceutique pour l'utilisation selon l'une des revendications 1 ou 5 à 10, dans laquelle la cellule hôte est non proliférative.

12. Composition pharmaceutique pour l'utilisation selon l'une des revendications 1 à 11, le procédé comprenant la réduction ou l'inhibition de la migration ou de la métastatisation de cellules tumorales qui se caractérisent par l'expression d'un antigène associé à une tumeur.


### SEQUENCE LISTING

<110> BioNTech AG
<120> Differentiell in Tumoren exprimierte Genprodukte und deren Verwendung
<130> 342-14 PCTEPT6
<150> DE 103 41 812.1
   <151> 2003-09-10
<160> 464
<170> PatentIn version 3.1
<210> 19
   <211> 2168
   <212> DNA
   <213> Homo sapiens
<400> 19
   gaatccgcgg ggagggcaca acagctgcta cctgaacagt ttctgaccca acagttaccc     60
   agcgccggac tcgctgcgcc ccggcggctc tagggacccc cggcgcctac acttagctcc    120
   gcgcccgaga gaatgttgga ccgacgacac aagacctcag acttgtgtta ttctagcagc    180
   tgaacacacc ccaggctctt ctgaccggca gtggctctgg aagcagtctg gtgtatagag    240
   ttatggattc actaccagat tctactgtat gctcttgaca actatgacca caatggtcca    300
   cccacaaatg aattatcagg agtgaaccca gaggcacgta tgaatgaaag tcctgatccg    360
   actgacctgg cgggagtcat cattgagctc ggccccaatg acagtccaca gacaagtgaa    420
   tttaaaggag caaccgagga ggcacctgcg aaagaaagcc cacacacaag tgaatttaaa    480
   ggagcagccc gggtgtcacc tatcagtgaa agtgtgttag cacgactttc caagtttgaa    540
   gttgaagatg ctgaaaatgt tgcttcatat gacagcaaga ttaagaaaat tgtgcattca    600
   attgtatcat cctttgcatt tggactattt ggagttttcc tggtcttact ggatgtcact    660
   ctcatccttg ccgacctaat tttcactgac agcaaacttt atattccttt ggagtatcgt     720
   tctatttctc tagctattgc cttatttttt ctcatggatg ttcttcttcg agtatttgta     780
   gaaaggagac agcagtattt ttctgactta tttaacattt tagatactgc cattattgtg     840
   attcttctgc tggttgatgt cgtttacatt ttttttgaca ttaagttgct taggaatatt     900
   cccagatgga cacatttact tcgacttcta cgacttatta ttctgttaag aatttttcat     960
   ctgtttcatc aaaaaagaca acttgaaaag ctgataagaa ggcgggtttc agaaaacaaa    1020
   aggcgataca caagggatgg atttgaccta gacctcactt acgttacaga acgtattatt    1080
   gctatgtcat ttccatcttc tggaaggcag tctttctata gaaatccaat caaggaagtt    1140
   gtgcggtttc tagataagaa acaccgaaac cactatcgag tctacaatct atgcagtgaa    1200
   agagcttacg atcctaagca cttccataat agggtcgtta gaatcatgat tgatgatcat    1260
   aatgtcccca ctctacatca gatggtggtt ttcaccaagg aagtaaatga gtggatggct    1320
   caagatcttg aaaacatcgt agcgattcac tgtaaaggag gcacagatag aacaggaact    1380
   atggtttgtg ccttccttat tgcctctgaa atatgttcaa ctgcaaagga aagcctgtat    1440
   tattttggag aaaggcgaac agataaaacc cacagcgaaa aatttcaggg agtagaaact    1500
   ccttctcaga agagatatgt tgcatatttt gcacaagtga aacatctcta caactggaat    1560
   ctccctccaa gacggatact ctttataaaa cacttcatta tttattcgat tcctcgttat    1620
   gtacgtgatc taaaaatcca aatagaaatg gagaaaaagg ttgtcttttc cactatttca    1680
   ttaggaaaat gttcggtact tgataacatt acaacagaca aaatattaat tgatgtattc    1740
   gacggtccac ctctgtatga tgatgtgaaa gtgcagtttt tctattcgaa tcttcctaca    1800
   tactatgaca attgctcatt ttacttctgg ttgcacacat cttttattga aaataacagg    1860
   ctttatctac caaaaaatga attggataat ctacataaac aaaaagcacg gagaatttat    1920
   ccatcagatt ttgccgtgga gatacttttt ggcgagaaaa tgacttccag tgatgttgta    1980
   gctggatccg attaagtata gctccccctt ccccttctgg gaaagaatta tgttctttcc    2040
   aaccctgcca catgttcata tatcctaaat ctatcctaaa tgttcccttg aagtatttat    2100
   ttatgtttat atatgtttat acatgttctt caataaatct attacatata tataaaaaaa    2160
   aaaaaaaa                                                             2168
<210> 20
   <211> 2114
   <212> DNA
   <213> Homo sapiens
<400> 20
   gaatccgcgg ggagggcaca acagctgcta cctgaacagt ttctgaccca acagttaccc     60
   agcgccggac tcgctgcgcc ccggcggctc tagggacccc cggcgcctac acttagctcc    120
   gcgcccgaga gaatgttgga ccgacgacac aagacctcag acttgtgtta ttctagcagc    180
   tgaacacacc ccaggctctt ctgaccggca gtggctctgg aagcagtctg gtgtatagag    240
   ttatggattc actaccagat tctactgtat gctcttgaca actatgacca caatggtcca    300
   cccacaaatg aattatcagg agtgaaccca gaggcacgta tgaatgaaag tcctgatccg    360
   actgacctgg cgggagtcat cattgagctc ggccccaatg acagtccaca gacaagtgaa    420
   tttaaaggag caaccgagga ggcacctgcg aaagaaagtg tgttagcacg actttccaag    480
   tttgaagttg aagatgctga aaatgttgct tcatatgaca gcaagattaa gaaaattgtg    540
   cattcaattg tatcatcctt tgcatttgga ctatttggag ttttcctggt cttactggat    600
   gtcactctca tccttgccga cctaattttc actgacagca aactttatat tcctttggag    660
   tatcgttcta tttctctagc tattgcctta ttttttctca tggatgttct tcttcgagta     720
   tttgtagaaa ggagacagca gtatttttct gacttattta acattttaga tactgccatt    780
   attgtgattc ttctgctggt tgatgtcgtt tacatttttt ttgacattaa gttgcttagg    840
   aatattccca gatggacaca tttacttcga cttctacgac ttattattct gttaagaatt    900
   tttcatctgt ttcatcaaaa aagacaactt gaaaagctga taagaaggcg ggtttcagaa    960
   aacaaaaggc gatacacaag ggatggattt gacctagacc tcacttacgt tacagaacgt   1020
   attattgcta tgtcatttcc atcttctgga aggcagtctt tctatagaaa tccaatcaag   1080
   gaagttgtgc ggtttctaga taagaaacac cgaaaccact atcgagtcta caatctatgc   1140
   agtgaaagag cttacgatcc taagcacttc cataataggg tcgttagaat catgattgat   1200
   gatcataatg tccccactct acatcagatg gtggttttca ccaaggaagt aaatgagtgg   1260
   atggctcaag atcttgaaaa catcgtagcg attcactgta aaggaggcac agatagaaca   1320
   ggaactatgg tttgtgcctt ccttattgcc tctgaaatat gttcaactgc aaaggaaagc   1380
   ctgtattatt ttggagaaag gcgaacagat aaaacccaca gcgaaaaatt tcagggagta   1440
   gaaactcctt ctcagaagag atatgttgca tattttgcac aagtgaaaca tctctacaac   1500
   tggaatctcc ctccaagacg gatactcttt ataaaacact tcattattta ttcgattcct   1560
   cgttatgtac gtgatctaaa aatccaaata gaaatggaga aaaaggttgt cttttccact   1620
   atttcattag gaaaatgttc ggtacttgat aacattacaa cagacaaaat attaattgat   1680
   gtattcgacg gtccacctct gtatgatgat gtgaaagtgc agtttttcta ttcgaatctt 1740
   cctacatact atgacaattg ctcattttac ttctggttgc acacatcttt tattgaaaat 1800
   aacaggcttt atctaccaaa aaatgaattg gataatctac ataaacaaaa agcacggaga 1860
   atttatccat cagattttgc cgtggagata ctttttggcg agaaaatgac ttccagtgat 1920
   gttgtagctg gatccgatta agtatagctc cccctccccc ttctgggaaa gaattatgtt 1980
   ctttccaacc ctgccacatg ttcatatatc ctaaatctat cctaaatgtt cccttgaagt 2040
   atttatttat gtttatatat gtttatacat gttcttcaat aaatctatta catatatata 2100
   aaaaaaaaaa aaaa 2114
<210> 21
   <211> 2222
   <212> DNA
   <213> Homo sapiens
<400> 21
   gaatccgcgg ggagggcaca acagctgcta cctgaacagt ttctgaccca acagttaccc 60
   agcgccggac tcgctgcgcc ccggcggctc tagggacccc cggcgcctac acttagctcc 120
   gcgcccgaga gaatgttgga ccgacgacac aagacctcag acttgtgtta ttctagcagc 180
   tgaacacacc ccaggctctt ctgaccggca gtggctctgg aagcagtctg gtgtatagag 240
   ttatggattc actaccagat tctactgtat gctcttgaca actatgacca caatggtcca 300
   cccacaaatg aattatcagg agtgaaccca gaggcacgta tgaatgaaag tcctgatccg 360
   actgacctgg cgggagtcat cattgagctc ggccccaatg acagtccaca gacaagtgaa 420
   tttaaaggag caaccgagga ggcacctgcg aaagaaagcc cacacacaag tgaatttaaa 480
   ggagcagccc gggtgtcacc tatcagtgaa agtgtgttag cacgactttc caagtttgaa 540
   gttgaagatg ctgaaaatgt tgcttcatat gacagcaaga ttaagaaaat tgtgcattca 600
   attgtatcat cctttgcatt tggactattt ggagttttcc tggtcttact ggatgtcact 660
   ctcatccttg ccgacctaat tttcactgac agcaaacttt atattccttt ggagtatcgt 720
   tctatttctc tagctattgc cttatttttt ctcatggatg ttcttcttcg agtatttgta 780
   gaaaggagac agcagtattt ttctgactta tttaacattt tagatactgc cattattgtg 840
   attcttctgc tggttgatgt cgtttacatt ttttttgaca ttaagttgct taggaatatt 900
   cccagatgga cacatttact tcgacttcta cgacttatta ttctgttaag aatttttcat 960
   ctgtttcatc aaaaaagaca acttgaaaag ctgataagaa ggcgggtttc agaaaacaaa   1020
   aggcgataca caagggatgg atttgaccta gacctcactt acgttacaga acgtattatt   1080
   gctatgtcat ttccatcttc tggaaggcag tctttctata gaaatccaat caaggaagtt   1140
   gtgcggtttc tagataagaa acaccgaaac cactatcgag tctacaatct atgcagtatg   1200
   tacattactc tatattgtgc tactgtagat agaaaacaga ttactgcacg tgaaagagct   1260
   tacgatccta agcacttcca taatagggtc gttagaatca tgattgatga tcataatgtc   1320
   cccactctac atcagatggt ggttttcacc aaggaagtaa atgagtggat ggctcaagat   1380
   cttgaaaaca tcgtagcgat tcactgtaaa ggaggcacag atagaacagg aactatggtt   1440
   tgtgccttcc ttattgcctc tgaaatatgt tcaactgcaa aggaaagcct gtattatttt   1500
   ggagaaaggc gaacagataa aacccacagc gaaaaatttc agggagtaga aactccttct   1560
   cagaagagat atgttgcata ttttgcacaa gtgaaacatc tctacaactg gaatctccct   1620
   ccaagacgga tactctttat aaaacacttc attatttatt cgattcctcg ttatgtacgt   1680
   gatctaaaaa tccaaataga aatggagaaa aaggttgtct tttccactat ttcattagga   1740
   aaatgttcgg tacttgataa cattacaaca gacaaaatat taattgatgt attcgacggt   1800
   ccacctctgt atgatgatgt gaaagtgcag tttttctatt cgaatcttcc tacatactat   1860
   gacaattgct cattttactt ctggttgcac acatctttta ttgaaaataa caggctttat   1920
   ctaccaaaaa atgaattgga taatctacat aaacaaaaag cacggagaat ttatccatca   1980
   gattttgccg tggagatact ttttggcgag aaaatgactt ccagtgatgt tgtagctgga   2040
   tccgattaag tatagctccc ccttcccctt ctgggaaaga attatgttct ttccaaccct   2100
   gccacatgtt catatatcct aaatctatcc taaatgttcc cttgaagtat ttatttatgt   2160
   ttatatatgt ttatacatgt tcttcaataa atctattaca tatatataaa aaaaaaaaaa   2220
   aa                                                                  2222
<210> 22
   <211> 551
   <212> PRT
   <213> Homo sapiens
<400> 22
   Met Asn Glu Ser Pro Asp Pro Thr Asp Leu Ala Gly Val Ile Ile Glu
   1               5                   10                  15
   Leu Gly Pro Asn Asp Ser Pro Gln Thr Ser Glu Phe Lys Gly Ala Thr
               20                  25                  30
   Glu Glu Ala Pro Ala Lys Glu Ser Pro His Thr Ser Glu Phe Lys Gly
           35                  40                  45
   Ala Ala Arg Val Ser Pro Ile Ser Glu Ser Val Leu Ala Arg Leu Ser
       50                  55                  60
   Lys Phe Glu Val Glu Asp Ala Glu Asn Val Ala Ser Tyr Asp Ser Lys
   65                  70                  75                  80
   Ile Lys Lys Ile Val His Ser Ile Val Ser Ser Phe Ala Phe Gly Leu
                   85                  90                  95
   Phe Gly Val Phe Leu Val Leu Leu Asp Val Thr Leu Ile Leu Ala Asp
               100                 105                 110
   Leu Ile Phe Thr Asp Ser Lys Leu Tyr Ile Pro Leu Glu Tyr Arg Ser
           115                 120                 125
   Ile Ser Leu Ala Ile Ala Leu Phe Phe Leu Met Asp Val Leu Leu Arg
       130                 135                 140
   Val Phe Val Glu Arg Arg Gln Gln Tyr Phe Ser Asp Leu Phe Asn Ile
   145                 150                 155                 160
   Leu Asp Thr Ala Ile Ile Val Ile Leu Leu Leu Val Asp Val Val Tyr
                   165                 170                 175
   Ile Phe Phe Asp Ile Lys Leu Leu Arg Asn Ile Pro Arg Trp Thr His
               180                 185                 190
   Leu Leu Arg Leu Leu Arg Leu Ile Ile Leu Leu Arg Ile Phe His Leu
           195                 200                 205
   Phe His Gln Lys Arg Gln Leu Glu Lys Leu Ile Arg Arg Arg Val Ser
       210                 215                 220
   Glu Asn Lys Arg Arg Tyr Thr Arg Asp Gly Phe Asp Leu Asp Leu Thr
   225                 230                 235                 240
   Tyr Val Thr Glu Arg Ile Ile Ala Met Ser Phe Pro Ser Ser Gly Arg
                   245                 250                 255
   Gln Ser Phe Tyr Arg Asn Pro Ile Lys Glu Val Val Arg Phe Leu Asp
               260                 265                 270
   Lys Lys His Arg Asn His Tyr Arg Val Tyr Asn Leu Cys Ser Glu Arg
           275                 280                 285
   Ala Tyr Asp Pro Lys His Phe His Asn Arg Val Val Arg Ile Met Ile
       290                 295                 300
   Asp Asp His Asn Val Pro Thr Leu His Gln Met Val Val Phe Thr Lys
   305                 310                 315                 320
   Glu Val Asn Glu Trp Met Ala Gln Asp Leu Glu Asn Ile Val Ala Ile
                   325                 330                 335
   His Cys Lys Gly Gly Thr Asp Arg Thr Gly Thr Met Val Cys Ala Phe
               340                 345                 350
   Leu Ile Ala Ser Glu Ile Cys Ser Thr Ala Lys Glu Ser Leu Tyr Tyr
           355                 360                 365
   Phe Gly Glu Arg Arg Thr Asp Lys Thr His Ser Glu Lys Phe Gln Gly
       370                 375                 380
   Val Glu Thr Pro Ser Gln Lys Arg Tyr Val Ala Tyr Phe Ala Gln Val
   385                 390                 395                 400
   Lys His Leu Tyr Asn Trp Asn Leu Pro Pro Arg Arg Ile Leu Phe Ile
                   405                 410                 415
   Lys His Phe Ile Ile Tyr Ser Ile Pro Arg Tyr Val Arg Asp Leu Lys
               420                 425                 430
   Ile Gln Ile Glu Met Glu Lys Lys Val Val Phe Ser Thr Ile Ser Leu
           435                 440                 445
   Gly Lys Cys Ser Val Leu Asp Asn Ile Thr Thr Asp Lys Ile Leu Ile
       450                 455                 460
   Asp Val Phe Asp Gly Pro Pro Leu Tyr Asp Asp Val Lys Val Gln Phe
   465                 470                 475                 480
   Phe Tyr Ser Asn Leu Pro Thr Tyr Tyr Asp Asn Cys Ser Phe Tyr Phe
                   485                 490                 495
   Trp Leu His Thr Ser Phe Ile Glu Asn Asn Arg Leu Tyr Leu Pro Lys
               500                 505                 510
   Asn Glu Leu Asp Asn Leu His Lys Gln Lys Ala Arg Arg Ile Tyr Pro
           515                 520                 525
   Ser Asp Phe Ala Val Glu Ile Leu Phe Gly Glu Lys Met Thr Ser Ser
       530                 535                 540
   Asp Val Val Ala Gly Ser Asp
   545                 550
<210> 23
   <211> 533
   <212> PRT
   <213> Homo sapiens
<400> 23
<210> 24
   <211> 569
   <212> PRT
   <213> Homo sapiens
<400> 24
   Met Asn Glu Ser Pro Asp Pro Thr Asp Leu Ala Gly Val Ile Ile Glu
   1 5 10 15
   Leu Gly Pro Asn Asp Ser Pro Gln Thr Ser Glu Phe Lys Gly Ala Thr
               20                  25                  30
   Glu Glu Ala Pro Ala Lys Glu Ser Pro His Thr Ser Glu Phe Lys Gly
           35                  40                  45
   Ala Ala Arg Val Ser Pro Ile Ser Glu Ser Val Leu Ala Arg Leu Ser
       50                  55                  60
   Lys Phe Glu Val Glu Asp Ala Glu Asn Val Ala Ser Tyr Asp Ser Lys
   65                  70                  75                  80
   Ile Lys Lys Ile Val His Ser Ile Val Ser Ser Phe Ala Phe Gly Leu
                   85                  90                  95
   Phe Gly Val Phe Leu Val Leu Leu Asp Val Thr Leu Ile Leu Ala Asp
               100                 105                 110
   Leu Ile Phe Thr Asp Ser Lys Leu Tyr Ile Pro Leu Glu Tyr Arg Ser
           115                 120                 125
   Ile Ser Leu Ala Ile Ala Leu Phe Phe Leu Met Asp Val Leu Leu Arg
       130                 135                 140
   Val Phe Val Glu Arg Arg Gln Gln Tyr Phe Ser Asp Leu Phe Asn Ile
   145                 150                 155                 160
   Leu Asp Thr Ala Ile Ile Val Ile Leu Leu Leu Val Asp Val Val Tyr
                   165                 170                 175
   Ile Phe Phe Asp Ile Lys Leu Leu Arg Asn Ile Pro Arg Trp Thr His
               180                 185                 190
   Leu Leu Arg Leu Leu Arg Leu Ile Ile Leu Leu Arg Ile Phe His Leu
           195                 200                 205
   Phe His Gln Lys Arg Gln Leu Glu Lys Leu Ile Arg Arg Arg Val Ser
       210                 215                 220
   Glu Asn Lys Arg Arg Tyr Thr Arg Asp Gly Phe Asp Leu Asp Leu Thr
   225                 230                 235                 240
   Tyr Val Thr Glu Arg Ile Ile Ala Met Ser Phe Pro Ser Ser Gly Arg
                   245                 250                 255
   Gln Ser Phe Tyr Arg Asn Pro Ile Lys Glu Val Val Arg Phe Leu Asp
               260                 265                 270
   Lys Lys His Arg Asn His Tyr Arg Val Tyr Asn Leu Cys Ser Met Tyr
           275                 280                 285
   Ile Thr Leu Tyr Cys Ala Thr Val Asp Arg Lys Gln Ile Thr Ala Arg
       290                 295                 300
   Glu Arg Ala Tyr Asp Pro Lys His Phe His Asn Arg Val Val Arg Ile
   305                 310                 315                 320
   Met Ile Asp Asp His Asn Val Pro Thr Leu His Gln Met Val Val Phe
                   325                 330                 335
   Thr Lys Glu Val Asn Glu Trp Met Ala Gln Asp Leu Glu Asn Ile Val
               340                 345                 350
   Ala Ile His Cys Lys Gly Gly Thr Asp Arg Thr Gly Thr Met Val Cys
           355                 360                 365
   Ala Phe Leu Ile Ala Ser Glu Ile Cys Ser Thr Ala Lys Glu Ser Leu
       370                 375                 380
   Tyr Tyr Phe Gly Glu Arg Arg Thr Asp Lys Thr His Ser Glu Lys Phe
   385                 390                 395                 400
   Gln Gly Val Glu Thr Pro Ser Gln Lys Arg Tyr Val Ala Tyr Phe Ala
                   405                 410                 415
   Gln Val Lys His Leu Tyr Asn Trp Asn Leu Pro Pro Arg Arg Ile Leu
               420                 425                 430
   Phe Ile Lys His Phe Ile Ile Tyr Ser Ile Pro Arg Tyr Val Arg Asp
           435                 440                 445
   Leu Lys Ile Gln Ile Glu Met Glu Lys Lys Val Val Phe Ser Thr Ile
       450                 455                 460
   Ser Leu Gly Lys Cys Ser Val Leu Asp Asn Ile Thr Thr Asp Lys Ile
   465                 470                 475                 480
   Leu Ile Asp Val Phe Asp Gly Pro Pro Leu Tyr Asp Asp Val Lys Val
                   485                 490                 495
   Gln Phe Phe Tyr Ser Asn Leu Pro Thr Tyr Tyr Asp Asn Cys Ser Phe
               500                 505                 510
   Tyr Phe Trp Leu His Thr Ser Phe Ile Glu Asn Asn Arg Leu Tyr Leu
           515                 520                 525
   Pro Lys Asn Glu Leu Asp Asn Leu His Lys Gln Lys Ala Arg Arg Ile
       530                 535                 540
   Tyr Pro Ser Asp Phe Ala Val Glu Ile Leu Phe Gly Glu Lys Met Thr
   545                 550                 555                 560
   Ser Ser Asp Val Val Ala Gly Ser Asp
                   565
<210> 27
   <211> 21
   <212> DNA
   <213> Künstliche Sequenz
<220>
   <223> Beschreibung der künstlichen Sequenz: Oligonukleotid
<400> 27
   tggatgtcac tctcatcctt g 21
<210> 28
   <211> 21
   <212> DNA
   <213> Künstliche Sequenz
<220>
   <223> Beschreibung der künstlichen Sequenz: Oligonukleotid
<400> 28
   ccatagttcc tgttctatct g 21
<210> 52
   <211> 23
   <212> DNA
   <213> Künstliche Sequenz
<220>
   <223> Beschreibung der künstlichen Sequenz: Oligonukleotid
<400> 52
   cgtgagcgct tcgagatgtt ccg 23
<210> 53
   <211> 23
   <212> DNA
   <213> Künstliche Sequenz
<220>
   <223> Beschreibung der künstlichen Sequenz: Oligonukleotid
<400> 53
   cctaaccagc tgcccaactg tag 23
<210> 54
   <211> 1550
   <212> DNA
   <213> Homo sapiens
<400> 54
   atgaatgaaa gtcctgatcc gactgacctg gcgggagtca tcattgagct cggccccaat 60
   gacagtccac agacaagtga atttaaagga gcaaccgagg aggcacctgc gaaagaaagc 120
   ccacacacaa gtgaatttaa aggagcagcc cgggtgtcac ctatcagtga aagtgtgtta 180
   gcacgacttt ccaagtttga agttgaagat gctgaaaatg ttgcttcata tgacagcaag 240
   attaagaaaa ttgtgcattc aattgtatca tcctttgcat ttggactatt tggagttttc 300
   ctggtcttac tggatgtcac tctcatcctt gccgacctaa ttttcactga cagcaaactt 360
   tatattcctt tggagtatcg ttctatttct ctagctattg ccttattttt tctcatggat 420
   gttcttcttc gagtatttgt agaaaggaga cagcagtatt tttctgactt atttaacatt 480
   ttagatactg ccattattgt gattcttctg ctggttgatg tcgtttacat tttttttgac 540
   attaagttgc ttaggaatat tcccagatgg acacatttac ttcgacttct acgacttatt 600
   attctgttaa gaatttttca tctgtttcat caaaaaagac aacttgaaaa gctgataaga 660
   aggcgggttt cagaaaacaa aaggcgatac acaagggatg gatttgacct agacctcact 720
   tacgttacag aacgtattat tgctatgtca tttccatctt ctggaaggca gtctttctat 780
   agaaatccaa tcaaggaagt tgtgcggttt ctagataaga aacaccgaaa ccactatcga 840
   gtctacaatc tatgcagtga aagagcttac gatcctaagc acttccataa tagggtcgtt 900
   agaatcatga ttgatgatca taatgtcccc actctacatc agatggtggt tttcaccaag 960
   gaagtaaatg agtggatggc tcaagatctt gaaaacatcg tagcgattca ctgtaaagga 1020
   ggcacagata gaacaggaac tatggtttgt gccttcctta ttgcctctga aatatgttca 1080
   actgcaaagg aaagcctgta ttattttgga gaaaggcgaa cagataaaac ccacagcgaa 1140
   aaatttcagg gagtagaaac tccttctcag gttatgtacg tgatctaaaa atccaaatag 1200
   aaatggagaa aaaggttgtc ttttccacta tttcattagg aaaatgttcg gtacttgata 1260
   acattacaac agacaaaata ttaattgatg tattcgacgg tccacctctg tatgatgatg 1320
   tgaaagtgca gtttttctat tcgaatcttc ctacatacta tgacaattgc tcattttact 1380
   tctggttgca cacatctttt attgaaaata acaggcttta tctaccaaaa aatgaattgg 1440
   ataatctaca taaacaaaaa gcacggagaa tttatccatc agattttgcc gtggagatac 1500
   tttttggcga gaaaatgact tccagtgatg ttgtagctgg atccgattaa 1550
<210> 55
   <211> 1407
   <212> DNA
   <213> Homo sapiens
<400> 55
   atgaatgaaa gtcctgatcc gactgacctg gcgggagtca tcattgagct cggccccaat 60
   gacagtccac agacaagtga atttaaagga gcaaccgagg aggcacctgc gaaagaaagc 120
   ccacacacaa gtgaatttaa aggagcagcc cgggtgtcac ctatcagtga aagtgtgtta 180
   gcacgacttt ccaagtttga agttgaagat gctgaaaatg ttgcttcata tgacagcaag 240
   attaagaaaa ttgtgcattc aattgtatca tcctttgcat ttggactatt tggagttttc 300
   ctggtcttac tggatgtcac tctcatcctt gccgacctaa ttttcactga cagcaaactt 360
   tatattcctt tggagtatcg ttctatttct ctagctattg ccttattttt tctcatggat 420
   gttcttcttc gagtatttgt agaaaggaga cagcagtatt tttctgactt atttaacatt 480
   ttagatactg ccattattgt gattcttctg ctggttgatg tcgtttacat tttttttgac 540
   attaagttgc ttaggaatat tcccagatgg acacatttac ttcgacttct acgacttatt 600
   attctgttaa gaatttttca tctgtttcat caaaaaagac aacttgaaaa gctgataaga 660
   aggcgggttt cagaaaacaa aaggcgatac acaagggatg gatttgacct agacctcact 720
   tacgttacag aacgtattat tgctatgtca tttccatctt ctggaaggca gtctttctat 780
   agaaatccaa tcaaggaagt tgtgcggttt ctagataaga aacaccgaaa ccactatcga 840
   gtctacaatc tatgcagtga aagagcttac gatcctaagc acttccataa tagggtcgtt 900
   agaatcatga ttgatgatca taatgtcccc actctacatc agatggtggt tttcaccaag 960
   gaagtaaatg agtggatggc tcaagatctt gaaaacatcg tagcgattca ctgtaaagga 1020
   ggcacaggtt atgtacgtga tctaaaaatc caaatagaaa tggagaaaaa ggttgtcttt 1080
   tccactattt cattaggaaa atgttcggta cttgataaca ttacaacaga caaaatatta 1140
   attgatgtat tcgacggtcc acctctgtat gatgatgtga aagtgcagtt tttctattcg 1200
   aatcttccta catactatga caattgctca ttttacttct ggttgcacac atcttttatt 1260
   gaaaataaca ggctttatct accaaaaaat gaattggata atctacataa acaaaaagca 1320
   cggagaattt atccatcaga ttttgccgtg gagatacttt ttggcgagaa aatgacttcc 1380
   agtgatgttg tagctggatc cgattaa 1407
<210> 56
   <211> 1413
   <212> DNA
   <213> Homo sapiens
<400> 56
   atgaatgaaa gtcctgatcc gactgacctg gcgggagtca tcattgagct cggccccaat 60
   gacagtccac agacaagtga atttaaagga gcaaccgagg aggcacctgc gaaagaaagt 120
   gtgttagcac gactttccaa gtttgaagtt gaagatgctg aaaatgttgc ttcatatgac 180
   agcaagatta agaaaattgt gcattcaatt gtatcatcct ttgcatttgg actatttgga 240
   gttttcctgg tcttactgga tgtcactctc atccttgccg acctaatttt cactgacagc 300
   aaactttata ttcctttgga gtatcgttct atttctctag ctattgcctt attttttctc 360
   atggatgttc ttcttcgagt atttgtagaa aggagacagc agtatttttc tgacttattt 420
   aacattttag atactgccat tattgtgatt cttctgctgg ttgatgtcgt ttacattttt 480
   tttgacatta agttgcttag gaatattccc agatggacac atttacttcg acttctacga 540
   cttattattc tgttaagaat ttttcatctg tttcatcaaa aaagacaact tgaaaagctg 600
   ataagaaggc gggtttcaga aaacaaaagg cgatacacaa gggatggatt tgacctagac 660
   ctcacttacg ttacagaacg tattattgct atgtcatttc catcttctgg aaggcagtct 720
   ttctatagaa atccaatcaa ggaagttgtg cggtttctag ataagaaaca ccgaaaccac 780
   tatcgagtct acaatctatg cagtgaaaga gcttacgatc ctaagcactt ccataatagg 840
   gtcgttagaa tcatgattga tgatcataat gtccccactc tacatcagat ggtggttttc 900
   accaaggaag taaatgagtg gatggctcaa gatcttgaaa acatcgtagc gattcactgt 960
   aaaggaggca cagatagaac aggaactatg gtttgtgcct tccttattgc ctctgaaata 1020
   tgttcaactg caaaggaaag cctgtattat tttggagaaa ggcgaacaga taaaacccac 1080
   agcgaaaaat ttcagggagt agaaactcct tctgtacttg ataacattac aacagacaaa 1140
   atattaattg atgtattcga cggtccacct ctgtatgatg atgtgaaagt gcagtttttc 1200
   tattcgaatc ttcctacata ctatgacaat tgctcatttt acttctggtt gcacacatct 1260
   tttattgaaa ataacaggct ttatctacca aaaaatgaat tggataatct acataaacaa 1320
   aaagcacgga gaatttatcc atcagatttt gccgtggaga tactttttgg cgagaaaatg 1380
   acttccagtg atgttgtagc tggatccgat taa 1413
<210> 57
   <211> 1353
   <212> DNA
   <213> Homo sapiens
<400> 57
   atgaatgaaa gtcctgatcc gactgacctg gcgggagtca tcattgagct cggccccaat 60
   gacagtccac agacaagtga atttaaagga gcaaccgagg aggcacctgc gaaagaaagt 120
   gtgttagcac gactttccaa gtttgaagtt gaagatgctg aaaatgttgc ttcatatgac 180
   agcaagatta agaaaattgt gcattcaatt gtatcatcct ttgcatttgg actatttgga 240
   gttttcctgg tcttactgga tgtcactctc atccttgccg acctaatttt cactgacagc 300
   aaactttata ttcctttgga gtatcgttct atttctctag ctattgcctt attttttctc 360
   atggatgttc ttcttcgagt atttgtagaa aggagacagc agtatttttc tgacttattt 420
   aacattttag atactgccat tattgtgatt cttctgctgg ttgatgtcgt ttacattttt 480
   tttgacatta agttgcttag gaatattccc agatggacac atttacttcg acttctacga     540
   cttattattc tgttaagaat ttttcatctg tttcatcaaa aaagacaact tgaaaagctg     600
   ataagaaggc gggtttcaga aaacaaaagg cgatacacaa gggatggatt tgacctagac     660
   ctcacttacg ttacagaacg tattattgct atgtcatttc catcttctgg aaggcagtct     720
   ttctatagaa atccaatcaa ggaagttgtg cggtttctag ataagaaaca ccgaaaccac     780
   tatcgagtct acaatctatg cagtgaaaga gcttacgatc ctaagcactt ccataatagg     840
   gtcgttagaa tcatgattga tgatcataat gtccccactc tacatcagat ggtggttttc     900
   accaaggaag taaatgagtg gatggctcaa gatcttgaaa acatcgtagc gattcactgt     960
   aaaggaggca caggttatgt acgtgatcta aaaatccaaa tagaaatgga gaaaaaggtt    1020
   gtcttttcca ctatttcatt aggaaaatgt tcggtacttg ataacattac aacagacaaa    1080
   atattaattg atgtattcga cggtccacct ctgtatgatg atgtgaaagt gcagtttttc    1140
   tattcgaatc ttcctacata ctatgacaat tgctcatttt acttctggtt gcacacatct    1200
   tttattgaaa ataacaggct ttatctacca aaaaatgaat tggataatct acataaacaa    1260
   aaagcacgga gaatttatcc atcagatttt gccgtggaga tactttttgg cgagaaaatg    1320
   acttccagtg atgttgtagc tggatccgat taa                                 1353
<210> 58
   <211> 395
   <212> PRT
   <213> Homo sapiens
<400> 58
   Met Asn Glu Ser Pro Asp Pro Thr Asp Leu Ala Gly Val Ile Ile Glu
   1               5                   10                  15
   Leu Gly Pro Asn Asp Ser Pro Gln Thr Ser Glu Phe Lys Gly Ala Thr
               20                  25                  30
   Glu Glu Ala Pro Ala Lys Glu Ser Pro His Thr Ser Glu Phe Lys Gly
           35                  40                  45
   Ala Ala Arg Val Ser Pro Ile Ser Glu Ser Val Leu Ala Arg Leu Ser
       50                  55                  60
   Lys Phe Glu Val Glu Asp Ala Glu Asn Val Ala Ser Tyr Asp Ser Lys
   65 70 75 80
   Ile Lys Lys Ile Val His Ser Ile Val Ser Ser Phe Ala Phe Gly Leu
   85 90 95
   Phe Gly Val Phe Leu Val Leu Leu Asp Val Thr Leu Ile Leu Ala Asp
   100 105 110
   Leu Ile Phe Thr Asp Ser Lys Leu Tyr Ile Pro Leu Glu Tyr Arg Ser
   115 120 125
   Ile Ser Leu Ala Ile Ala Leu Phe Phe Leu Met Asp Val Leu Leu Arg
   130 135 140
   Val Phe Val Glu Arg Arg Gln Gln Tyr Phe Ser Asp Leu Phe Asn Ile
   145 150 155 160
   Leu Asp Thr Ala Ile Ile Val Ile Leu Leu Leu Val Asp Val Val Tyr
   165 170 175
   Ile Phe Phe Asp Ile Lys Leu Leu Arg Asn Ile Pro Arg Trp Thr His
   180 185 190
   Leu Leu Arg Leu Leu Arg Leu Ile Ile Leu Leu Arg Ile Phe His Leu
   195 200 205
   Phe His Gln Lys Arg Gln Leu Glu Lys Leu Ile Arg Arg Arg Val Ser
   210 215 220
   Glu Asn Lys Arg Arg Tyr Thr Arg Asp Gly Phe Asp Leu Asp Leu Thr
   225 230 235 240
   Tyr Val Thr Glu Arg Ile Ile Ala Met Ser Phe Pro Ser Ser Gly Arg
   245 250 255
   Gln Ser Phe Tyr Arg Asn Pro Ile Lys Glu Val Val Arg Phe Leu Asp
   260 265 270
   Lys Lys His Arg Asn His Tyr Arg Val Tyr Asn Leu Cys Ser Glu Arg
   275 280 285
   Ala Tyr Asp Pro Lys His Phe His Asn Arg Val Val Arg Ile Met Ile
   290 295 300
   Asp Asp His Asn Val Pro Thr Leu His Gln Met Val Val Phe Thr Lys
   305 310 315 320
   Glu Val Asn Glu Trp Met Ala Gln Asp Leu Glu Asn Ile Val Ala Ile
   325 330 335
   His Cys Lys Gly Gly Thr Asp Arg Thr Gly Thr Met Val Cys Ala Phe
   340 345 350
   Leu Ile Ala Ser Glu Ile Cys Ser Thr Ala Lys Glu Ser Leu Tyr Tyr
   355 360 365
   Phe Gly Glu Arg Arg Thr Asp Lys Thr His Ser Glu Lys Phe Gln Gly
   370 375 380
   Val Glu Thr Pro Ser Gln Val Met Tyr Val Ile
   385 390 395
<210> 59
   <211> 468
   <212> PRT
   <213> Homo sapiens
<400> 59
   Met Asn Glu Ser Pro Asp Pro Thr Asp Leu Ala Gly Val Ile Ile Glu
   1 5 10 15
   Leu Gly Pro Asn Asp Ser Pro Gln Thr Ser Glu Phe Lys Gly Ala Thr
   20 25 30
   Glu Glu Ala Pro Ala Lys Glu Ser Pro His Thr Ser Glu Phe Lys Gly
   35 40 45
   Ala Ala Arg Val Ser Pro Ile Ser Glu Ser Val Leu Ala Arg Leu Ser
   50 55 60
   Lys Phe Glu Val Glu Asp Ala Glu Asn Val Ala Ser Tyr Asp Ser Lys
   65 70 75 80
   Ile Lys Lys Ile Val His Ser Ile Val Ser Ser Phe Ala Phe Gly Leu
   85 90 95
   Phe Gly Val Phe Leu Val Leu Leu Asp Val Thr Leu Ile Leu Ala Asp
               100                 105                 110
   Leu Ile Phe Thr Asp Ser Lys Leu Tyr Ile Pro Leu Glu Tyr Arg Ser
           115                 120                 125
   Ile Ser Leu Ala Ile Ala Leu Phe Phe Leu Met Asp Val Leu Leu Arg
       130                 135                 140
   Val Phe Val Glu Arg Arg Gln Gln Tyr Phe Ser Asp Leu Phe Asn Ile
   145                 150                 155                 160
   Leu Asp Thr Ala Ile Ile Val Ile Leu Leu Leu Val Asp Val Val Tyr
                   165                 170                 175
   Ile Phe Phe Asp Ile Lys Leu Leu Arg Asn Ile Pro Arg Trp Thr His
               180                 185                 190
   Leu Leu Arg Leu Leu Arg Leu Ile Ile Leu Leu Arg Ile Phe His Leu
           195                 200                 205
   Phe His Gln Lys Arg Gln Leu Glu Lys Leu Ile Arg Arg Arg Val Ser
       210                 215                 220
   Glu Asn Lys Arg Arg Tyr Thr Arg Asp Gly Phe Asp Leu Asp Leu Thr
   225                 230                 235                 240
   Tyr Val Thr Glu Arg Ile Ile Ala Met Ser Phe Pro Ser Ser Gly Arg
                   245                 250                 255
   Gln Ser Phe Tyr Arg Asn Pro Ile Lys Glu Val Val Arg Phe Leu Asp
               260                 265                 270
   Lys Lys His Arg Asn His Tyr Arg Val Tyr Asn Leu Cys Ser Glu Arg
           275                 280                 285
   Ala Tyr Asp Pro Lys His Phe His Asn Arg Val Val Arg Ile Met Ile
       290                 295                 300
   Asp Asp His Asn Val Pro Thr Leu His Gln Met Val Val Phe Thr Lys
   305                 310                 315                 320
   Glu Val Asn Glu Trp Met Ala Gln Asp Leu Glu Asn Ile Val Ala Ile
                   325                 330                 335
   His Cys Lys Gly Gly Thr Gly Tyr Val Arg Asp Leu Lys Ile Gln Ile
   340 345 350
   Glu Met Glu Lys Lys Val Val Phe Ser Thr Ile Ser Leu Gly Lys Cys
   355 360 365
   Ser Val Leu Asp Asn Ile Thr Thr Asp Lys Ile Leu Ile Asp Val Phe
   370 375 380
   Asp Gly Pro Pro Leu Tyr Asp Asp Val Lys Val Gln Phe Phe Tyr Ser
   385 390 395 400
   Asn Leu Pro Thr Tyr Tyr Asp Asn Cys Ser Phe Tyr Phe Trp Leu His
   405 410 415
   Thr Ser Phe Ile Glu Asn Asn Arg Leu Tyr Leu Pro Lys Asn Glu Leu
   420 425 430
   Asp Asn Leu His Lys Gln Lys Ala Arg Arg Ile Tyr Pro Ser Asp Phe
   435 440 445
   Ala Val Glu Ile Leu Phe Gly Glu Lys Met Thr Ser Ser Asp Val Val
   450 455 460
   Ala Gly Ser Asp
   465
<210> 60
   <211> 470
   <212> PRT
   <213> Homo sapiens
<400> 60
   Met Asn Glu Ser Pro Asp Pro Thr Asp Leu Ala Gly Val Ile Ile Glu
   1 5 10 15
   Leu Gly Pro Asn Asp Ser Pro Gln Thr Ser Glu Phe Lys Gly Ala Thr
   20 25 30
   Glu Glu Ala Pro Ala Lys Glu Ser Val Leu Ala Arg Leu Ser Lys Phe
   35 40 45
   Glu Val Glu Asp Ala Glu Asn Val Ala Ser Tyr Asp Ser Lys Ile Lys
       50                  55                  60
   Lys Ile Val His Ser Ile Val Ser Ser Phe Ala Phe Gly Leu Phe Gly
   65                  70                  75                  80
   Val Phe Leu Val Leu Leu Asp Val Thr Leu Ile Leu Ala Asp Leu Ile
                   85                  90                  95
   Phe Thr Asp Ser Lys Leu Tyr Ile Pro Leu Glu Tyr Arg Ser Ile Ser
               100                 105                 110
   Leu Ala Ile Ala Leu Phe Phe Leu Met Asp Val Leu Leu Arg Val Phe
           115                 120                 125
   Val Glu Arg Arg Gln Gln Tyr Phe Ser Asp Leu Phe Asn Ile Leu Asp
       130                 135                 140
   Thr Ala Ile Ile Val Ile Leu Leu Leu Val Asp Val Val Tyr Ile Phe
   145                 150                 155                 160
   Phe Asp Ile Lys Leu Leu Arg Asn Ile Pro Arg Trp Thr His Leu Leu
                   165                 170                 175
   Arg Leu Leu Arg Leu Ile Ile Leu Leu Arg Ile Phe His Leu Phe His
               180                 185                 190
   Gln Lys Arg Gln Leu Glu Lys Leu Ile Arg Arg Arg Val Ser Glu Asn
           195                 200                 205
   Lys Arg Arg Tyr Thr Arg Asp Gly Phe Asp Leu Asp Leu Thr Tyr Val
       210                 215                 220
   Thr Glu Arg Ile Ile Ala Met Ser Phe Pro Ser Ser Gly Arg Gln Ser
   225                 230                 235                 240
   Phe Tyr Arg Asn Pro Ile Lys Glu Val Val Arg Phe Leu Asp Lys Lys
                   245                 250                 255
   His Arg Asn His Tyr Arg Val Tyr Asn Leu Cys Ser Glu Arg Ala Tyr
               260                 265                 270
   Asp Pro Lys His Phe His Asn Arg Val Val Arg Ile Met Ile Asp Asp
           275                 280                 285
   His Asn Val Pro Thr Leu His Gln Met Val Val Phe Thr Lys Glu Val
       290                 295                 300
   Asn Glu Trp Met Ala Gln Asp Leu Glu Asn Ile Val Ala Ile His Cys
   305                 310                 315                 320
   Lys Gly Gly Thr Asp Arg Thr Gly Thr Met Val Cys Ala Phe Leu Ile
                   325                 330                 335
   Ala Ser Glu Ile Cys Ser Thr Ala Lys Glu Ser Leu Tyr Tyr Phe Gly
               340                 345                 350
   Glu Arg Arg Thr Asp Lys Thr His Ser Glu Lys Phe Gln Gly Val Glu
           355                 360                 365
   Thr Pro Ser Val Leu Asp Asn Ile Thr Thr Asp Lys Ile Leu Ile Asp
       370                 375                 380
   Val Phe Asp Gly Pro Pro Leu Tyr Asp Asp Val Lys Val Gln Phe Phe
   385                 390                 395                 400
   Tyr Ser Asn Leu Pro Thr Tyr Tyr Asp Asn Cys Ser Phe Tyr Phe Trp
                   405                 410                 415
   Leu His Thr Ser Phe Ile Glu Asn Asn Arg Leu Tyr Leu Pro Lys Asn
               420                 425                 430
   Glu Leu Asp Asn Leu His Lys Gln Lys Ala Arg Arg Ile Tyr Pro Ser
           435                 440                 445
   Asp Phe Ala Val Glu Ile Leu Phe Gly Glu Lys Met Thr Ser Ser Asp
       450                 455                 460
   Val Val Ala Gly Ser Asp
   465                 470
<210> 61
   <211> 450
   <212> PRT
   <213> Homo sapiens
<400> 61
   Met Asn Glu Ser Pro Asp Pro Thr Asp Leu Ala Gly Val Ile Ile Glu
   1               5                   10                  15
   Leu Gly Pro Asn Asp Ser Pro Gln Thr Ser Glu Phe Lys Gly Ala Thr
               20                  25                  30
   Glu Glu Ala Pro Ala Lys Glu Ser Val Leu Ala Arg Leu Ser Lys Phe
           35                  40                  45
   Glu Val Glu Asp Ala Glu Asn Val Ala Ser Tyr Asp Ser Lys Ile Lys
       50                  55                  60
   Lys Ile Val His Ser Ile Val Ser Ser Phe Ala Phe Gly Leu Phe Gly
   65                  70                  75                  80
   Val Phe Leu Val Leu Leu Asp Val Thr Leu Ile Leu Ala Asp Leu Ile
                   85                  90                  95
   Phe Thr Asp Ser Lys Leu Tyr Ile Pro Leu Glu Tyr Arg Ser Ile Ser
               100                 105                 110
   Leu Ala Ile Ala Leu Phe Phe Leu Met Asp Val Leu Leu Arg Val Phe
           115                 120                 125
   Val Glu Arg Arg Gln Gln Tyr Phe Ser Asp Leu Phe Asn Ile Leu Asp
       130                 135                 140
   Thr Ala Ile Ile Val Ile Leu Leu Leu Val Asp Val Val Tyr Ile Phe
   145                 150                 155                 160
   Phe Asp Ile Lys Leu Leu Arg Asn Ile Pro Arg Trp Thr His Leu Leu
                   165                 170                 175
   Arg Leu Leu Arg Leu Ile Ile Leu Leu Arg Ile Phe His Leu Phe His
               180                 185                 190
   Gln Lys Arg Gln Leu Glu Lys Leu Ile Arg Arg Arg Val Ser Glu Asn
           195                 200                 205
   Lys Arg Arg Tyr Thr Arg Asp Gly Phe Asp Leu Asp Leu Thr Tyr Val
       210                 215                 220
   Thr Glu Arg Ile Ile Ala Met Ser Phe Pro Ser Ser Gly Arg Gln Ser
   225                 230                 235                 240
   Phe Tyr Arg Asn Pro Ile Lys Glu Val Val Arg Phe Leu Asp Lys Lys
                   245                 250                 255
   His Arg Asn His Tyr Arg Val Tyr Asn Leu Cys Ser Glu Arg Ala Tyr
               260                 265                 270
   Asp Pro Lys His Phe His Asn Arg Val Val Arg Ile Met Ile Asp Asp
           275                 280                 285
   His Asn Val Pro Thr Leu His Gln Met Val Val Phe Thr Lys Glu Val
       290                 295                 300
   Asn Glu Trp Met Ala Gln Asp Leu Glu Asn Ile Val Ala Ile His Cys
   305                 310                 315                 320
   Lys Gly Gly Thr Gly Tyr Val Arg Asp Leu Lys Ile Gln Ile Glu Met
                   325                 330                 335
   Glu Lys Lys Val Val Phe Ser Thr Ile Ser Leu Gly Lys Cys Ser Val
               340                 345                 350
   Leu Asp Asn Ile Thr Thr Asp Lys Ile Leu Ile Asp Val Phe Asp Gly
           355                 360                 365
   Pro Pro Leu Tyr Asp Asp Val Lys Val Gln Phe Phe Tyr Ser Asn Leu
       370                 375                 380
   Pro Thr Tyr Tyr Asp Asn Cys Ser Phe Tyr Phe Trp Leu His Thr Ser
   385                 390                 395                 400
   Phe Ile Glu Asn Asn Arg Leu Tyr Leu Pro Lys Asn Glu Leu Asp Asn
                   405                 410                 415
   Leu His Lys Gln Lys Ala Arg Arg Ile Tyr Pro Ser Asp Phe Ala Val
               420                 425                 430
   Glu Ile Leu Phe Gly Glu Lys Met Thr Ser Ser Asp Val Val Ala Gly
           435                 440                 445
   Ser Asp
       450
<210> 81
   <211> 14
   <212> PRT
   <213> Homo sapiens
<400> 81
   Phe Thr Asp Ser Lys Leu Tyr Ile Pro Leu Glu Tyr Arg Ser
   1               5                   10
<210> 82
   <211> 13
   <212> PRT
   <213> Homo sapiens
<400> 82
   Phe Asp Ile Lys Leu Leu Arg Asn Ile Pro Arg Trp Thr
   1               5                   10
<210> 103
   <211> 15
   <212> PRT
   <213> Homo sapiens
<400> 103
   Phe Thr Asp Ser Lys Leu Tyr Ile Pro Leu Glu Tyr Arg Ser Cys
   1               5                   10                  15
<210> 104
   <211> 14
   <212> PRT
   <213> Homo sapiens
<400> 104
   Cys Phe Asp Ile Lys Leu Leu Arg Asn Ile Pro Arg Trp Thr
   1               5                   10
<210> 105
   <211> 54
   <212> PRT
   <213> Homo sapiens
<400> 105
   Met Asn Glu Ser Pro Asp Pro Thr Asp Leu Ala Gly Val Ile Ile Glu
   1               5                   10                  15
   Leu Gly Pro Asn Asp Ser Pro Gln Thr Ser Glu Phe Lys Gly Ala Thr
               20                  25                  30
   Glu Glu Ala Pro Ala Lys Glu Ser Pro His Thr Ser Glu Phe Lys Gly
           35                  40                  45
   Ala Ala Arg Val Ser Pro
       50
<210> 383
   <211> 8
   <212> PRT
   <213> Homo sapiens
<400> 383
   Ser Pro Asp Pro Thr Asp Leu Ala
   1               5
<210> 384
   <211> 8
   <212> PRT
   <213> Homo sapiens
<400> 384
   Gln Thr Ser Glu Phe Lys Gly Ala
   1               5
<210> 385
   <211> 8
   <212> PRT
   <213> Homo sapiens
<400> 385
   Phe Lys Gly Ala Thr Glu Glu Ala
   1               5
<210> 386
   <211> 8
   <212> PRT
   <213> Homo sapiens
<400> 386
   Gly Ala Thr Glu Glu Ala Pro Ala
   1               5
<210> 387
   <211> 8
   <212> PRT
   <213> Homo sapiens
<400> 387
   His Thr Ser Glu Phe Lys Gly Ala
   1               5
<210> 388
   <211> 8
   <212> PRT
   <213> Homo sapiens
<400> 388
   Thr Ser Glu Phe Lys Gly Ala Ala
   1               5
<210> 389
   <211> 8
   <212> PRT
   <213> Homo sapiens
<400> 389
   Pro Ile Ser Glu Ser Val Leu Ala
   1               5
<210> 390
   <211> 8
   <212> PRT
   <213> Homo sapiens
<400> 390
   Ser Lys Phe Glu Val Glu Asp Ala
   1               5
<210> 391
   <211> 8
   <212> PRT
   <213> Homo sapiens
<400> 391
   Val Glu Asp Ala Glu Asn Val Ala
   1               5
<210> 392
   <211> 8
   <212> PRT
   <213> Homo sapiens
<400> 392
   His Ser Ile Val Ser Ser Phe Ala
   1 5
<210> 393
   <211> 8
   <212> PRT
   <213> Homo sapiens
<400> 393
   Leu Asp Val Thr Leu Ile Leu Ala
   1 5
<210> 394
   <211> 8
   <212> PRT
   <213> Homo sapiens
<400> 394
   Glu Tyr Arg Ser Ile Ser Leu Ala
   1 5
<210> 395
   <211> 8
   <212> PRT
   <213> Homo sapiens
<400> 395
   Arg Ser Ile Ser Leu Ala Ile Ala
   1 5
<210> 396
   <211> 8
   <212> PRT
   <213> Homo sapiens
<400> 396
   Leu Phe Asn Ile Leu Asp Thr Ala
   1 5
<210> 397
   <211> 8
   <212> PRT
   <213> Homo sapiens
<400> 397
   Tyr Val Thr Glu Arg Ile Ile Ala
   1 5
<210> 398
   <211> 8
   <212> PRT
   <213> Homo sapiens
<400> 398
   Tyr Asn Leu Cys Ser Glu Arg Ala
   1 5
<210> 399
   <211> 8
   <212> PRT
   <213> Homo sapiens
<400> 399
   Lys Glu Val Asn Glu Trp Met Ala
   1 5
<210> 400
   <211> 8
   <212> PRT
   <213> Homo sapiens
<400> 400
   Gln Asp Leu Glu Asn Ile Val Ala
   1 5
<210> 401
   <211> 8
   <212> PRT
   <213> Homo sapiens
<400> 401
   Arg Thr Gly Thr Met Val Cys Ala
   1 5
<210> 402
   <211> 8
   <212> PRT
   <213> Homo sapiens
<400> 402
   Met Val Cys Ala Phe Leu Ile Ala
   1 5
<210> 403
   <211> 8
   <212> PRT
   <213> Homo sapiens
<400> 403
   Ala Ser Glu Ile Cys Ser Thr Ala
   1               5
<210> 404
   <211> 8
   <212> PRT
   <213> Homo sapiens
<400> 404
   Pro Ser Gln Lys Arg Tyr Val Ala
   1               5
<210> 405
   <211> 8
   <212> PRT
   <213> Homo sapiens
<400> 405
   Lys Arg Tyr Val Ala Tyr Phe Ala
   1               5
<210> 406
   <211> 8
   <212> PRT
   <213> Homo sapiens
<400> 406
   Asp Asn Leu His Lys Gln Lys Ala
   1               5
<210> 407
   <211> 8
   <212> PRT
   <213> Homo sapiens
<400> 407
   Arg Ile Tyr Pro Ser Asp Phe Ala
   1               5
<210> 408
   <211> 8
   <212> PRT
   <213> Homo sapiens
<400> 408
   Met Thr Ser Ser Asp Val Val Ala
   1 5
<210> 409
   <211> 9
   <212> PRT
   <213> Homo sapiens
<400> 409
   Asp Leu Ala Gly Val Ile Ile Glu Leu
   1 5
<210> 410
   <211> 9
   <212> PRT
   <213> Homo sapiens
<400> 410
   Ser Leu Ala Ile Ala Leu Phe Phe Leu
   1 5
<210> 411
   <211> 9
   <212> PRT
   <213> Homo sapiens
<400> 411
   Phe Leu Met Asp Val Leu Leu Arg Val
   1 5
<210> 412
   <211> 9
   <212> PRT
   <213> Homo sapiens
<400> 412
   Leu Leu Leu Val Asp Val Val Tyr Ile
   1 5
<210> 413
   <211> 9
   <212> PRT
   <213> Homo sapiens
<400> 413
   Val Leu Leu Asp Val Thr Leu Ile Leu
   1 5
<210> 414
   <211> 9
   <212> PRT
   <213> Homo sapiens
<400> 414
   Ala Ile Ile Val Ile Leu Leu Leu Val
   1 5
<210> 415
   <211> 9
   <212> PRT
   <213> Homo sapiens
<400> 415
   Arg Leu Leu Arg Leu Ile Ile Leu Leu
   1 5
<210> 416
   <211> 9
   <212> PRT
   <213> Homo sapiens
<400> 416
   Phe Leu Val Leu Leu Asp Val Thr Leu
   1 5
<210> 417
   <211> 9
   <212> PRT
   <213> Homo sapiens
<400> 417
   Val Ile Leu Leu Leu Val Asp Val Val
   1 5
<210> 418
   <211> 9
   <212> PRT
   <213> Homo sapiens
<400> 418
   Ile Ile Leu Leu Arg Ile Phe His Leu
   1 5
<210> 419
   <211> 9
   <212> PRT
   <213> Homo sapiens
<400> 419
   Gly Leu Phe Gly Val Phe Leu Val Leu
   1 5
<210> 420
   <211> 9
   <212> PRT
   <213> Homo sapiens
<400> 420
   Ile Val Ile Leu Leu Leu Val Asp Val
   1 5
<210> 421
   <211> 9
   <212> PRT
   <213> Homo sapiens
<400> 421
   His Leu Leu Arg Leu Leu Arg Leu Ile
   1 5
<210> 422
   <211> 9
   <212> PRT
   <213> Homo sapiens
<400> 422
   Ser Ile Pro Arg Tyr Val Arg Asp Leu
   1 5
<210> 423
   <211> 9
   <212> PRT
   <213> Homo sapiens
<400> 423
   Arg Leu Tyr Leu Pro Lys Asn Glu Leu
   1 5
<210> 424
   <211> 9
   <212> PRT
   <213> Homo sapiens
<400> 424
   Ile Leu Asp Thr Ala Ile Ile Val Ile
   1 5
<210> 425
   <211> 9
   <212> PRT
   <213> Homo sapiens
<400> 425
   Lys Ile Lys Lys Ile Val His Ser Ile
   1 5
<210> 426
   <211> 9
   <212> PRT
   <213> Homo sapiens
<400> 426
   Ala Leu Phe Phe Leu Met Asp Val Leu
   1 5
<210> 427
   <211> 9
   <212> PRT
   <213> Homo sapiens
<400> 427
   Tyr Ile Phe Phe Asp Ile Lys Leu Leu
   1 5
<210> 428
   <211> 9
   <212> PRT
   <213> Homo sapiens
<400> 428
   Ile Leu Ala Asp Leu Ile Phe Thr Asp
   1 5
<210> 429
   <211> 9
   <212> PRT
   <213> Homo sapiens
<400> 429
   Asp Leu Ile Phe Thr Asp Ser Lys Leu
   1 5
<210> 430
   <211> 9
   <212> PRT
   <213> Homo sapiens
<400> 430
   Ile Ala Leu Phe Phe Leu Met Asp Val
   1 5
<210> 431
   <211> 9
   <212> PRT
   <213> Homo sapiens
<400> 431
   Leu Leu Arg Leu Leu Arg Leu Ile Ile
   1 5
<210> 432
   <211> 9
   <212> PRT
   <213> Homo sapiens
<400> 432
   Gln Met Val Val Phe Thr Lys Glu Val
   1 5
<210> 433
   <211> 9
   <212> PRT
   <213> Homo sapiens
<400> 433
   Trp Met Ala Gln Asp Leu Glu Asn Ile
   1 5
<210> 434
   <211> 9
   <212> PRT
   <213> Homo sapiens
<400> 434
   Leu Ile Ala Ser Glu Ile Cys Ser Thr
   1 5
<210> 435
   <211> 9
   <212> PRT
   <213> Homo sapiens
<400> 435
   Ile Leu Phe Ile Lys His Phe Ile Ile
   1               5
<210> 436
   <211> 9
   <212> PRT
   <213> Homo sapiens
<400> 436
   Thr Ile Ser Leu Gly Lys Cys Ser Val
   1               5
<210> 437
   <211> 9
   <212> PRT
   <213> Homo sapiens
<400> 437
   Arg Ile Tyr Pro Ser Asp Phe Ala Val
   1               5
<210> 438
   <211> 9
   <212> PRT
   <213> Homo sapiens
<400> 438
   Gly Val Phe Leu Val Leu Leu Asp Val
   1               5
<210> 439
   <211> 9
   <212> PRT
   <213> Homo sapiens
<400> 439
   Asn Ile Leu Asp Thr Ala Ile Ile Val
   1               5
<210> 440
   <211> 9
   <212> PRT
   <213> Homo sapiens
<400> 440
   Thr Ala Ile Ile Val Ile Leu Leu Leu
   1 5
<210> 441
   <211> 9
   <212> PRT
   <213> Homo sapiens
<400> 441
   Asn Ile Pro Arg Trp Thr His Leu Leu
   1 5
<210> 442
   <211> 9
   <212> PRT
   <213> Homo sapiens
<400> 442
   His Leu Phe His Gln Lys Arg Gln Leu
   1 5
<210> 443
   <211> 9
   <212> PRT
   <213> Homo sapiens
<400> 443
   Ile Ile Tyr Ser Ile Pro Arg Tyr Val
   1 5
<210> 444
   <211> 9
   <212> PRT
   <213> Homo sapiens
<400> 444
   Ser Phe Ala Phe Gly Leu Phe Gly Val
   1 5
<210> 445
   <211> 9
   <212> PRT
   <213> Homo sapiens
<400> 445
   Leu Val Leu Leu Asp Val Thr Leu Ile
   1 5
<210> 446
   <211> 9
   <212> PRT
   <213> Homo sapiens
<400> 446
   Leu Leu Asp Val Thr Leu Ile Leu Ala
   1 5
<210> 447
   <211> 9
   <212> PRT
   <213> Homo sapiens
<400> 447
   Leu Ile Leu Ala Asp Leu Ile Phe Thr
   1 5
<210> 448
   <211> 9
   <212> PRT
   <213> Homo sapiens
<400> 448
   Tyr Ile Pro Leu Glu Tyr Arg Ser Ile
   1 5
<210> 449
   <211> 9
   <212> PRT
   <213> Homo sapiens
<400> 449
   Asp Leu Phe Asn Ile Leu Asp Thr Ala
   1 5
<210> 450
   <211> 9
   <212> PRT
   <213> Homo sapiens
<400> 450
   Pro Ile Lys Glu Val Val Arg Phe Leu
   1 5
<210> 451
   <211> 9
   <212> PRT
   <213> Homo sapiens
<400> 451
   Arg Ile Met Ile Asp Asp His Asn Val
   1 5
<210> 452
   <211> 9
   <212> PRT
   <213> Homo sapiens
<400> 452
   Thr Leu His Gln Met Val Val Phe Thr
   1 5
<210> 453
   <211> 9
   <212> PRT
   <213> Homo sapiens
<400> 453
   Leu Phe Gly Val Phe Leu Val Leu Leu
   1 5
<210> 454
   <211> 9
   <212> PRT
   <213> Homo sapiens
<400> 454
   Asp Thr Ala Ile Ile Val Ile Leu Leu
   1 5
<210> 455
   <211> 9
   <212> PRT
   <213> Homo sapiens
<400> 455
   Ile Asp Asp His Asn Val Pro Thr Leu
   1 5
<210> 456
   <211> 9
   <212> PRT
   <213> Homo sapiens
<400> 456
   Gln Asp Leu Glu Asn Ile Val Ala Ile
   1 5
<210> 457
   <211> 9
   <212> PRT
   <213> Homo sapiens
<400> 457
   Phe Ile Glu Asn Asn Arg Leu Tyr Leu
   1 5
<210> 458
   <211> 9
   <212> PRT
   <213> Homo sapiens
<400> 458
   Arg Val Ser Pro Ile Ser Glu Ser Val
   1 5
<210> 459
   <211> 9
   <212> PRT
   <213> Homo sapiens
<400> 459
   Leu Ala Arg Leu Ser Lys Phe Glu Val
   1 5
<210> 460
   <211> 9
   <212> PRT
   <213> Homo sapiens
<400> 460
   Ile Val Ser Ser Phe Ala Phe Gly Leu
   1 5
<210> 461
   <211> 9
   <212> PRT
   <213> Homo sapiens
<400> 461
   Phe Gly Leu Phe Gly Val Phe Leu Val
   1 5
<210> 462
   <211> 9
   <212> PRT
   <213> Homo sapiens
<400> 462
   Asp Val Thr Leu Ile Leu Ala Asp Leu
   1 5
<210> 463
   <211> 9
   <212> PRT
   <213> Homo sapiens
<400> 463
   Leu Leu Val Asp Val Val Tyr Ile Phe
   1 5
<210> 464
   <211> 9
   <212> PRT
   <213> Homo sapiens
<400> 464
   Lys Leu Leu Arg Asn Ile Pro Arg Trp
   1 5
<210> 465
   <211> 9
   <212> PRT
   <213> Homo sapiens
<400> 465
   Leu Arg Leu Leu Arg Leu Ile Ile Leu
   1 5
<210> 466
   <211> 9
   <212> PRT
   <213> Homo sapiens
<400> 466
   Arg Ile Leu Phe Ile Lys His Phe Ile
   1 5
<210> 467
   <211> 9
   <212> PRT
   <213> Homo sapiens
<400> 467
   Lys Val Val Phe Ser Thr Ile Ser Leu
   1 5
<210> 468
   <211> 9
   <212> PRT
   <213> Homo sapiens
<400> 468
   Thr Thr Asp Lys Ile Leu Ile Asp Val
   1 5
<210> 469
   <211> 10
   <212> PRT
   <213> Homo sapiens
<400> 469
   Gly Leu Phe Gly Val Phe Leu Val Leu Leu
   1 5 10
<210> 470
   <211> 10
   <212> PRT
   <213> Homo sapiens
<400> 470
   Ile Leu Asp Thr Ala Ile Ile Val Ile Leu
   1 5 10
<210> 471
   <211> 10
   <212> PRT
   <213> Homo sapiens
<400> 471
   Ile Leu Leu Leu Val Asp Val Val Tyr Ile
   1 5 10
<210> 472
   <211> 10
   <212> PRT
   <213> Homo sapiens
<400> 472
   Ala Ile Ala Leu Phe Phe Leu Met Asp Val
   1 5 10
<210> 473
   <211> 10
   <212> PRT
   <213> Homo sapiens
<400> 473
   Ile Ile Val Ile Leu Leu Leu Val Asp Val
   1 5 10
<210> 474
   <211> 10
   <212> PRT
   <213> Homo sapiens
<400> 474
   Leu Leu Arg Leu Leu Arg Leu Ile Ile Leu
   1 5 10
<210> 475
   <211> 10
   <212> PRT
   <213> Homo sapiens
<400> 475
   Leu Ile Ile Leu Leu Arg Ile Phe His Leu
   1 5 10
<210> 476
   <211> 10
   <212> PRT
   <213> Homo sapiens
<400> 476
   Met Ile Asp Asp His Asn Val Pro Thr Leu
   1 5 10
<210> 477
   <211> 10
   <212> PRT
   <213> Homo sapiens
<400> 477
   Tyr Leu Pro Lys Asn Glu Leu Asp Asn Leu
   1 5 10
<210> 478
   <211> 10
   <212> PRT
   <213> Homo sapiens
<400> 478
   Val Leu Ala Arg Leu Ser Lys Phe Glu Val
   1 5 10
<210> 479
   <211> 10
   <212> PRT
   <213> Homo sapiens
<400> 479
   Leu Leu Arg Leu Ile Ile Leu Leu Arg Ile
   1 5 10
<210> 480
   <211> 10
   <212> PRT
   <213> Homo sapiens
<400> 480
   Asn Leu Pro Pro Arg Arg Ile Leu Phe Ile
   1 5 10
<210> 481
   <211> 10
   <212> PRT
   <213> Homo sapiens
<400> 481
   Ala Leu Phe Phe Leu Met Asp Val Leu Leu
   1 5 10
<210> 482
   <211> 10
   <212> PRT
   <213> Homo sapiens
<400> 482
   Pro Ile Ser Glu Ser Val Leu Ala Arg Leu
   1 5 10
<210> 483
   <211> 10
   <212> PRT
   <213> Homo sapiens
<400> 483
   Phe Leu Val Leu Leu Asp Val Thr Leu Ile
   1 5 10
<210> 484
   <211> 10
   <212> PRT
   <213> Homo sapiens
<400> 484
   Asn Ile Leu Asp Thr Ala Ile Ile Val Ile
   1 5 10
<210> 485
   <211> 10
   <212> PRT
   <213> Homo sapiens
<400> 485
   Phe Leu Ile Ala Ser Glu Ile Cys Ser Thr
   1 5 10
<210> 486
   <211> 10
   <212> PRT
   <213> Homo sapiens
<400> 486
   Phe Ile Lys His Phe Ile Ile Tyr Ser Ile
   1 5 10
<210> 487
   <211> 10
   <212> PRT
   <213> Homo sapiens
<400> 487
   Val Leu Leu Asp Val Thr Leu Ile Leu Ala
   1 5 10
<210> 488
   <211> 10
   <212> PRT
   <213> Homo sapiens
<400> 488
   Leu Met Asp Val Leu Leu Arg Val Phe Val
   1 5 10
<210> 489
   <211> 10
   <212> PRT
   <213> Homo sapiens
<400> 489
   Ile Val Ile Leu Leu Leu Val Asp Val Val
   1 5 10
<210> 490
   <211> 10
   <212> PRT
   <213> Homo sapiens
<400> 490
   Ser Pro Asp Pro Thr Asp Leu Ala Gly Val
   1 5 10
<210> 491
   <211> 10
   <212> PRT
   <213> Homo sapiens
<400> 491
   Ser Ile Val Ser Ser Phe Ala Phe Gly Leu
   1 5 10
<210> 492
   <211> 10
   <212> PRT
   <213> Homo sapiens
<400> 492
   Thr Ala Ile Ile Val Ile Leu Leu Leu Val
   1 5 10
<210> 493
   <211> 10
   <212> PRT
   <213> Homo sapiens
<400> 493
   His Leu Leu Arg Leu Leu Arg Leu Ile Ile
   1 5 10
<210> 494
   <211> 10
   <212> PRT
   <213> Homo sapiens
<400> 494
   Trp Met Ala Gln Asp Leu Glu Asn Ile Val
   1 5 10
<210> 495
   <211> 10
   <212> PRT
   <213> Homo sapiens
<400> 495
   Val Leu Asp Asn Ile Thr Thr Asp Lys Ile
   1 5 10
<210> 496
   <211> 10
   <212> PRT
   <213> Homo sapiens
<400> 496
   Leu Ile Asp Val Phe Asp Gly Pro Pro Leu
   1 5 10
<210> 497
   <211> 10
   <212> PRT
   <213> Homo sapiens
<400> 497
   Phe Ala Phe Gly Leu Phe Gly Val Phe Leu
   1 5 10
<210> 498
   <211> 10
   <212> PRT
   <213> Homo sapiens
<400> 498
   Thr Leu Ile Leu Ala Asp Leu Ile Phe Thr
   1 5 10
<210> 499
   <211> 10
   <212> PRT
   <213> Homo sapiens
<400> 499
   Leu Ile Phe Thr Asp Ser Lys Leu Tyr Ile
   1 5 10
<210> 500
   <211> 10
   <212> PRT
   <213> Homo sapiens
<400> 500
   Asp Thr Ala Ile Ile Val Ile Leu Leu Leu
   1 5 10
<210> 501
   <211> 10
   <212> PRT
   <213> Homo sapiens
<400> 501
   Phe Ile Ile Tyr Ser Ile Pro Arg Tyr Val
   1 5 10
<210> 502
   <211> 10
   <212> PRT
   <213> Homo sapiens
<400> 502
   Tyr Ser Ile Pro Arg Tyr Val Arg Asp Leu
   1 5 10
<210> 503
   <211> 10
   <212> PRT
   <213> Homo sapiens
<400> 503
   Lys Ile Gln Ile Glu Met Glu Lys Lys Val
   1 5 10
<210> 504
   <211> 10
   <212> PRT
   <213> Homo sapiens
<400> 504
   Ser Thr Ile Ser Leu Gly Lys Cys Ser Val
   1 5 10
<210> 505
   <211> 10
   <212> PRT
   <213> Homo sapiens
<400> 505
   Ile Thr Thr Asp Lys Ile Leu Ile Asp Val
   1 5 10
<210> 506
   <211> 10
   <212> PRT
   <213> Homo sapiens
<400> 506
   Ser Lys Ile Lys Lys Ile Val His Ser Ile
   1 5 10
<210> 507
   <211> 10
   <212> PRT
   <213> Homo sapiens
<400> 507
   Leu Val Leu Leu Asp Val Thr Leu Ile Leu
   1 5 10
<210> 508
   <211> 10
   <212> PRT
   <213> Homo sapiens
<400> 508
   Asp Leu Phe Asn Ile Leu Asp Thr Ala Ile
   1 5 10
<210> 509
   <211> 10
   <212> PRT
   <213> Homo sapiens
<400> 509
   Leu Leu Leu Val Asp Val Val Tyr Ile Phe
   1 5 10
<210> 510
   <211> 10
   <212> PRT
   <213> Homo sapiens
<400> 510
   Trp Thr His Leu Leu Arg Leu Leu Arg Leu
   1 5 10
<210> 511
   <211> 10
   <212> PRT
   <213> Homo sapiens
<400> 511
   Gln Leu Glu Lys Leu Ile Arg Arg Arg Val
   1 5 10
<210> 512
   <211> 10
   <212> PRT
   <213> Homo sapiens
<400> 512
   Lys Leu Ile Arg Arg Arg Val Ser Glu Asn
   1 5 10
<210> 513
   <211> 10
   <212> PRT
   <213> Homo sapiens
<400> 513
   Ile Met Ile Asp Asp His Asn Val Pro Thr
   1 5 10
<210> 514
   <211> 10
   <212> PRT
   <213> Homo sapiens
<400> 514
   Thr Asp Leu Ala Gly Val Ile Ile Glu Leu
   1 5 10
<210> 515
   <211> 10
   <212> PRT
   <213> Homo sapiens
<400> 515
   Arg Leu Ser Lys Phe Glu Val Glu Asp Ala
   1 5 10
<210> 516
   <211> 10
   <212> PRT
   <213> Homo sapiens
<400> 516
   Lys Ile Lys Lys Ile Val His Ser Ile Val
   1 5 10
<210> 517
   <211> 10
   <212> PRT
   <213> Homo sapiens
<400> 517
   Ser Ser Phe Ala Phe Gly Leu Phe Gly Val
   1 5 10
<210> 518
   <211> 10
   <212> PRT
   <213> Homo sapiens
<400> 518
   Leu Leu Asp Val Thr Leu Ile Leu Ala Asp
   1 5 10
<210> 519
   <211> 10
   <212> PRT
   <213> Homo sapiens
<400> 519
   Leu Asp Val Thr Leu Ile Leu Ala Asp Leu
   1 5 10
<210> 520
   <211> 10
   <212> PRT
   <213> Homo sapiens
<400> 520
   Leu Ile Leu Ala Asp Leu Ile Phe Thr Asp
   1 5 10
<210> 521
   <211> 10
   <212> PRT
   <213> Homo sapiens
<400> 521
   Ile Pro Leu Glu Tyr Arg Ser Ile Ser Leu
   1 5 10
<210> 522
   <211> 10
   <212> PRT
   <213> Homo sapiens
<400> 522
   Ile Ser Leu Ala Ile Ala Leu Phe Phe Leu
   1 5 10
<210> 523
   <211> 10
   <212> PRT
   <213> Homo sapiens
<400> 523
   Ser Leu Ala Ile Ala Leu Phe Phe Leu Met
   1 5 10
<210> 524
   <211> 10
   <212> PRT
   <213> Homo sapiens
<400> 524
   Phe Phe Leu Met Asp Val Leu Leu Arg Val
   1 5 10
<210> 525
   <211> 10
   <212> PRT
   <213> Homo sapiens
<400> 525
   Val Val Tyr Ile Phe Phe Asp Ile Lys Leu
   1               5                   10
<210> 526
   <211> 10
   <212> PRT
   <213> Homo sapiens
<400> 526
   Leu Arg Leu Leu Arg Leu Ile Ile Leu Leu
   1               5                   10
<210> 527
   <211> 10
   <212> PRT
   <213> Homo sapiens
<400> 527
   Tyr Thr Arg Asp Gly Phe Asp Leu Asp Leu
   1               5                   10
<210> 528
   <211> 10
   <212> PRT
   <213> Homo sapiens
<400> 528
   Ser Phe Tyr Arg Asn Pro Ile Lys Glu Val
   1               5                   10
<210> 529
   <211> 10
   <212> PRT
   <213> Homo sapiens
<400> 529
   Leu Ile Ala Ser Glu Ile Cys Ser Thr Ala
   1               5                   10
<210> 530
   <211> 10
   <212> PRT
   <213> Homo sapiens
<400> 530
   Val Ala Tyr Phe Ala Gln Val Lys His Leu
   1 5 10
<210> 531
   <211> 10
   <212> PRT
   <213> Homo sapiens
<400> 531
   Thr Ile Ser Leu Gly Lys Cys Ser Val Leu
   1 5 10
<210> 532
   <211> 10
   <212> PRT
   <213> Homo sapiens
<400> 532
   Asn Leu His Lys Gln Lys Ala Arg Arg Ile
   1 5 10
<210> 533
   <211> 9
   <212> PRT
   <213> Homo sapiens
<400> 533
   Val Tyr Ile Phe Phe Asp Ile Lys Leu
   1 5
<210> 534
   <211> 9
   <212> PRT
   <213> Homo sapiens
<400> 534
   Ser Tyr Asp Ser Lys Ile Lys Lys Ile
   1 5
<210> 535
   <211> 9
   <212> PRT
   <213> Homo sapiens
<400> 535
   Ala Tyr Phe Ala Gln Val Lys His Leu
   1 5
<210> 536
   <211> 9
   <212> PRT
   <213> Homo sapiens
<400> 536
   Leu Tyr Asp Asp Val Lys Val Gln Phe
   1 5
<210> 537
   <211> 9
   <212> PRT
   <213> Homo sapiens
<400> 537
   Gln Tyr Phe Ser Asp Leu Phe Asn Ile
   1 5
<210> 538
   <211> 9
   <212> PRT
   <213> Homo sapiens
<400> 538
   Arg Tyr Thr Arg Asp Gly Phe Asp Leu
   1 5
<210> 539
   <211> 9
   <212> PRT
   <213> Homo sapiens
<400> 539
   Tyr Tyr Asp Asn Cys Ser Phe Tyr Phe
   1 5
<210> 540
   <211> 9
   <212> PRT
   <213> Homo sapiens
<400> 540
   Phe Tyr Phe Trp Leu His Thr Ser Phe
   1 5
<210> 541
   <211> 9
   <212> PRT
   <213> Homo sapiens
<400> 541
   Glu Tyr Arg Ser Ile Ser Leu Ala Ile
   1 5
<210> 542
   <211> 9
   <212> PRT
   <213> Homo sapiens
<400> 542
   Leu Phe Gly Val Phe Leu Val Leu Leu
   1 5
<210> 543
   <211> 9
   <212> PRT
   <213> Homo sapiens
<400> 543
   Leu Phe Phe Leu Met Asp Val Leu Leu
   1 5
<210> 544
   <211> 9
   <212> PRT
   <213> Homo sapiens
<400> 544
   Tyr Phe Ser Asp Leu Phe Asn Ile Leu
   1 5
<210> 545
   <211> 9
   <212> PRT
   <213> Homo sapiens
<400> 545
   Ile Phe Thr Asp Ser Lys Leu Tyr Ile
   1 5
<210> 546
   <211> 9
   <212> PRT
   <213> Homo sapiens
<400> 546
   Thr Ala Ile Ile Val Ile Leu Leu Leu
   1 5
<210> 547
   <211> 9
   <212> PRT
   <213> Homo sapiens
<400> 547
   His Phe His Asn Arg Val Val Arg Ile
   1 5
<210> 548
   <211> 9
   <212> PRT
   <213> Homo sapiens
<400> 548
   Tyr Phe Trp Leu His Thr Ser Phe Ile
   1 5
<210> 549
   <211> 9
   <212> PRT
   <213> Homo sapiens
<400> 549
   Leu Tyr Leu Pro Lys Asn Glu Leu Asp
   1 5
<210> 550
   <211> 9
   <212> PRT
   <213> Homo sapiens
<400> 550
   Ala Phe Gly Leu Phe Gly Val Phe Leu
   1 5
<210> 551
   <211> 9
   <212> PRT
   <213> Homo sapiens
<400> 551
   Val Leu Leu Asp Val Thr Leu Ile Leu
   1 5
<210> 552
   <211> 9
   <212> PRT
   <213> Homo sapiens
<400> 552
   Val Thr Leu Ile Leu Ala Asp Leu Ile
   1 5
<210> 553
   <211> 9
   <212> PRT
   <213> Homo sapiens
<400> 553
   Leu Phe Asn Ile Leu Asp Thr Ala Ile
   1 5
<210> 554
   <211> 9
   <212> PRT
   <213> Homo sapiens
<400> 554
   Ile Ile Leu Leu Arg Ile Phe His Leu
   1 5
<210> 555
   <211> 9
   <212> PRT
   <213> Homo sapiens
<400> 555
   Ile Leu Leu Arg Ile Phe His Leu Phe
   1 5
<210> 556
   <211> 9
   <212> PRT
   <213> Homo sapiens
<400> 556
   Asn Leu Pro Pro Arg Arg Ile Leu Phe
   1 5
<210> 557
   <211> 9
   <212> PRT
   <213> Homo sapiens
<400> 557
   Ser Val Leu Ala Arg Leu Ser Lys Phe
   1               5
<210> 558
   <211> 9
   <212> PRT
   <213> Homo sapiens
<400> 558
   Val Ser Ser Phe Ala Phe Gly Leu Phe
   1               5
<210> 559
   <211> 9
   <212> PRT
   <213> Homo sapiens
<400> 559
   Leu Val Leu Leu Asp Val Thr Leu Ile
   1               5
<210> 560
   <211> 9
   <212> PRT
   <213> Homo sapiens
<400> 560
   Arg Asn Ile Pro Arg Trp Thr His Leu
   1               5
<210> 561
   <211> 9
   <212> PRT
   <213> Homo sapiens
<400> 561
   Arg Leu Leu Arg Leu Ile Ile Leu Leu
   1               5
<210> 562
   <211> 9
   <212> PRT
   <213> Homo sapiens
<400> 562
   Ala Tyr Asp Pro Lys His Phe His Asn
   1 5
<210> 563
   <211> 9
   <212> PRT
   <213> Homo sapiens
<400> 563
   Gln Asp Leu Glu Asn Ile Val Ala Ile
   1 5
<210> 564
   <211> 10
   <212> PRT
   <213> Homo sapiens
<400> 564
   Val Tyr Ile Phe Phe Asp Ile Lys Leu Leu
   1 5 10
<210> 565
   <211> 10
   <212> PRT
   <213> Homo sapiens
<400> 565
   Leu Tyr Ile Pro Leu Glu Tyr Arg Ser Ile
   1 5 10
<210> 566
   <211> 10
   <212> PRT
   <213> Homo sapiens
<400> 566
   Thr Tyr Tyr Asp Asn Cys Ser Phe Tyr Phe
   1 5 10
<210> 567
   <211> 10
   <212> PRT
   <213> Homo sapiens
<400> 567
   Leu Tyr Asp Asp Val Lys Val Gln Phe Phe
   1 5 10
<210> 568
   <211> 10
   <212> PRT
   <213> Homo sapiens
<400> 568
   Ile Tyr Pro Ser Asp Phe Ala Val Glu Ile
   1 5 10
<210> 569
   <211> 10
   <212> PRT
   <213> Homo sapiens
<400> 569
   Gln Tyr Phe Ser Asp Leu Phe Asn Ile Leu
   1 5 10
<210> 570
   <211> 10
   <212> PRT
   <213> Homo sapiens
<400> 570
   Val Phe Leu Val Leu Leu Asp Val Thr Leu
   1 5 10
<210> 571
   <211> 10
   <212> PRT
   <213> Homo sapiens
<400> 571
   Val Phe Val Glu Arg Arg Gln Gln Tyr Phe
   1 5 10
<210> 572
   <211> 10
   <212> PRT
   <213> Homo sapiens
<400> 572
   Arg Tyr Val Arg Asp Leu Lys Ile Gln Ile
   1 5 10
<210> 573
   <211> 10
   <212> PRT
   <213> Homo sapiens
<400> 573
   Phe Tyr Phe Trp Leu His Thr Ser Phe Ile
   1 5 10
<210> 574
   <211> 10
   <212> PRT
   <213> Homo sapiens
<400> 574
   Ser Phe Ile Glu Asn Asn Arg Leu Tyr Leu
   1 5 10
<210> 575
   <211> 10
   <212> PRT
   <213> Homo sapiens
<400> 575
   Ser Phe Pro Ser Ser Gly Arg Gln Ser Phe
   1 5 10
<210> 576
   <211> 10
   <212> PRT
   <213> Homo sapiens
<400> 576
   Ser Phe Ala Phe Gly Leu Phe Gly Val Phe
   1 5 10
<210> 577
   <211> 10
   <212> PRT
   <213> Homo sapiens
<400> 577
   Ser Phe Tyr Phe Trp Leu His Thr Ser Phe
   1 5 10
<210> 578
   <211> 10
   <212> PRT
   <213> Homo sapiens
<400> 578
   Leu Phe Asn Ile Leu Asp Thr Ala Ile Ile
   1 5 10
<210> 579
   <211> 10
   <212> PRT
   <213> Homo sapiens
<400> 579
   Phe Phe Asp Ile Lys Leu Leu Arg Asn Ile
   1 5 10
<210> 580
   <211> 10
   <212> PRT
   <213> Homo sapiens
<400> 580
   Arg Ser Ile Ser Leu Ala Ile Ala Leu Phe
   1 5 10
<210> 581
   <211> 10
   <212> PRT
   <213> Homo sapiens
<400> 581
   Arg Asn Ile Pro Arg Trp Thr His Leu Leu
   1 5 10
<210> 582
   <211> 10
   <212> PRT
   <213> Homo sapiens
<400> 582
   Thr His Leu Leu Arg Leu Leu Arg Leu Ile
   1 5 10
<210> 583
   <211> 10
   <212> PRT
   <213> Homo sapiens
<400> 583
   Leu Arg Leu Leu Arg Leu Ile Ile Leu Leu
   1 5 10
<210> 584
   <211> 10
   <212> PRT
   <213> Homo sapiens
<400> 584
   Leu Ile Ile Leu Leu Arg Ile Phe His Leu
   1 5 10
<210> 585
   <211> 10
   <212> PRT
   <213> Homo sapiens
<400> 585
   Ile Ile Leu Leu Arg Ile Phe His Leu Phe
   1 5 10
<210> 586
   <211> 10
   <212> PRT
   <213> Homo sapiens
<400> 586
   Trp Asn Leu Pro Pro Arg Arg Ile Leu Phe
   1 5 10
<210> 587
   <211> 9
   <212> PRT
   <213> Homo sapiens
<400> 587
   Ser Thr Ala Lys Glu Ser Leu Tyr Tyr
   1 5
<210> 588
   <211> 9
   <212> PRT
   <213> Homo sapiens
<400> 588
   Ser Lys Leu Tyr Ile Pro Leu Glu Tyr
   1 5
<210> 589
   <211> 9
   <212> PRT
   <213> Homo sapiens
<400> 589
   Ser Pro Asp Pro Thr Asp Leu Ala Gly
   1 5
<210> 590
   <211> 9
   <212> PRT
   <213> Homo sapiens
<400> 590
   Phe Ser Asp Leu Phe Asn Ile Leu Asp
   1 5
<210> 591
   <211> 9
   <212> PRT
   <213> Homo sapiens
<400> 591
   Leu Phe Ile Lys His Phe Ile Ile Tyr
   1 5
<210> 592
   <211> 9
   <212> PRT
   <213> Homo sapiens
<400> 592
   Asp Gly Phe Asp Leu Asp Leu Thr Tyr
   1 5
<210> 593
   <211> 9
   <212> PRT
   <213> Homo sapiens
<400> 593
   Cys Ser Thr Ala Lys Glu Ser Leu Tyr
   1 5
<210> 594
   <211> 9
   <212> PRT
   <213> Homo sapiens
<400> 594
   Tyr Phe Ala Gln Val Lys His Leu Tyr
   1 5
<210> 595
   <211> 9
   <212> PRT
   <213> Homo sapiens
<400> 595
   Pro Ser Ser Gly Arg Gln Ser Phe Tyr
   1 5
<210> 596
   <211> 9
   <212> PRT
   <213> Homo sapiens
<400> 596
   Val Glu Thr Pro Ser Gln Lys Arg Tyr
   1 5
<210> 597
   <211> 9
   <212> PRT
   <213> Homo sapiens
<400> 597
   Thr Thr Asp Lys Ile Leu Ile Asp Val
   1 5
<210> 598
   <211> 9
   <212> PRT
   <213> Homo sapiens
<400> 598
   Ser Phe Ile Glu Asn Asn Arg Leu Tyr
   1 5
<210> 599
   <211> 9
   <212> PRT
   <213> Homo sapiens
<400> 599
   Ala Thr Glu Glu Ala Pro Ala Lys Glu
   1 5
<210> 600
   <211> 9
   <212> PRT
   <213> Homo sapiens
<400> 600
   Ile Leu Leu Leu Val Asp Val Val Tyr
   1 5
<210> 601
   <211> 9
   <212> PRT
   <213> Homo sapiens
<400> 601
   Pro Ser Gln Lys Arg Tyr Val Ala Tyr
   1 5
<210> 602
   <211> 9
   <212> PRT
   <213> Homo sapiens
<400> 602
   Asp Val Phe Asp Gly Pro Pro Leu Tyr
   1 5
<210> 603
   <211> 9
   <212> PRT
   <213> Homo sapiens
<400> 603
   Val Phe Asp Gly Pro Pro Leu Tyr Asp
   1 5
<210> 604
   <211> 9
   <212> PRT
   <213> Homo sapiens
<400> 604
   Val Thr Glu Arg Ile Ile Ala Met Ser
   1 5
<210> 605
   <211> 9
   <212> PRT
   <213> Homo sapiens
<400> 605
   Gly Thr Asp Arg Thr Gly Thr Met Val
   1 5
<210> 606
   <211> 9
   <212> PRT
   <213> Homo sapiens
<400> 606
   Phe Ile Glu Asn Asn Arg Leu Tyr Leu
   1 5
<210> 607
   <211> 9
   <212> PRT
   <213> Homo sapiens
<400> 607
   Ser Ser Asp Val Val Ala Gly Ser Asp
   1 5
<210> 608
   <211> 9
   <212> PRT
   <213> Homo sapiens
<400> 608
   Pro Thr Asp Leu Ala Gly Val Ile Ile
   1 5
<210> 609
   <211> 9
   <212> PRT
   <213> Homo sapiens
<400> 609
   Leu Leu Asp Val Thr Leu Ile Leu Ala
   1 5
<210> 610
   <211> 9
   <212> PRT
   <213> Homo sapiens
<400> 610
   Leu Ile Phe Thr Asp Ser Lys Leu Tyr
   1 5
<210> 611
   <211> 9
   <212> PRT
   <213> Homo sapiens
<400> 611
   Phe Thr Asp Ser Lys Leu Tyr Ile Pro
   1 5
<210> 612
   <211> 9
   <212> PRT
   <213> Homo sapiens
<400> 612
   Val Phe Val Glu Arg Arg Gln Gln Tyr
   1 5
<210> 613
   <211> 9
   <212> PRT
   <213> Homo sapiens
<400> 613
   Phe Phe Asp Ile Lys Leu Leu Arg Asn
   1 5
<210> 614
   <211> 9
   <212> PRT
   <213> Homo sapiens
<400> 614
   Thr Arg Asp Gly Phe Asp Leu Asp Leu
   1 5
<210> 615
   <211> 9
   <212> PRT
   <213> Homo sapiens
<400> 615
   Tyr Asn Leu Cys Ser Glu Arg Ala Tyr
   1 5
<210> 616
   <211> 9
   <212> PRT
   <213> Homo sapiens
<400> 616
   Ala Gln Asp Leu Glu Asn Ile Val Ala
   1 5
<210> 617
   <211> 9
   <212> PRT
   <213> Homo sapiens
<400> 617
   Asp Asp Val Lys Val Gln Phe Phe Tyr
   1 5
<210> 618
   <211> 9
   <212> PRT
   <213> Homo sapiens
<400> 618
   Lys Asn Glu Leu Asp Asn Leu His Lys
   1 5
<210> 619
   <211> 9
   <212> PRT
   <213> Homo sapiens
<400> 619
   Ile Ser Glu Ser Val Leu Ala Arg Leu
   1 5
<210> 620
   <211> 9
   <212> PRT
   <213> Homo sapiens
<400> 620
   Glu Asp Ala Glu Asn Val Ala Ser Tyr
   1 5
<210> 621
   <211> 9
   <212> PRT
   <213> Homo sapiens
<400> 621
   Ile Leu Asp Thr Ala Ile Ile Val Ile
   1 5
<210> 622
   <211> 9
   <212> PRT
   <213> Homo sapiens
<400> 622
   Leu Val Asp Val Val Tyr Ile Phe Phe
   1 5
<210> 623
   <211> 9
   <212> PRT
   <213> Homo sapiens
<400> 623
   Val Ser Glu Asn Lys Arg Arg Tyr Thr
   1 5
<210> 624
   <211> 9
   <212> PRT
   <213> Homo sapiens
<400> 624
   Leu Asp Lys Lys His Arg Asn His Tyr
   1 5
<210> 625
   <211> 9
   <212> PRT
   <213> Homo sapiens
<400> 625
   Lys His Arg Asn His Tyr Arg Val Tyr
   1 5
<210> 626
   <211> 9
   <212> PRT
   <213> Homo sapiens
<400> 626
   Arg Thr Asp Lys Thr His Ser Glu Lys
   1               5
<210> 627
   <211> 9
   <212> PRT
   <213> Homo sapiens
<400> 627
   Phe Ile Ile Tyr Ser Ile Pro Arg Tyr
   1               5
<210> 628
   <211> 9
   <212> PRT
   <213> Homo sapiens
<400> 628
   Phe Phe Tyr Ser Asn Leu Pro Thr Tyr
   1               5
<210> 629
   <211> 9
   <212> PRT
   <213> Homo sapiens
<400> 629
   Phe Tyr Ser Asn Leu Pro Thr Tyr Tyr
   1               5
<210> 630
   <211> 9
   <212> PRT
   <213> Homo sapiens
<400> 630
   His Lys Gln Lys Ala Arg Arg Ile Tyr
   1               5
<210> 631
   <211> 9
   <212> PRT
   <213> Homo sapiens
<400> 631
   Thr Ser Glu Phe Lys Gly Ala Thr Glu
   1 5
<210> 632
   <211> 9
   <212> PRT
   <213> Homo sapiens
<400> 632
   Thr Ser Glu Phe Lys Gly Ala Ala Arg
   1 5
<210> 633
   <211> 9
   <212> PRT
   <213> Homo sapiens
<400> 633
   Pro Leu Glu Tyr Arg Ser Ile Ser Leu
   1 5
<210> 634
   <211> 9
   <212> PRT
   <213> Homo sapiens
<400> 634
   Trp Thr His Leu Leu Arg Leu Leu Arg
   1 5
<210> 635
   <211> 9
   <212> PRT
   <213> Homo sapiens
<400> 635
   Arg Val Ser Glu Asn Lys Arg Arg Tyr
   1 5
<210> 636
   <211> 9
   <212> PRT
   <213> Homo sapiens
<400> 636
   Tyr Thr Arg Asp Gly Phe Asp Leu Asp
   1 5
<210> 637
   <211> 9
   <212> PRT
   <213> Homo sapiens
<400> 637
   Ile Lys Glu Val Val Arg Phe Leu Asp
   1 5
<210> 638
   <211> 9
   <212> PRT
   <213> Homo sapiens
<400> 638
   Ala Tyr Asp Pro Lys His Phe His Asn
   1 5
<210> 639
   <211> 9
   <212> PRT
   <213> Homo sapiens
<400> 639
   Ala Ser Glu Ile Cys Ser Thr Ala Lys
   1 5
<210> 640
   <211> 9
   <212> PRT
   <213> Homo sapiens
<400> 640
   His Ser Glu Lys Phe Gln Gly Val Glu
   1 5
<210> 641
   <211> 9
   <212> PRT
   <213> Homo sapiens
<400> 641
   Thr Tyr Tyr Asp Asn Cys Ser Phe Tyr
   1 5
<210> 642
   <211> 9
   <212> PRT
   <213> Homo sapiens
<400> 642
   Pro Ser Asp Phe Ala Val Glu Ile Leu
   1 5
<210> 643
   <211> 10
   <212> PRT
   <213> Homo sapiens
<400> 643
   Phe Leu Asp Lys Lys His Arg Asn His Tyr
   1 5 10
<210> 644
   <211> 10
   <212> PRT
   <213> Homo sapiens
<400> 644
   Val Glu Asp Ala Glu Asn Val Ala Ser Tyr
   1 5 10
<210> 645
   <211> 10
   <212> PRT
   <213> Homo sapiens
<400> 645
   Tyr Asp Asp Val Lys Val Gln Phe Phe Tyr
   1 5 10
<210> 646
   <211> 10
   <212> PRT
   <213> Homo sapiens
<400> 646
   Asp Ser Lys Leu Tyr Ile Pro Leu Glu Tyr
   1 5 10
<210> 647
   <211> 10
   <212> PRT
   <213> Homo sapiens
<400> 647
   Cys Ser Thr Ala Lys Glu Ser Leu Tyr Tyr
   1 5 10
<210> 648
   <211> 10
   <212> PRT
   <213> Homo sapiens
<400> 648
   Gly Val Glu Thr Pro Ser Gln Lys Arg Tyr
   1 5 10
<210> 649
   <211> 10
   <212> PRT
   <213> Homo sapiens
<400> 649
   Ile Leu Phe Ile Lys His Phe Ile Ile Tyr
   1 5 10
<210> 650
   <211> 10
   <212> PRT
   <213> Homo sapiens
<400> 650
   Thr Ser Phe Ile Glu Asn Asn Arg Leu Tyr
   1 5 10
<210> 651
   <211> 10
   <212> PRT
   <213> Homo sapiens
<400> 651
   Pro Thr Asp Leu Ala Gly Val Ile Ile Glu
   1 5 10
<210> 652
   <211> 10
   <212> PRT
   <213> Homo sapiens
<400> 652
   Phe Thr Asp Ser Lys Leu Tyr Ile Pro Leu
   1 5 10
<210> 653
   <211> 10
   <212> PRT
   <213> Homo sapiens
<400> 653
   Phe Ser Asp Leu Phe Asn Ile Leu Asp Thr
   1 5 10
<210> 654
   <211> 10
   <212> PRT
   <213> Homo sapiens
<400> 654
   Arg Asp Gly Phe Asp Leu Asp Leu Thr Tyr
   1 5 10
<210> 655
   <211> 10
   <212> PRT
   <213> Homo sapiens
<400> 655
   Pro Thr Tyr Tyr Asp Asn Cys Ser Phe Tyr
   1 5 10
<210> 656
   <211> 10
   <212> PRT
   <213> Homo sapiens
<400> 656
   Pro Ser Asp Phe Ala Val Glu Ile Leu Phe
   1 5 10
<210> 657
   <211> 10
   <212> PRT
   <213> Homo sapiens
<400> 657
   Ile Lys Glu Val Val Arg Phe Leu Asp Lys
   1 5 10
<210> 658
   <211> 10
   <212> PRT
   <213> Homo sapiens
<400> 658
   Asp Leu Ile Phe Thr Asp Ser Lys Leu Tyr
   1 5 10
<210> 659
   <211> 10
   <212> PRT
   <213> Homo sapiens
<400> 659
   Ala Tyr Asp Pro Lys His Phe His Asn Arg
   1 5 10
<210> 660
   <211> 10
   <212> PRT
   <213> Homo sapiens
<400> 660
   Arg Thr Asp Lys Thr His Ser Glu Lys Phe
   1 5 10
<210> 661
   <211> 10
   <212> PRT
   <213> Homo sapiens
<400> 661
   Ala Tyr Phe Ala Gln Val Lys His Leu Tyr
   1 5 10
<210> 662
   <211> 10
   <212> PRT
   <213> Homo sapiens
<400> 662
   Ala Thr Glu Glu Ala Pro Ala Lys Glu Ser
   1 5 10
<210> 663
   <211> 10
   <212> PRT
   <213> Homo sapiens
<400> 663
   Leu Leu Asp Val Thr Leu Ile Leu Ala Asp
   1 5 10
<210> 664
   <211> 10
   <212> PRT
   <213> Homo sapiens
<400> 664
   Val Ile Leu Leu Leu Val Asp Val Val Tyr
   1 5 10
<210> 665
   <211> 10
   <212> PRT
   <213> Homo sapiens
<400> 665
   Val Thr Glu Arg Ile Ile Ala Met Ser Phe
   1 5 10
<210> 666
   <211> 10
   <212> PRT
   <213> Homo sapiens
<400> 666
   Gly Thr Asp Arg Thr Gly Thr Met Val Cys
   1 5 10
<210> 667
   <211> 10
   <212> PRT
   <213> Homo sapiens
<400> 667
   Ala Ser Glu Ile Cys Ser Thr Ala Lys Glu
   1 5 10
<210> 668
   <211> 10
   <212> PRT
   <213> Homo sapiens
<400> 668
   Ile Cys Ser Thr Ala Lys Glu Ser Leu Tyr
   1               5                   10
<210> 669
   <211> 10
   <212> PRT
   <213> Homo sapiens
<400> 669
   Thr Thr Asp Lys Ile Leu Ile Asp Val Phe
   1               5                   10
<210> 670
   <211> 10
   <212> PRT
   <213> Homo sapiens
<400> 670
   Ile Asp Val Phe Asp Gly Pro Pro Leu Tyr
   1               5                   10
<210> 671
   <211> 10
   <212> PRT
   <213> Homo sapiens
<400> 671
   Ser Pro Asp Pro Thr Asp Leu Ala Gly Val
   1               5                   10
<210> 672
   <211> 10
   <212> PRT
   <213> Homo sapiens
<400> 672
   Ile Ser Glu Ser Val Leu Ala Arg Leu Ser
   1               5                   10
<210> 673
   <211> 10
   <212> PRT
   <213> Homo sapiens
<400> 673
   Arg Val Phe Val Glu Arg Arg Gln Gln Tyr
   1 5 10
<210> 674
   <211> 10
   <212> PRT
   <213> Homo sapiens
<400> 674
   Val Ser Glu Asn Lys Arg Arg Tyr Thr Arg
   1 5 10
<210> 675
   <211> 10
   <212> PRT
   <213> Homo sapiens
<400> 675
   Phe Pro Ser Ser Gly Arg Gln Ser Phe Tyr
   1 5 10
<210> 676
   <211> 10
   <212> PRT
   <213> Homo sapiens
<400> 676
   Lys Lys His Arg Asn His Tyr Arg Val Tyr
   1 5 10
<210> 677
   <211> 10
   <212> PRT
   <213> Homo sapiens
<400> 677
   Val Tyr Asn Leu Cys Ser Glu Arg Ala Tyr
   1 5 10
<210> 678
   <211> 10
   <212> PRT
   <213> Homo sapiens
<400> 678
   Cys Ser Glu Arg Ala Tyr Asp Pro Lys His
   1 5 10
<210> 679
   <211> 10
   <212> PRT
   <213> Homo sapiens
<400> 679
   Phe Phe Tyr Ser Asn Leu Pro Thr Tyr Tyr
   1 5 10
<210> 680
   <211> 10
   <212> PRT
   <213> Homo sapiens
<400> 680
   Leu His Lys Gln Lys Ala Arg Arg Ile Tyr
   1 5 10
<210> 681
   <211> 10
   <212> PRT
   <213> Homo sapiens
<400> 681
   Arg Arg Val Ser Glu Asn Lys Arg Arg Tyr
   1 5 10
<210> 682
   <211> 10
   <212> PRT
   <213> Homo sapiens
<400> 682
   Asp Leu Glu Asn Ile Val Ala Ile His Cys
   1 5 10
<210> 683
   <211> 10
   <212> PRT
   <213> Homo sapiens
<400> 683
   Gly Thr Met Val Cys Ala Phe Leu Ile Ala
   1 5 10
<210> 684
   <211> 10
   <212> PRT
   <213> Homo sapiens
<400> 684
   His Ser Glu Lys Phe Gln Gly Val Glu Thr
   1 5 10
<210> 685
   <211> 10
   <212> PRT
   <213> Homo sapiens
<400> 685
   Thr Pro Ser Gln Lys Arg Tyr Val Ala Tyr
   1 5 10
<210> 686
   <211> 10
   <212> PRT
   <213> Homo sapiens
<400> 686
   His Phe Ile Ile Tyr Ser Ile Pro Arg Tyr
   1 5 10
<210> 687
   <211> 10
   <212> PRT
   <213> Homo sapiens
<400> 687
   Val Arg Asp Leu Lys Ile Gln Ile Glu Met
   1 5 10
<210> 688
   <211> 10
   <212> PRT
   <213> Homo sapiens
<400> 688
   Gln Phe Phe Tyr Ser Asn Leu Pro Thr Tyr
   1 5 10
<210> 689
   <211> 10
   <212> PRT
   <213> Homo sapiens
<400> 689
   Tyr Tyr Asp Asn Cys Ser Phe Tyr Phe Trp
   1 5 10
<210> 690
   <211> 9
   <212> PRT
   <213> Homo sapiens
<400> 690
   Ile Leu Leu Leu Val Asp Val Val Tyr
   1 5
<210> 691
   <211> 9
   <212> PRT
   <213> Homo sapiens
<400> 691
   Lys Leu Ile Arg Arg Arg Val Ser Glu
   1 5
<210> 692
   <211> 9
   <212> PRT
   <213> Homo sapiens
<400> 692
   Tyr Val Ala Tyr Phe Ala Gln Val Lys
   1 5
<210> 693
   <211> 9
   <212> PRT
   <213> Homo sapiens
<400> 693
   Arg Val Tyr Asn Leu Cys Ser Glu Arg
   1 5
<210> 694
   <211> 9
   <212> PRT
   <213> Homo sapiens
<400> 694
   Val Val Tyr Ile Phe Phe Asp Ile Lys
   1               5
<210> 695
   <211> 9
   <212> PRT
   <213> Homo sapiens
<400> 695
   Arg Ile Phe His Leu Phe His Gln Lys
   1               5
<210> 696
   <211> 9
   <212> PRT
   <213> Homo sapiens
<400> 696
   Ala Val Glu Ile Leu Phe Gly Glu Lys
   1               5
<210> 697
   <211> 9
   <212> PRT
   <213> Homo sapiens
<400> 697
   Ser Val Leu Ala Arg Leu Ser Lys Phe
   1               5
<210> 698
   <211> 9
   <212> PRT
   <213> Homo sapiens
<400> 698
   Val Leu Leu Arg Val Phe Val Glu Arg
   1               5
<210> 699
   <211> 9
   <212> PRT
   <213> Homo sapiens
<400> 699
   Glu Leu Asp Asn Leu His Lys Gln Lys
   1               5
<210> 700
   <211> 9
   <212> PRT
   <213> Homo sapiens
<400> 700
   Glu Val Val Arg Phe Leu Asp Lys Lys
   1               5
<210> 701
   <211> 9
   <212> PRT
   <213> Homo sapiens
<400> 701
   Lys Leu Tyr Ile Pro Leu Glu Tyr Arg
   1               5
<210> 702
   <211> 9
   <212> PRT
   <213> Homo sapiens
<400> 702
   Arg Leu Ile Ile Leu Leu Arg Ile Phe
   1               5
<210> 703
   <211> 9
   <212> PRT
   <213> Homo sapiens
<400> 703
   Ser Leu Tyr Tyr Phe Gly Glu Arg Arg
   1               5
<210> 704
   <211> 9
   <212> PRT
   <213> Homo sapiens
<400> 704
   Lys Ile Gln Ile Glu Met Glu Lys Lys
   1               5
<210> 705
   <211> 9
   <212> PRT
   <213> Homo sapiens
<400> 705
   Val Leu Asp Asn Ile Thr Thr Asp Lys
   1 5
<210> 706
   <211> 9
   <212> PRT
   <213> Homo sapiens
<400> 706
   Ile Val His Ser Ile Val Ser Ser Phe
   1 5
<210> 707
   <211> 9
   <212> PRT
   <213> Homo sapiens
<400> 707
   Thr Leu Ile Leu Ala Asp Leu Ile Phe
   1 5
<210> 708
   <211> 9
   <212> PRT
   <213> Homo sapiens
<400> 708
   Leu Leu Arg Leu Ile Ile Leu Leu Arg
   1 5
<210> 709
   <211> 9
   <212> PRT
   <213> Homo sapiens
<400> 709
   Ile Leu Leu Arg Ile Phe His Leu Phe
   1 5
<210> 710
   <211> 9
   <212> PRT
   <213> Homo sapiens
<400> 710
   Leu Leu Arg Ile Phe His Leu Phe His
   1 5
<210> 711
   <211> 9
   <212> PRT
   <213> Homo sapiens
<400> 711
   Arg Val Ser Glu Asn Lys Arg Arg Tyr
   1 5
<210> 712
   <211> 9
   <212> PRT
   <213> Homo sapiens
<400> 712
   Gly Leu Phe Gly Val Phe Leu Val Leu
   1 5
<210> 713
   <211> 9
   <212> PRT
   <213> Homo sapiens
<400> 713
   Ile Val Ile Leu Leu Leu Val Asp Val
   1 5
<210> 714
   <211> 9
   <212> PRT
   <213> Homo sapiens
<400> 714
   Ala Ile His Cys Lys Gly Gly Thr Asp
   1 5
<210> 715
   <211> 9
   <212> PRT
   <213> Homo sapiens
<400> 715
   Gln Gly Val Glu Thr Pro Ser Gln Lys
   1 5
<210> 716
   <211> 9
   <212> PRT
   <213> Homo sapiens
<400> 716
   Asn Leu Pro Pro Arg Arg Ile Leu Phe
   1               5
<210> 717
   <211> 9
   <212> PRT
   <213> Homo sapiens
<400> 717
   Asp Val Phe Asp Gly Pro Pro Leu Tyr
   1               5
<210> 718
   <211> 9
   <212> PRT
   <213> Homo sapiens
<400> 718
   Arg Ile Tyr Pro Ser Asp Phe Ala Val
   1               5
<210> 719
   <211> 9
   <212> PRT
   <213> Homo sapiens
<400> 719
   Arg Val Ser Pro Ile Ser Glu Ser Val
   1               5
<210> 720
   <211> 9
   <212> PRT
   <213> Homo sapiens
<400> 720
   Phe Leu Val Leu Leu Asp Val Thr Leu
   1               5
<210> 721
   <211> 9
   <212> PRT
   <213> Homo sapiens
<400> 721
   Asp Val Leu Leu Arg Val Phe Val Glu
   1 5
<210> 722
   <211> 9
   <212> PRT
   <213> Homo sapiens
<400> 722
   Phe Val Glu Arg Arg Gln Gln Tyr Phe
   1 5
<210> 723
   <211> 9
   <212> PRT
   <213> Homo sapiens
<400> 723
   Arg Leu Leu Arg Leu Ile Ile Leu Leu
   1 5
<210> 724
   <211> 9
   <212> PRT
   <213> Homo sapiens
<400> 724
   Leu Ile Arg Arg Arg Val Ser Glu Asn
   1 5
<210> 725
   <211> 9
   <212> PRT
   <213> Homo sapiens
<400> 725
   Ile Arg Arg Arg Val Ser Glu Asn Lys
   1 5
<210> 726
   <211> 9
   <212> PRT
   <213> Homo sapiens
<400> 726
   Asp Leu Asp Leu Thr Tyr Val Thr Glu
   1 5
<210> 727
   <211> 9
   <212> PRT
   <213> Homo sapiens
<400> 727
   Ser Val Leu Asp Asn Ile Thr Thr Asp
   1 5
<210> 728
   <211> 9
   <212> PRT
   <213> Homo sapiens
<400> 728
   Pro Leu Tyr Asp Asp Val Lys Val Gln
   1 5
<210> 729
   <211> 9
   <212> PRT
   <213> Homo sapiens
<400> 729
   Arg Leu Tyr Leu Pro Lys Asn Glu Leu
   1 5
<210> 730
   <211> 9
   <212> PRT
   <213> Homo sapiens
<400> 730
   Ile Leu Phe Gly Glu Lys Met Thr Ser
   1 5
<210> 731
   <211> 9
   <212> PRT
   <213> Homo sapiens
<400> 731
   Arg Leu Ser Lys Phe Glu Val Glu Asp
   1 5
<210> 732
   <211> 9
   <212> PRT
   <213> Homo sapiens
<400> 732
   Ala Ser Tyr Asp Ser Lys Ile Lys Lys
   1 5
<210> 733
   <211> 9
   <212> PRT
   <213> Homo sapiens
<400> 733
   Ile Leu Ala Asp Leu Ile Phe Thr Asp
   1 5
<210> 734
   <211> 9
   <212> PRT
   <213> Homo sapiens
<400> 734
   Ala Asp Leu Ile Phe Thr Asp Ser Lys
   1 5
<210> 735
   <211> 9
   <212> PRT
   <213> Homo sapiens
<400> 735
   Arg Val Phe Val Glu Arg Arg Gln Gln
   1 5
<210> 736
   <211> 9
   <212> PRT
   <213> Homo sapiens
<400> 736
   Ile Ile Val Ile Leu Leu Leu Val Asp
   1 5
<210> 737
   <211> 9
   <212> PRT
   <213> Homo sapiens
<400> 737
   Lys Leu Leu Arg Asn Ile Pro Arg Trp
   1 5
<210> 738
   <211> 9
   <212> PRT
   <213> Homo sapiens
<400> 738
   Leu Leu Arg Leu Leu Arg Leu Ile Ile
   1 5
<210> 739
   <211> 9
   <212> PRT
   <213> Homo sapiens
<400> 739
   Arg Asn Pro Ile Lys Glu Val Val Arg
   1 5
<210> 740
   <211> 9
   <212> PRT
   <213> Homo sapiens
<400> 740
   Lys Glu Val Val Arg Phe Leu Asp Lys
   1 5
<210> 741
   <211> 9
   <212> PRT
   <213> Homo sapiens
<400> 741
   Val Val Arg Ile Met Ile Asp Asp His
   1 5
<210> 742
   <211> 9
   <212> PRT
   <213> Homo sapiens
<400> 742
   Asp Leu Glu Asn Ile Val Ala Ile His
   1 5
<210> 743
   <211> 9
   <212> PRT
   <213> Homo sapiens
<400> 743
   His Leu Tyr Asn Trp Asn Leu Pro Pro
   1 5
<210> 744
   <211> 10
   <212> PRT
   <213> Homo sapiens
<400> 744
   Arg Leu Leu Arg Leu Ile Ile Leu Leu Arg
   1 5 10
<210> 745
   <211> 10
   <212> PRT
   <213> Homo sapiens
<400> 745
   Pro Leu Tyr Asp Asp Val Lys Val Gln Phe
   1 5 10
<210> 746
   <211> 10
   <212> PRT
   <213> Homo sapiens
<400> 746
   Arg Ile Tyr Pro Ser Asp Phe Ala Val Glu
   1 5 10
<210> 747
   <211> 10
   <212> PRT
   <213> Homo sapiens
<400> 747
   Val Ile Leu Leu Leu Val Asp Val Val Tyr
   1 5 10
<210> 748
   <211> 10
   <212> PRT
   <213> Homo sapiens
<400> 748
   Ser Val Leu Asp Asn Ile Thr Thr Asp Lys
   1 5 10
<210> 749
   <211> 10
   <212> PRT
   <213> Homo sapiens
<400> 749
   Arg Val Ser Pro Ile Ser Glu Ser Val Leu
   1 5 10
<210> 750
   <211> 10
   <212> PRT
   <213> Homo sapiens
<400> 750
   Arg Val Phe Val Glu Arg Arg Gln Gln Tyr
   1 5 10
<210> 751
   <211> 10
   <212> PRT
   <213> Homo sapiens
<400> 751
   Ile Leu Leu Arg Ile Phe His Leu Phe His
   1 5 10
<210> 752
   <211> 10
   <212> PRT
   <213> Homo sapiens
<400> 752
   Leu Ile Arg Arg Arg Val Ser Glu Asn Lys
   1 5 10
<210> 753
   <211> 10
   <212> PRT
   <213> Homo sapiens
<400> 753
   Thr Leu His Gln Met Val Val Phe Thr Lys
   1 5 10
<210> 754
   <211> 10
   <212> PRT
   <213> Homo sapiens
<400> 754
   Leu Leu Arg Asn Ile Pro Arg Trp Thr His
   1 5 10
<210> 755
   <211> 10
   <212> PRT
   <213> Homo sapiens
<400> 755
   Arg Leu Ile Ile Leu Leu Arg Ile Phe His
   1 5 10
<210> 756
   <211> 10
   <212> PRT
   <213> Homo sapiens
<400> 756
   Arg Tyr Val Ala Tyr Phe Ala Gln Val Lys
   1 5 10
<210> 757
   <211> 10
   <212> PRT
   <213> Homo sapiens
<400> 757
   Ile Ile Tyr Ser Ile Pro Arg Tyr Val Arg
   1 5 10
<210> 758
   <211> 10
   <212> PRT
   <213> Homo sapiens
<400> 758
   Ser Ile Pro Arg Tyr Val Arg Asp Leu Lys
   1 5 10
<210> 759
   <211> 10
   <212> PRT
   <213> Homo sapiens
<400> 759
   Asp Leu Lys Ile Gln Ile Glu Met Glu Lys
   1 5 10
<210> 760
   <211> 10
   <212> PRT
   <213> Homo sapiens
<400> 760
   Val Val Phe Ser Thr Ile Ser Leu Gly Lys
   1 5 10
<210> 761
   <211> 10
   <212> PRT
   <213> Homo sapiens
<400> 761
   Lys Leu Ile Arg Arg Arg Val Ser Glu Asn
   1 5 10
<210> 762
   <211> 10
   <212> PRT
   <213> Homo sapiens
<400> 762
   Gln Ile Glu Met Glu Lys Lys Val Val Phe
   1 5 10
<210> 763
   <211> 10
   <212> PRT
   <213> Homo sapiens
<400> 763
   Asn Val Ala Ser Tyr Asp Ser Lys Ile Lys
   1               5                   10
<210> 764
   <211> 10
   <212> PRT
   <213> Homo sapiens
<400> 764
   Asp Val Leu Leu Arg Val Phe Val Glu Arg
   1               5                   10
<210> 765
   <211> 10
   <212> PRT
   <213> Homo sapiens
<400> 765
   Ala Ile Ile Val Ile Leu Leu Leu Val Asp
   1               5                   10
<210> 766
   <211> 10
   <212> PRT
   <213> Homo sapiens
<400> 766
   Arg Val Val Arg Ile Met Ile Asp Asp His
   1               5                   10
<210> 767
   <211> 10
   <212> PRT
   <213> Homo sapiens
<400> 767
   Lys Ile Val His Ser Ile Val Ser Ser Phe
   1               5                   10
<210> 768
   <211> 10
   <212> PRT
   <213> Homo sapiens
<400> 768
   Ser Ile Ser Leu Ala Ile Ala Leu Phe Phe
   1               5                   10
<210> 769
   <211> 10
   <212> PRT
   <213> Homo sapiens
<400> 769
   Ile Val Ile Leu Leu Leu Val Asp Val Val
   1 5 10
<210> 770
   <211> 10
   <212> PRT
   <213> Homo sapiens
<400> 770
   Asp Val Val Tyr Ile Phe Phe Asp Ile Lys
   1 5 10
<210> 771
   <211> 10
   <212> PRT
   <213> Homo sapiens
<400> 771
   Lys Leu Leu Arg Asn Ile Pro Arg Trp Thr
   1 5 10
<210> 772
   <211> 10
   <212> PRT
   <213> Homo sapiens
<400> 772
   Asp Leu Ile Phe Thr Asp Ser Lys Leu Tyr
   1 5 10
<210> 773
   <211> 10
   <212> PRT
   <213> Homo sapiens
<400> 773
   Phe Leu Met Asp Val Leu Leu Arg Val Phe
   1 5 10
<210> 774
   <211> 10
   <212> PRT
   <213> Homo sapiens
<400> 774
   Val Leu Leu Arg Val Phe Val Glu Arg Arg
   1 5 10
<210> 775
   <211> 10
   <212> PRT
   <213> Homo sapiens
<400> 775
   Ile Leu Leu Leu Val Asp Val Val Tyr Ile
   1 5 10
<210> 776
   <211> 10
   <212> PRT
   <213> Homo sapiens
<400> 776
   His Leu Leu Arg Leu Leu Arg Leu Ile Ile
   1 5 10
<210> 777
   <211> 10
   <212> PRT
   <213> Homo sapiens
<400> 777
   Arg Ile Ile Ala Met Ser Phe Pro Ser Ser
   1 5 10
<210> 778
   <211> 10
   <212> PRT
   <213> Homo sapiens
<400> 778
   Arg Val Tyr Asn Leu Cys Ser Glu Arg Ala
   1 5 10
<210> 779
   <211> 10
   <212> PRT
   <213> Homo sapiens
<400> 779
   Ala Ile His Cys Lys Gly Gly Thr Asp Arg
   1 5 10
<210> 780
   <211> 10
   <212> PRT
   <213> Homo sapiens
<400> 780
   Tyr Val Ala Tyr Phe Ala Gln Val Lys His
   1 5 10
<210> 781
   <211> 10
   <212> PRT
   <213> Homo sapiens
<400> 781
   His Leu Tyr Asn Trp Asn Leu Pro Pro Arg
   1 5 10
<210> 782
   <211> 10
   <212> PRT
   <213> Homo sapiens
<400> 782
   Ile Leu Phe Ile Lys His Phe Ile Ile Tyr
   1 5 10
<210> 783
   <211> 10
   <212> PRT
   <213> Homo sapiens
<400> 783
   Arg Leu Tyr Leu Pro Lys Asn Glu Leu Asp
   1 5 10
<210> 784
   <211> 10
   <212> PRT
   <213> Homo sapiens
<400> 784
   Lys Gly Ala Thr Glu Glu Ala Pro Ala Lys
   1 5 10
<210> 785
   <211> 10
   <212> PRT
   <213> Homo sapiens
<400> 785
   Lys Leu Tyr Ile Pro Leu Glu Tyr Arg Ser
   1 5 10
<210> 786
   <211> 10
   <212> PRT
   <213> Homo sapiens
<400> 786
   Asn Ile Pro Arg Trp Thr His Leu Leu Arg
   1 5 10
<210> 787
   <211> 10
   <212> PRT
   <213> Homo sapiens
<400> 787
   Arg Arg Thr Asp Lys Thr His Ser Glu Lys
   1 5 10
<210> 788
   <211> 9
   <212> PRT
   <213> Homo sapiens
<400> 788
   Ala Pro Ala Lys Glu Ser Pro His Thr
   1 5
<210> 789
   <211> 9
   <212> PRT
   <213> Homo sapiens
<400> 789
   Asn Pro Ile Lys Glu Val Val Arg Phe
   1 5
<210> 790
   <211> 9
   <212> PRT
   <213> Homo sapiens
<400> 790
   Thr Pro Ser Gln Lys Arg Tyr Val Ala
   1 5
<210> 791
   <211> 9
   <212> PRT
   <213> Homo sapiens
<400> 791
   Leu Pro Lys Asn Glu Leu Asp Asn Leu
   1 5
<210> 792
   <211> 9
   <212> PRT
   <213> Homo sapiens
<400> 792
   Tyr Pro Ser Asp Phe Ala Val Glu Ile
   1 5
<210> 793
   <211> 9
   <212> PRT
   <213> Homo sapiens
<400> 793
   Phe Pro Ser Ser Gly Arg Gln Ser Phe
   1 5
<210> 794
   <211> 9
   <212> PRT
   <213> Homo sapiens
<400> 794
   Leu Pro Pro Arg Arg Ile Leu Phe Ile
   1 5
<210> 795
   <211> 9
   <212> PRT
   <213> Homo sapiens
<400> 795
   Ser Pro Ile Ser Glu Ser Val Leu Ala
   1 5
<210> 796
   <211> 9
   <212> PRT
   <213> Homo sapiens
<400> 796
   Pro Pro Leu Tyr Asp Asp Val Lys Val
   1 5
<210> 797
   <211> 9
   <212> PRT
   <213> Homo sapiens
<400> 797
   Asp Pro Thr Asp Leu Ala Gly Val Ile
   1 5
<210> 798
   <211> 9
   <212> PRT
   <213> Homo sapiens
<400> 798
   Ala Phe Gly Leu Phe Gly Val Phe Leu
   1 5
<210> 799
   <211> 9
   <212> PRT
   <213> Homo sapiens
<400> 799
   Val Pro Thr Leu His Gln Met Val Val
   1 5
<210> 800
   <211> 9
   <212> PRT
   <213> Homo sapiens
<400> 800
   Gly Leu Phe Gly Val Phe Leu Val Leu
   1 5
<210> 801
   <211> 9
   <212> PRT
   <213> Homo sapiens
<400> 801
   Asp Pro Lys His Phe His Asn Arg Val
   1               5
<210> 802
   <211> 9
   <212> PRT
   <213> Homo sapiens
<400> 802
   Ser Pro Asp Pro Thr Asp Leu Ala Gly
   1               5
<210> 803
   <211> 9
   <212> PRT
   <213> Homo sapiens
<400> 803
   Ile Pro Arg Trp Thr His Leu Leu Arg
   1               5
<210> 804
   <211> 9
   <212> PRT
   <213> Homo sapiens
<400> 804
   Thr Arg Asp Gly Phe Asp Leu Asp Leu
   1               5
<210> 805
   <211> 9
   <212> PRT
   <213> Homo sapiens
<400> 805
   Trp Asn Leu Pro Pro Arg Arg Ile Leu
   1               5
<210> 806
   <211> 10
   <212> PRT
   <213> Homo sapiens
<400> 806
   Ile Pro Arg Trp Thr His Leu Leu Arg Leu
   1 5 10
<210> 807
   <211> 10
   <212> PRT
   <213> Homo sapiens
<400> 807
   Asn Pro Ile Lys Glu Val Val Arg Phe Leu
   1 5 10
<210> 808
   <211> 10
   <212> PRT
   <213> Homo sapiens
<400> 808
   Tyr Pro Ser Asp Phe Ala Val Glu Ile Leu
   1 5 10
<210> 809
   <211> 10
   <212> PRT
   <213> Homo sapiens
<400> 809
   Ile Pro Leu Glu Tyr Arg Ser Ile Ser Leu
   1 5 10
<210> 810
   <211> 10
   <212> PRT
   <213> Homo sapiens
<400> 810
   Ile Pro Arg Tyr Val Arg Asp Leu Lys Ile
   1 5 10
<210> 811
   <211> 10
   <212> PRT
   <213> Homo sapiens
<400> 811
   Ser Pro Asp Pro Thr Asp Leu Ala Gly Val
   1 5 10
<210> 812
   <211> 10
   <212> PRT
   <213> Homo sapiens
<400> 812
   Val Pro Thr Leu His Gln Met Val Val Phe
   1 5 10
<210> 813
   <211> 10
   <212> PRT
   <213> Homo sapiens
<400> 813
   Asp Pro Thr Asp Leu Ala Gly Val Ile Ile
   1 5 10
<210> 814
   <211> 10
   <212> PRT
   <213> Homo sapiens
<400> 814
   Gly Pro Pro Leu Tyr Asp Asp Val Lys Val
   1 5 10
<210> 815
   <211> 10
   <212> PRT
   <213> Homo sapiens
<400> 815
   Ser Pro Gln Thr Ser Glu Phe Lys Gly Ala
   1 5 10
<210> 816
   <211> 10
   <212> PRT
   <213> Homo sapiens
<400> 816
   Ser Pro His Thr Ser Glu Phe Lys Gly Ala
   1 5 10
<210> 817
   <211> 10
   <212> PRT
   <213> Homo sapiens
<400> 817
   Asp Pro Lys His Phe His Asn Arg Val Val
   1 5 10
<210> 818
   <211> 10
   <212> PRT
   <213> Homo sapiens
<400> 818
   Leu Pro Thr Tyr Tyr Asp Asn Cys Ser Phe
   1 5 10
<210> 819
   <211> 10
   <212> PRT
   <213> Homo sapiens
<400> 819
   Arg Val Ser Pro Ile Ser Glu Ser Val Leu
   1 5 10
<210> 820
   <211> 10
   <212> PRT
   <213> Homo sapiens
<400> 820
   Phe Ala Phe Gly Leu Phe Gly Val Phe Leu
   1 5 10
<210> 821
   <211> 10
   <212> PRT
   <213> Homo sapiens
<400> 821
   Thr Pro Ser Gln Lys Arg Tyr Val Ala Tyr
   1 5 10
<210> 822
   <211> 1320
   <212> DNA
   <213> Homo sapiens
<400> 822
   accttgcaga ggagggggcg gcggggacag gacccgggtc tgtgcctgcg cacaccctcg     60
   cgggccaacc ccctcccccc ccgccccgcc gggtacctgt cacggctact gccgcggcgc    120
   gcgcccgcca cgttaagccg gatggcggga ggaggggtgc ggggagggtg gcacggcgcg    180
   cgcgtgcgcg ggagtcgccg agcaagaact ggtagtgcgc gcgctcgccg ctcgctcgcg    240
   tcccggaggc ggagtctgcg gcggcgggcg gaacgggggc gcgcctatgc tagtcacgtg    300
   ggcgctgggg cggggccggg cggccgttca aggcaggggg cggggcgtct ccgagcggcg    360
   gggccaaggg agggcacaac agctgctacc tgaacagttt ctgacccaac agttacccag    420
   cgccggactc gctgcgcccc ggcggctcta gggacccccg gcgcctacac ttagctccgc    480
   gcccgaggtg agcccaggcc ctaagtcctc cgggcggggg taggggtggg ggacgctcct    540
   ttttgttggg ggggggtctt ggaggcgcga aggcactagg cgcctcggcg gatggctgaa    600
   cccctcgccc gcggctcccc gtgtcttttg gggggccggg tgcgggcgcg gaatccggga    660
   ggtgtccgca caaaaggccg agaaaaactc cgcgacgcct ccctccctcc ctccgccctc    720
   cccgtcccct cctctccgcg cccgctcctc ctcattcaaa cccggccggc ctgagtggtg    780
   ttagctcagt cccggccgcc gccgcgtgag gaaatggcct aggagccgga gccgcaggta    840
   aagggggcgc gccccccgcc cgcgccagcc ggggcgcccg cccggtcctg cggaggctcc    900
   cgcgccgccc ccgaggcgcc cggctctcgt tgtcttgtcc ccccccagga tgacctgagt    960
   tccccgtgtc tgctttctag ccgctcctct agggcgcccc ctccccagct ggctcctgcg   1020
   cttccgccca cgggaaggtg cgggcgatcc cgggctgcat ccgctcttgg ccgtcacact   1080
   cacgctgcac gatttagaag ggcttggggt gcggggttcc ctgagtctcc gccgggagcg   1140
   ggggtccggg ggtctgcggg ggtgggcggt ggggtgctgt tccgaaaagt tgggtcgccc   1200
   cgcggggcgg gtctcccaat ttgcccagtc ggcccttggg gcgcgggcgg aggggggacg   1260
   tcggcgtagg gtccccagcg atgccccggg aagggctggg acaagttgga aacggtcaaa   1320